(19) Europäisches Patentamt
European Patent Office
Office européen des brevets

(11) EP 2 227 486 B1

(12) # EUROPEAN PATENT SPECIFICATION

(45) Date of publication and mention of the grant of the patent:
**08.03.2017 Bulletin 2017/10**

(21) Application number: **08851987.1**

(22) Date of filing: **20.11.2008**

(51) Int Cl.:
***C07K 14/47*** *(2006.01)* ***C07K 7/64*** *(2006.01)*
***A61K 39/00*** *(2006.01)*

(86) International application number:
**PCT/IB2008/003493**

(87) International publication number:
**WO 2009/066175 (28.05.2009 Gazette 2009/22)**

(54) **PEPTIDE ANALOGUES AND CONJUGATES THEREOF**

PEPTIDANALOGA UND KONJUGATE DAVON

ANALOGUES PEPTIDIQUES ET LEURS CONJUGUÉS

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **20.11.2007 GR 20070100697**
**20.12.2007 GB 0724878**
**09.06.2008 GB 0810553**
**12.09.2008 AU 2008904757**

(43) Date of publication of application:
**15.09.2010 Bulletin 2010/37**

(73) Proprietor: **Vianex S.A.**
**146 71 Nea Erythrea (GR)**

(72) Inventors:
- **MATSOUKAS, John**
**Patras (GR)**
- **APOSTOLOPOULOS, Vasso**
**St Albans**
**Victoria 3021 (AU)**
- **TSELIOS, Theodoros**
**Mesologgi (FR)**
- **KATSARA, Maria**
**Kalamata (GR)**
- **DERAOS, George**
**Patras (GR)**
- **GRIGORIADIS, Nikos**
**Thessaloniki (GR)**
- **LOURBOPOULOS, Athanasios**
**Thessaloniki (GR)**

(74) Representative: **D Young & Co LLP**
**120 Holborn**
**London EC1N 2DY (GB)**

(56) References cited:
**WO-A-99/57241 WO-A-03/033645**
**WO-A-03/040165**

- **SINGH R A ET AL: "Th1 and Th2 deviation of myelin-autoreactive T cells by altered peptide ligands is associated with reciprocal regulation of Lck, Fyn, and ZAP-70." JOURNAL OF IMMUNOLOGY (BALTIMORE, MD. : 1950) 15 DEC 1999, vol. 163, no. 12, 15 December 1999 (1999-12-15), pages 6393-6402, XP002523847 ISSN: 0022-1767**
- **KATSARA MARIA ET AL: "Design of novel cyclic altered peptide ligands of myelin basic protein MBP83-99 that modulate immune responses in SJL/J mice" JOURNAL OF MEDICINAL CHEMISTRY, vol. 51, no. 13, July 2008 (2008-07), pages 3971-3978, XP002523848 ISSN: 0022-2623**
- **John Matsoukas ET AL: "Design And Synthesis of a Novel Potent Myelin Basic Protein Epitope 87-99 Cyclic Analogue: Enhanced Stability and Biological Properties of Mimics Render Them a Potentially New Class of Immunomodulators +", Journal of Medicinal Chemistry, vol. 48, no. 5, 1 March 2005 (2005-03-01), pages 1470-1480, XP055190111, ISSN: 0022-2623, DOI: 10.1021/jm040849g**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• THEODORE TSELIOS ET AL: "Antagonistic effects of human cyclic MBP87-99 altered peptide ligands in experimental allergic encephalomyelitis and human T-cell proliferation", JOURNAL OF MEDICINAL CHEMISTRY, AMERICAN CHEMICAL SOCIETY, US, vol. 45, no. 2, 1 January 2002 (2002-01-01), pages 275-283, XP009118911, ISSN: 0022-2623, DOI: 10.1021/JM0102147 [retrieved on 2001-12-15]

## Description

The present invention relates to linear and cyclic peptide analogues of myelin antigens and conjugates thereof. The peptides and conjugates of the invention are useful candidates for the immunotherapy of Multiple Sclerosis (MS).

## BACKGROUND TO THE INVENTION

Multiple Sclerosis (MS) is an inflammatory disease of the central nervous system (CNS) and is characterized by a dysregulated T cell response. An association between MHC class II alleles and disease has been observed in MS patients, in particular HLA-DR1, HLA-DR2 and HLA-DR4 (Valli et al., J Clin. Invest. 1993).

Current peptide therapies of multiple sclerosis include treatment with interferons (Interferon beta-1$\alpha$ and Interferon beta-1$\beta$) and glatiramer acetate (copolymer-1) which is a mixture of synthetic polypeptides comprised of the major aminoacids Glu, Gln, Lys, Arg of MBP. These immunomodulators have been approved by the FDA for patients with relapsing-remitting MS. Interferons given by subcutaneous or intramuscular injection reduce the frequency, severity and duration of exacerbation but their impact on preventing disability over the long-term is not yet established. Side effects are also common and include reactions at the injection site, fever, myalgia and flu-like syndrome. So far the reported benefits from the use of interferons and copolymer are marginal and there is therefore a need for improved therapeutics. Another approach under clinical investigation for autoimmune suppression is the oral administration of autoantigens. Orally administered antigens suppress autoimmunity in animal models, including EAE, collagen and adjuvant-induced arthritis, uveitis and diabetes in the non-obese diabetic mouse. Low doses of oral antigen induce antigen-specific regulatory T-cells which act by releasing inhibitory cytokines such as transforming growth factor-beta, IL-4, and IL-10 at the target organ. Thus, one can suppress inflammation at a target organ by orally administering an antigen derived from the site of inflammation, even if it is not the target of the autoimmune response. Initial human trials of orally administered antigen have shown positive findings in patients with MS and Rheumatoid Arthritis. A double-blind, placebo-controlled, phase III multi-centre trial of oral myelin in relapsing-remitting multiple sclerosis patients is in progress, as are phase II clinical trials investigating the oral administration of type II collagen in rheumatoid arthritis, S-antigen in uveitis and insulin in type I diabetes. This promising method is advantageous because it allows oral administration in contrast to previous methods using interferons and copolymer-1. However, issues related to the peptide nature and cost of administered substance renders the non-peptide mimetic approach, even in its infancy, an attractive goal to pursue.

### Strategies in the immunotherapy of MS and clinical perspectives

In the immunotherapeutic approach towards the development of therapeutic vaccines for MS, the assumption is that MBP epitopes or their analogues can actively inhibit or prevent disease through the activation of antigen-specific regulatory T cells, or antibodies related to myelin sheath destruction. The myelin sheath is constituted from the proteins: MBP, Proteolipid Protein (PLP), Myelin Oligodentrocyte Glycoprotein (MOG) and heat-shock protein which are implicated in MS. Thus, epitopes of these myelin sheath proteins are targets for immunotherapeutic techniques. In areas of inflammation in MS, antibodies against the minor protein MOG have been demonstrated. MOG antibodies are related to significant myelin disruption, probably by coating the myelin so that macrophages can engulf and destroy coated sections of myelin, blocking nerve impulses temporarily or permanently. Thus, it is known that antibodies play a role in MS, and cooperate with antigen presenting cells in myelin destruction. Blocking the effects of these MOG antibodies with secondary antibodies or non-peptide mimetics may be an important avenue for future therapy.

Another direction in the immunotherapy of autoimmune diseases is the use of Multiple-Antigen Peptide (MAP) systems introduced by Tam [Tam *et.al*., 1990]. This system represents a novel approach to anti-peptide antibody production. It is built on a resin which bears a core of radial branching lysine dendrites on which a number of copies of a given peptide antigen can be synthesized. Lysine derivatives have been used for the solid phase synthesis of lysine cores suitable for the assembly of antigenic peptides. These peptides have found applications in raising antibodies and in the preparation of synthetic vaccines. On a lysine core several different epitopes of a protein or of different proteins can be assembled to create the required antigenic synthetic protein. Following assembly of the peptide on the MAP core, the peptide is deprotected and cleaved from the support using standard techniques, yielding a highly immunogenic macromolecular structure without the need for conjugation to a carrier protein. The MAP approach has been shown to yield higher antibody titers than using monomeric peptide-conjugates. Alternatively, the two epitopes can be synthesized on alternate branches of the lysine core, using Boc and Fmoc chemistry. T-cell and B-cell epitopes can also be combined sequentially within a single linear sequence.

Another challenging strategy in the immunotherapy of MS or other autoimmune diseases is the use of peptide poly-lysine analogues conjugated to reduced mannan via KLH linker in its oxidized or reduced form to develop Th1/Th2 responses followed by release of appropriate cytokines [Apostolopoulos, *et al*., 1995, Apostolopoulos, *et al*., 1996, Apostolopoulos, *et al*., 1998, Lofthouse, *et al*., 1997]. The aim of this approach is the development of an immunotherapeutic approach for prevention or control of disease. Presently, antigen peptides from MBP, PLP and MOG proteins conjugated to mannan in its oxidized or reduced form are under investigation. In order to study their effect on cellular proliferation, antibody production and cytokines secretion assays were determined in Lewis rats, mouse models and in human peripheral blood T-cells. These studies include the development of recently rationally designed constrained cyclic antagonist peptide analogues based on MBP epitope 87-99 which suppresses the development of clinical E.A.E., CNS inflammation and demyelination. The use of oxidized or reduced mannan to develop a Th1 or Th2 response (and the appropriate cytokines) to myelin peptides expressed in MS constitutes a novel strategy for the treatment of disease.

**Role of Myelin Basic Protein (MBP)**

Although the pathology of MS remains unclear, there is evidence that T cells recognizing encephalitogenic epitopes of myelin, such as Myelin Basic Protein (MBP), play a pathogenic role in the induction of MS. Studies have shown that T cell responses in patients are associated with the recognition of the 81-105 region of MBP (QDENPVVHFFKNIVTPRTPPPSQGK) (Valli et al., J Clin. Invest. 1993), and with highest affinity and binding to HLA-DR2 for the peptide epitope $MBP_{83-99}$ (ENPVVHFFKNIVTPRTP). T cell recognition of this region of MBP has also been shown in healthy individuals, but at relatively low precursor frequencies. The binding of $MBP_{83-99}$ to HLA-DR2 is via hydrophobic V87 and F90 residues, whilst, H88, F89, and K91 are TCR contact residues.

The pathogenic role of autoimmune T cells recognizing encephalitogenic epitopes of MBP has also been noted in experimental autoimmune encephalomyelitis (EAE), an animal model for MS. Similar clinical and histopathological features to MS can be induced in susceptible mouse strains by immunization of myelin components. EAE is mediated by CD4+ T cells of the Th1 phenotype (IFN$\gamma$). Similar to MS, EAE susceptibility is dependent on the MHC background of the mouse and different peptides are immunogenic and induce EAE in different mouse strains. Myelin oligodendrocyte glycoprotein (MOG) residues 35-55 induce chronic EAE in C57BL/6 mice, MBP residues 74-85 induce acute EAE in Lewis rats and proteolipid protein (PLP) residues 139-153 induce acute EAE in SJL/J mice (Zamvil et al., Annu Rev Immunol. 1990). The SJL/J mouse strain (H-$2^s$ haplotype), is commonly used for EAE, since numerous histopathological, clinical and immunological features resemble that of human MS, more similarly compared to other mouse or rat strains (Sommer et al., J Neuroimmunol, 1997). The SJL/J mouse does not express the H2-E-$\alpha$ chain, thus, H2-$A^s$ (I-$A^s$) is the only functional MHC class II molecule in the SJL/J mouse. In the SJL/J mouse strain residues from the encephalitogenic epitope $NMP_{81-100}$ have been shown to bind with high affinity. In fact the minimum epitope required for binding is $MBP_{83-99}$ (Kalbus et al., Eur J Immunol. 2001). Thus, the $MBP_{83-99}$ peptide may act as a lead peptide for the design of altered peptide ligands and peptide analogues, which could be used to alter T cell responses in the SJL/J mouse model and may aid in new therapeutic approaches in autoimmune diseases.

The present invention seeks to provide altered peptide ligands and conjugates thereof which have potential therapeutic applications in the treatment of autoimmune disorders, and in particular, multiple sclerosis.

**STATEMENT OF THE INVENTION**

A first aspect of the invention relates to a peptide comprising the amino acid sequence of formula (I),

ENPVVHFFK$^{91}$NIVTP$^{96}$RTP (I)

wherein $K^{91}$ is substituted by an amino acid selected from A, R, E, F and Y and $P^{96}$ is substituted by the amino acid A, wherein said peptide is in linear or cyclic form.

A second aspect of the invention relates to a peptide comprising the amino acid sequence of formula (Ib),

ENPVVHFFK$^{91}$NIVTP$^{96}$RTP (Ib)

wherein at least one of $K^{91}$ and $P^{96}$ is substituted by an amino acid selected from R, E, F and Y.

A third aspect of the invention relates to a peptide consisting of the amino acid sequence of formula (IIb),

VHFFK$^{91}$NIVTP$^{96}$RTP (IIb)

wherein $K^{91}$ is substituted by the amino acid A and $P^{96}$ is substituted by the amino acid A, wherein said peptide is cyclised head to tail.

Compared to linear peptides, cyclic peptides have been considered to have greater potential as therapeutic agents due to their increased chemical and enzymatic stability, receptor selectively, and improved pharmacodynamic properties.

A fourth aspect of the invention relates to a conjugate which comprises a peptide as defined above and mannan.

Advantageously, biological assays have demonstrated that specific altered peptide ligands of the epitope $MBP_{83-99}$ ($E^{83}NPVVHFFK^{91}NIVTP^{96}RTP$) with modifications at positions 91 and 96, and which are conjugated to reduced mannan:

- Divert immune responses from Th1 to therapeutic Th2 in SJL/J mice, i.e. generate useful cytokines such as IL4, IL10 and not at all destructive interferon gamma (IFNγ);
- GenerateIgG and in particular IgG1 antibodies which do not cross react with the whole MBP native protein;
- Suppress guinea pig MBP 74-85 induced EAE in Lewis rats after three times vaccination with reduced mannan-KLH-$MBP_{83-99}$;
- Suppress $MOG_{35-55}$ induced chronic EAE in C57BL/6 mice;
- The cyclo(83-99)$MBP_{83-99}$ peptide alone (or conjugated to mannan) increases stability to the proteolysis compared to linear counterparts.

A fifth aspect of the invention relates to a peptide or conjugate as described above for use in medicine.
A sixth aspect of the invention relates to the use of a peptide or a conjugate as described above in the preparation of a medicament for treating an immune disorder.
A seventh aspect of the invention relates to a pharmaceutical composition comprising a peptide or a conjugate as defined above, admixed with a pharmaceutically acceptable diluent, excipient or carrier.
An eighth aspect of the invention relates to the use of a peptide or a conjugate as described above in an assay for elucidating agents capable of regulating experimental autoimmune encephalomyelitis (EAE) or regulating multiple sclerosis.
A ninth aspect of the invention relates to a process for preparing a conjugate as described above, said process comprising the steps of:

(i) reacting a peptide as defined above with a Keyhole Limpit Hemocyanin (KLH);
(ii) reacting the product formed in step (i) with oxidized mannan; and
(iii) reducing the product formed in step (ii) to form a reduced mannan conjugate.

A tenth aspect of the invention relates to a peptide or conjugate as described above for use in the treatment of an autoimmune disease.
Another aspect of the invention relates to a peptide or a conjugate as described above for use in diverting the immune response in a subject from a Th1 response to a Th2 response, or inducing a Th2-specific immune response in a subject.
Another aspect of the invention relates to a vaccine composition comprising a peptide or a conjugate as described above and a suitable adjuvant.
Similarly, a further aspect relates to the use of a peptide or a conjugate as described above in the preparation of a vaccine composition.

**DETAILED DESCRIPTION**

The present invention relates to variants of the MBP peptide, and more specifically to variants of particular fragments of the MBP peptide, namely the $MBP_{83-99}$ peptide and the $MBP_{87-99}$ peptide. polar etc. Non-homologous substitution may also occur i.e. from one class of residue to another or alternatively involving the inclusion of unnatural amino acids such as ornithine (hereinafter referred to as Z), diaminobutyric acid ornithine (hereinafter referred to as B), norleucine ornithine (hereinafter referred to as O), pyridylalanine, thienylalanine, naphthylalanine and phenylglycine, a more detailed list of which appears below. Within each peptide more than one amino acid residue may be modified at a time.
As used herein, amino acids are classified according to the following classes;
basic; H, K, R
acidic; D, E
non-polar; A, F, G, I, L, M, P, V, W
polar; C, N, Q, S, T, Y,
(using the internationally accepted single letter amino acid notation)
and homologous and non-homologous substitution is defined using these classes. Thus, homologous substitution is

used to refer to substitution from within the same class, whereas non-homologous substitution refers to substitution from a different class or by an unnatural amino acid.

Suitable spacer groups that may be inserted between any two amino acid residues of the peptide include alkyl groups such as methyl, ethyl or propyl groups in addition to amino acid spacers such as glycine or β-alanine residues. A further form of variation, type (e), involving the presence of one or more amino acid residues in peptoid form, will be well understood by those skilled in the art. For the avoidance of doubt, "the peptoid form" is used to refer to variant amino acid residues wherein the α-carbon substituent group is on the residue's nitrogen atom rather than the α-carbon. Processes for preparing peptides in the peptoid form are known in the art, for example Simon RJ et al., PNAS (1992) 89(20), 9367-9371 and Horwell DC, Trends Biotechnol. (1995) 13(4), 132-134. Type (f) modification may occur by methods such as those described in International Application PCT/GB99/01855.

The peptide of the present invention may comprise amino acids in the L or D form, i.e. one or more residues, preferably all the residues may be in the L or D form.

One aspect of the invention relates to a peptide comprising the amino acid sequence of formula (I),

$$ENPVVHFFK^{91}NIVTP^{96}RTP \qquad (I)$$

wherein $K^{91}$ is substituted by an amino acid selected from A, R, E, F and Y and $P^{96}$ is substituted by the amino acid A, wherein said peptide is in linear or cyclic form.

In a more preferred embodiment, $K^{91}$ is substituted by an amino acid selected from A, E and Y.

In one highly preferred embodiment, $K^{91}$ is substituted by the amino acid Y.

In one preferred embodiment, $K^{91}$ is substituted by the amino acid R and $P^{96}$ is substituted by the amino acid A.

In another preferred embodiment, $K^{91}$ is substituted by the amino acid A and $P^{96}$ is substituted by the amino acid A.

In one particularly preferred embodiment, the peptide of the invention is selected from the following sequences:

| | |
|---|---|
| $[R^{91},A^{96}]MBP_{83-99}$ | ENPVVHFFRNIVTARTP |
| $[A^{91},A^{96}]MBP_{83-99}$ | ENPVVHFFANIVTARTP |

$[A^{91},A^{96}]MBP_{83-99}$ is particularly preferred.

In another embodiment, the peptide of the invention comprises the amino acid sequence of formula (Ib),

$$ENPVVHFFK^{91}NIVTP^{96}RTP \qquad (Ib)$$

wherein at least one of $K^{91}$ and $P^{96}$ is substituted by an amino acid selected from R, E, F and Y.

In one preferred embodiment, the peptide consists of the amino acid sequence of formula (Ib), wherein at least one of $K^{91}$ and $P^{96}$ is substituted by an amino acid selected from R, E, F and Y.

In one highly preferred embodiment, $K^{91}$ is substituted by an amino acid selected from R, E, F and Y.

More preferably still, the peptide of formula (Ib) is selected from the following:

| | |
|---|---|
| $[E^{91}]MBP_{83-99}$ | ENPVVHFFENIVTPRTP |
| $[F^{91}]MBP_{83-99}$ | ENPVVHFFFNIVTPRTP |
| $[Y^{91}]MBP_{83-99}$ | ENPVVHFFYNIVTPRTP |

Peptide $[Y^{91}]MBP_{83-99}$ is particularly preferred, linear or cyclic.

In one preferred embodiment the peptide of formula (I) or (Ib) is a linear peptide

Preferably, the peptide of formula (I) or (Ib) is selected from the above-mentioned sequences, each of which is attached to mannan, preferably reduced mannan.

Based on the minimum binding peptide $MBP_{83-91}$) to I-$A^s$, the applicants have designed and synthesized analogues by mutating the residue at position $K^{91}$ with alanine (A), glutamic acid (E), arginine (R), phenylalanine (F) and tyrosine (Y), in order to determine if immune responses can be altered from Th1 to Th2. $K^{91}$ was mutated as this is one of the principal TCR contact residues when bound to HLA-DR2 and has been predicted to also be a TCR contact residue when bound to H2 I-$A^s$ (Kalbus et al, Eur J Immunol. 2001).

## CYCLIC PEPTIDES

In one preferred embodiment, the peptide of the invention is in cyclic form. Compared to linear peptides, cyclic

peptides have been considered to have greater potential as therapeutic agents due to their increased chemical and enzymatic stability, receptor selectively, and improved pharmacodynamic properties.

Preferably, the peptide consists of the amino acid sequence of the invention cyclised head to tail.

In one particularly preferred embodiment, the cyclic peptide is selected from the following:

| peptide analogues | sequence |
|---|---|
| | cyclo(83-99)E N P V V H F F K N I V T P R T P |
| | cyclo(83-99)E N P V V H F F A N I V T P R T P |
| cyclo(83-99)[$R^{91}$]$MBP_{83-99}$ | cyclo(83-99)E N P V V H F F R N I V T P R T P |
| cyclo(83-99)[$F^{91}$]$MBP_{83-99}$ | cyclo(83-99)E N P V V H F F F N I V T P R T P |
| cyclo(83-99)[$Y^{91}$]$MBP_{83-99}$ | cyclo(83-99)E N P V V H F F Y N I V T P R T P |
| cyclo(83-99)[$E^{91}$]$MBP_{83-99}$ | cyclo(83-99)E N P V V H F F E N I V T P R T P |
| cyclo(83-99)[$A^{91}$,$A^{96}$]$MBP_{83-99}$ | cyclo(83-99)E N P V V H F F A N I V T A R T P |
| cyclo(83-99)[$R^{91}$,$A^{96}$]$MBP_{83-99}$ | cyclo(83-99)E N P V V H F F R N I V T A R T P |
| cyclo(83-99)[$F^{91}$,$A^{96}$]$MBP_{83-99}$ | cyclo(83-99)E N P V V H F F F N I V T A R T P |
| cyclo(83-99)[$Y^{91}$,$A^{96}$]$MBP_{83-99}$ | cyclo(83-99)E N P V V H F F Y N I V T A R T P |
| | cyclo(83-99)E N P V V H F F S N I V T A R T P |

In the above nomenclature, (83-99) denotes the site of cyclisation. For example, amino acid residue 83 is joined to residue 99; i.e. the peptide is cyclised "head-to-tail".

Cyclo(83-99)[$Y^{91}$]$MBP_{83-99}$ is particularly preferred.

The cyclization of $MBP_{83-99}$ peptide and analogues was pursued as a result of conformation studies in linear $MBP_{87-99}$ indicating a cyclic structure. In particular, design of the cyclic analogues of Human MBP epitope 83-99 was based on Nuclear Magnetic Resonance and Molecular Dynamics. These studies revealed a head to tail intramolecular proximity (ROE connectivity between $\zeta$NHLys$^{91}$-nNHArg$^{97}$) indicating a pseudocyclic conformation for the immunogenic peptide $MBP_{87-99}$. Similarly NMR studies of [Arg$^{91}$, Ala$^{96}$]$MBP_{87-99}$ (antagonist-altered peptide ligand) revealed a head to tail intramolecular interaction (ROE connectivity between $\alpha$Val$^{87}$-$\beta$Thr$^{98}$, $\alpha$Val$^{87}$-$\gamma$Thr$^{98}$).

Cyclization of amino acid sequences results in increased metabolic stability, potency, receptor selectivity and bioavailability all of them reflecting a better pharmacological profile [Scott, *et al.*, 1999, Oligino, *et al.*, 1997]. In particular, cyclic peptides have been used in several cases as synthetic immunogens [Bruggle, *et al.*, 1999], potent vaccine for diabetes [Berezhkovskiy, *et al.*, 1999], antigens for Herpes Simplex Virus [Mezo, *et al.*, 1999], transmembrane ion channels [Chaloin *et al.*, 1999], inhibitors of HIV-1 Tat-TAR interactions in human cells [Tamilarasu *et.al.*, 2000], of $\alpha$-amylase, pancreatic tripsin and as protein stabilizer [Iwai, *et al.*, 1999].

Furthermore, advantages of cyclic analogues over their linear counterparts include:

(i) the cyclic analogues are more stable molecules and thus more resistant to enzymatic degradation, a quality that makes them attractive candidates as drug leads;

(ii) it is an intermediate step towards the rational design and development of a non-peptide drug for oral administration, which is the ultimate goal of this work and technology; and

(iii) the conformation of the cyclic analogues is fixed compared to the conformational flexibility characterizing the linear counterparts. The active conformation of the potent linear peptides, which are very important for further drug development, has been identified through combined SAR, NMR and dynamics studies.

To date, this is the first time that cyclic analogues of MBP epitopes have been synthesized and shown to prevent the development of EAE.

In one particularly preferred embodiment of the invention, the peptide is of formula A, in which N to C termini (or head-to-tail) cyclo(83-99)$MBP_{83-99}$ analogues are substituted at positions 91 and/or 96 in accordance with formula (I) or (Ib) as described above, which are the TCR contact sites.

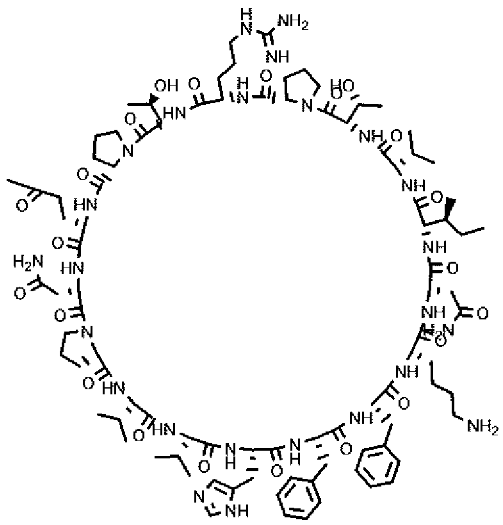

**Formula A**

**Cyclic analogues of human $MBP_{83-99}$ epitope inducing and suppressing the development of MBP EAE in Lewis rats**

The $MBP_{72-85}$ peptide (25μg) induced an acute monophasic disease with a peak clinical score at day 13 after the initial injection, followed by complete recovery in all animals by day 18. Co-injection of [$Arg^{91}$, $Ala^{96}$]$MBP_{87-99}$ (500μg) with the potent agonist peptide $MBP_{72-85}$ (25μg) completely prevented the development of EAE. Further modification and cyclization resulted in two cyclic antagonist peptides. The Lys side chain and C-terminus amide-linked cyclic analogue, cyclo(91-99)[$Ala^{96}$] $MBP_{87-99}$ (Val-His-Phe-Phe-$Lys^{91}$-Asn-Ile-Val-Thr-$Ala^{96}$-Arg-Thr-$Pro^{99}$) had low inhibitory activity in the EAE system while the amide-linked cyclic analogue, cyclo(87-99)[$Arg^{91}$, $Ala^{96}$] $MBP_{87-99}$ was a strong inhibitor of EAE when co-administered with $MBP_{72-85}$. Co-injection of any cyclic analogue (500μg) with $MBP_{72-85}$ (25μg) did not inhibit EAE activity. However all cyclic analogues except cyclo(1-5)$Phe^1$-Ala-Arg-Gln-$Acp^5$ resulted in delay of onset of EAE (from day 13 to day 15) but not its severity.

**Cyclic analogues of human $MBP_{83-99}$ epitope as an experimental preventive treatment in MOG EAE in C57BL/6 mice.**

Linear $MOG_{35-55}$ injection in C57BL/6 mice produces a chronic disease with a single relapse at the beginning of the disease (acute phase) followed by an incomplete remission which leads to chronic phase with a residual disease. This chronic EAE is produced by injection of 600μg of $MOG_{35-55}$, administered in 2 doses of 300μg each and 2 days apart. Co-injection of the cyclic $MBP_{83-99}$ peptide at disease induction at 1:1 ratio seems to offer a mild preventive benefit which is not always constant and depends on the statistical sample size and variance. On the other hand, histopathology reveals that cyclic $MBP_{83-99}$ is reducing some of the basic histopathological effects that are responsible for the clinical picture of the animals.

**ANALOGUES OF $MBP_{87-99}$**

Another aspect of the invention relates to a peptide consisting of the amino acid sequence of formula (IIb),

VHFFK$^{91}$NIVTP$^{96}$RTP (IIa)

wherein $K^{91}$ is substituted by the amino acid A and $P^{96}$ is substituted by the amino acid A, wherein said peptide is cyclised head to tail.

In one highly preferred embodiment, the peptide of formula (IIa) consists of the sequence VHFF**A**NIVT**A**RTP cyclised head to tail, i.e. cyclization between residues 87-99.

## CONJUGATES

Another aspect of the invention relates to a conjugate which comprises a peptide as described above and mannan. The mannan (oxidized or reduced) may be attached at any suitable site on the peptide. The conjugate may contain more than one mannan residue and more than one peptide according to the invention (each of which may be the same or different).

Preferably, the mannan is reduced mannan.

Thus, in one preferred embodiment, the invention relates to a peptide as described above conjugated to reduced mannan.

Mannan has been used as a successful carrier to target peptides to the macrophage/dendritic cell mannose receptor. Upon binding, MHC class I or MHC class II presentation of peptides is generated, stimulating either CTL/Ab or Th1/Th2 immune responses. Preliminary results suggest that conjugations of reduced mannan to cyclic antagonist/agonist peptides are more potent than cyclic analogues alone. Further investigations are being done to measure cytokines and T cells after immunization of oxidized/reduced mannan conjugates to cyclic MBP analogues.

Th1 cytokines released after therapeutic administration are associated with exacerbation of MS. However, Th2 cytokines (such as IL-4 and IL-10) have antiinflammatory properties and down-regulate Th1 responses. Mannan has been investigated extensively for its ability to generate responses in several model systems. Its adjuvant function has been shown to stem from its ability to target the mannose receptor on antigen presenting cells. Mice immunized with mannan-MUC1 protein are protected against a MUC1 expressing tumor challenge, as well as reversing established tumors in mice. Similar results were observed in MUC1 transgenic mice. Either a Th1 response (IL-2, IFN-$\gamma$, IL-12, TNF-$\alpha$ and IgG2a antibodies) or Th2 response (no IFN-$\gamma$ or IL-12, but significant amounts of IL-4, IL-10 and TGF-$\beta$. and IgG1 antibodies), depending on the mode of conjugation, and oxidized/reduced mannan respectively. Other cytokines, IL-5, IL-6, IL-13, IL-15, and IL-18 have also been measured with either oxidized or reduced mannan immunogens. In addition to Th1/Th2 type responses to MUC1 in mice, similar responses have been demonstrated in humans and monkeys with MUC1 protein and to an *Anaplasma marginale* MSP-1 peptide in cows. The use of reduced mannan to further divert immune responses to Th2 when conjugated to MBP peptides constitutes a novel strategy for immunotherapy of the disease.

The present applicants have demonstrated that mutated $MBP_{83-99}$ peptide analogues conjugated to reduced mannan diverts immune responses from Th1 to Th2 in SJL/J mice and generate antibodies which do not cross react with native MBP protein. $[A^{91}]MBP_{83-99}$, $[E^{91}]MBP_{83-99}$ and $[Y^{91}]MBP_{83-99}$ peptides gave the best cytokine and antibody reactivity profile with $[Y^{91}]MBP_{83-99}$ being the most promising as a therapeutic peptide against MS.

As used herein, to "divert immune responses from Th1 to Th2" means an increased production of at least one of the Th2-type cytokines, including, without limitation, IL-4, IL-5, IL-6, IL-9, IL-10 and IL-13 and/or a decreased production of at least one of the Th1-type cytokines, including, without limitation, IL-2, TNF$\alpha$ and IFN$\gamma$, as compared to the levels of these cytokines prior to peptide/conjugate administration, i.e. in the absence of the treatment.

In another highly preferred embodiment, the invention relates to the peptide $[Y^{91}]MBP_{83-99}$ or $[A^{91},A^{96}]MBP_{83-99}$ conjugated to reduced mannan.

In one preferred embodiment, the peptide is linked to the reduced mannan via a linker group.

Preferably, the linker group is a Keyhole Limpit Hemocyanin (KLH).

Keyhole Limpet Hemocyanin (KLH) is a copper-containing protein isolated from the mollusck, Megathura crenulata. It is a potent immunoactivator commonly used as a carrier in the conjugation of peptides for antibody production, and $CD4^+$ helper responses. It is composed of high and low molecular weight monomeric forms. KLH is used as a linker between mannan and peptide. KLH-peptide is conjugated to KLH via glutaraldehyde. Conjugation occurs via Schiff base formation between free amino groups of KLH and oxidized mannan. The oxidized mannan-KLH-peptide complex is then reduced with sodium borohydride to form reduced mannan-KLH-peptide complex. In addition, KLH provides $CD4^+$ help to further initiate immune responses. Reduced mannan, is able to divert immune responses from Th1 to Th2.

Preferably, mannan is oxidized to a polyaldehyde by treating with sodium periodate. Ethanedithiol is then added to stop oxidation. The mixture is passed through a PD-10 column pre-calibrated with carbonate buffer and the oxidized mannan fraction is collected. For the conjugation of peptide analogues to oxidized mannan, each peptide pre-conjugated to KLH using glutaraldehyde is allowed to react with oxidized mannan overnight at room temperature. Oxidized mannan contains aldehydes and Schiff bases (Figure 2). For conjugation of peptide analogues to reduced mannan (reducing the aldehydes to alcohol's and Schiff bases to amines): oxidized mannan peptide analogue complexes (from above) are reacted with sodium borohydride for 3 hours at room temperature. The conjugation (reduced mannan-peptide-KLH) is used without further purification (Figure 2).

A further aspect of the invention relates to a process for preparing a conjugate, said process comprising the steps of:

(i) reacting a peptide as defined above with a Keyhole Limpit Hemocyanin (KLH);
(ii) reacting the product formed in step (i) with oxidized mannan; and
(iii) reducing the product formed in step (ii) to form a reduced mannan conjugate.

There is gathering evidence that analogues of disease-associated epitopes can be conjugated to oxidized or reduced mannan via KLH linker and actively generate antigen specific regulatory $CD4^+/CD8^+$ T cells and Th1/Th2 cytokines. The ability to alter the cytokine secretion of autoreactive T cell lines using peptides or peptide mimetic treatment even in longstanding autoimmune disease indicates that cytokine therapy might have therapeutic benefits by switching the function of myelin autoreactive T cells which are non-pathogenic. Oxidized or reduced mannan conjugates of myelin antigens are promising to induce appropriate cytokine secretion.

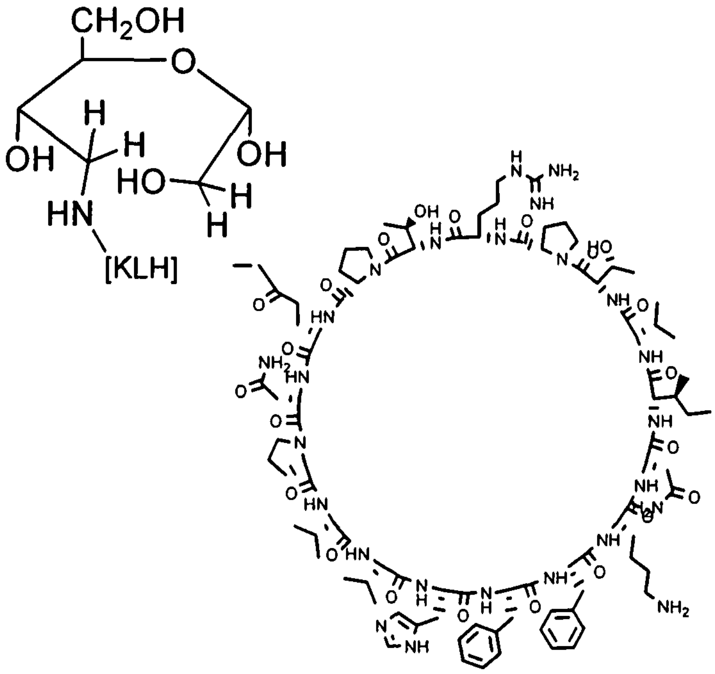

## Formula C

In one particularly preferred embodiment of the invention, the conjugate is of formula D, in which a cyclo(83-99)$N4BP_{83-99}$ peptide is optionally substituted at positions 91 and/or 96 and conjugated to mannan in its oxidized form.

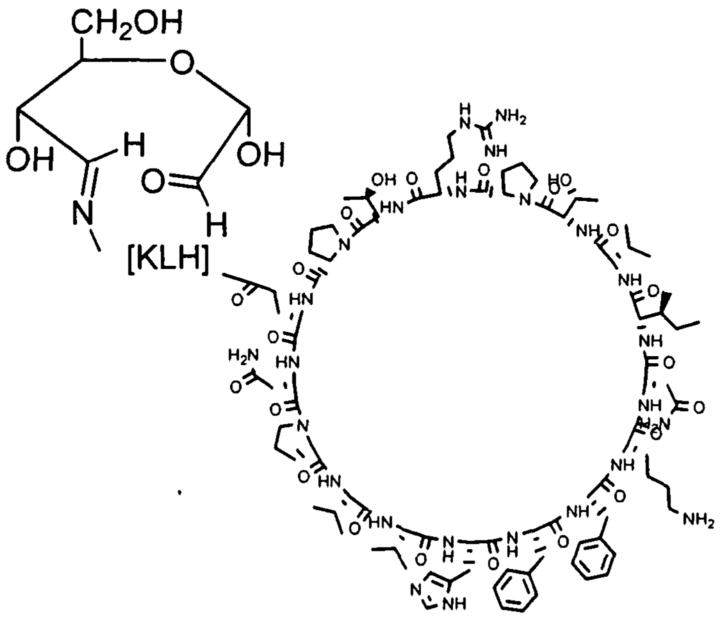

**Formula D**

## THERAPEUTIC APPLICATIONS

Another aspect of the invention relates to a peptide or conjugate as described above for use in medicine.

Yet another aspect relates to the use of a peptide or conjugate of the invention in the preparation of a medicament for treating an immune disorder.

In one preferred embodiment, the immune disorder is an autoimmune disease.

In one particularly preferred embodiment, the disorder is multiple sclerosis (MS).

Multiple Sclerosis (MS) is an autoimmune demyelinating disease mediated primarily by $CD4^+$ T cells of the Th1 subset. Candidate autoantigens include constituents of the myelin sheath - Myelin Basic Protein (MBP), Proteolipid Protein (PLP) and Myelin Oligodendrocyte Glycoprotein (MOG). Modern approaches toward the therapeutic management of MS involve the design and use of peptide analogues of disease-associated myelin epitopes to induce peripheral T-cell tolerance. The present invention provides a number of peptide ligands altered by mutating the principal TCR contact residues based on $MBP_{83-99}$ epitope. Experiments by the applicant have demonstrated that mutated $MBP_{83-99}$ peptide analogues conjugated to reduced mannan unexpectedly divert immune responses from Th1 to Th2 in SJL/J mice and generate antibodies which do not cross react with native MBP protein. $[A^{91}]MBP_{83-99}$, $[E^{91}]MBP_{83-99}$ and $[Y^{91}]MBP_{83-99}$ peptides gave the best cytokine and antibody reactivity profile with $[Y^{91}]MBP_{83-99}$ being the most promising as a therapeutic peptide against MS.

Furthermore, cyclic $MBP_{83-99}$ analogues suppressed chronic EAE induced by the linear $MOG_{35-55}$ and also immunization with linear $MBP_{83-99}$-KLH-reduced mannan conjugates prevented EAE induction.

In another preferred embodiment, the disorder is experimental autoimmune encephalomyelitis (EAE).

Structure-activity studies based on the human $MBP_{83-99}$ [Vergeli, *et al* 1996, Brocke, *et al* 1996] epitope (Glu-Asn-Pro-Val-Val-His-Phe-Phe-Lys-Asn-Ile-Val-Thr-Pro-Arg-Thr-Pro) resulted in linear and cyclic analogues namely $[Arg^{91},Ala^{96}]MBP_{87-99}$ (Val-His-Phe-Phe-$Arg^{91}$-Asn-Ile-Val-Thr-$Ala^{96}$-Arg-Thr-Pro) and cyclo(87-99)$[Arg^{91},Ala^{96}]MBP_{87-99}$ ($Val^{87}$-His-Phe-Phe-$Arg^{91}$-Asn-Ile-Val-Thr-$Ala^{96}$-Arg-Thr-$Pro^{99}$) which completely prevented the induction of EAE when co-injected to Lewis rats together with encephalitogenic agonist $MBP_{72-85}$. Blockade of $MBP_{72-85}$ induced EAE by the previous unrelated peptides could indicate that the mechanism of inhibition is not due to binding competition but rather due to the delivery of a negative signal by the antagonist which overcomes the agonist response possibly through the activation of antigen specific regulatory T cells.

Preferably, the peptide or conjugate is to be administered in an amount sufficient to cause suppression of $MOG_{35-55}$-induced chronic experimental autoimmune encephalomyelitis (EAE).

Preferably the peptide or conjugate is to be administered in an amount sufficient to inversely modulate the Th1 and Th2 responses of lymphocytes in a subject so as to increase the Th2 response and decrease the Th1 response respectively above and below the levels prevailing without said treatment.

Immunological assays to measure specific T cell responses to antigens can be carried out by ELISpot analysis to detect the secretion of specific cytokines such as IFN-$\gamma$, IL-4 or IL-10. Further details of these assays may be found in the accompanying Examples section.

Preferably, the peptide or conjugate is to be administered in an amount sufficient to enhance/induce the Th2-type response in a subject, as compared to the Th2-type response prior to peptide/conjugate administration (i.e. in the absence of treatment) and reduce the Th1-type response as compared to the Th1-type response prior to peptide/conjugate administration.

Another aspect of the invention relates to a peptide or conjugate as described above for use in diverting the immune response in a subject from a Th1 response to a Th2 response.

In one embodiment, the peptide or conjugate selectively inhibits the Th1 immune response over the Th2 immune response in a subject.

In one embodiment, the peptide or conjugate selectively increases the Th2 immune response in a subject relative to the Th1 immune response.

In one embodiment, the peptide or conjugate selectively reduces the Th1 response of lymphocytes in a subject relative to the Th2 response of lymphocytes.

Yet another aspect relates to a peptide or conjugate as described above for use in inducing a Th2-specific immune response in a subject.

In one embodiment, the peptide or conjugate reduces the level of IFN$\gamma$ in a subject.

Another aspect relates to the use of a peptide or a conjugate as described above for use in treating a disorder associated with an imbalance in lymphokine expression.

The lymphokines are a group of polypeptides belonging to the family of cytokines, i.e. hormone-like molecules that can affect various cell functions and enable communication between different cells. Recent developments have helped to clarify the role of lymphokines in the immune response. Lymphokine production by helper CD4 (and also in CD8+) T cells frequently fall into one of two phenotypes, Th1 and Th2, in both murine and human systems (Romagnani. 1991, Immunol Today 12: 256-257, Mosmann. 1989, Annu Rev Immunol, 7: 145 173). Th1 cells produce interleukin 2 (IL-2), tumor necrosis factor (TNF$\alpha$) and interferon gamma (IFN$\gamma$) and they are responsible primarily for cell-mediated immunity such as delayed type hypersensitivity. Th2 cells produce interleukins, IL-4, IL-5, IL-6, IL-9, IL-10 and IL-13 and are primarily involved in providing optimal help for humoral immune responses such as IgE and IgG4 antibody isotype switching (Mosmann, 1989, Annu Rev Immunol, 7: 145-173). Strongly polarized Th1 and Th2 responses not only play different roles in protection, they can promote different immunopathological reactions.

Another aspect relates to the use of a peptide or a conjugate as described above in the preparation of a medicament for treating a disorder associated with an imbalance in lymphokine expression.

Preferably, the peptide or conjugate is to be administered in an amount sufficient to inversely modulate the Th1 and Th2 responses of lymphocytes in the subject so as to increase the Th2 response and decrease the Th1 response respectively above and below the levels prevailing without said treatment.

In one preferred embodiment, the peptide or conjugate is to be administered in an amount sufficient to enhance/induce the Th2-type response in a subject, as compared to the Th2-type response prior to peptide/conjugate administration (i.e. in the absence of treatment) and reduce the Th1-type response as compared to the Th1-type response prior to peptide/conjugate administration.

In another preferred embodiment, the peptide or conjugate is to be administered in an amount sufficient to inversely modulate the Th1 and Th2 responses of lymphocytes in the subject so as to increase the Th2 response and decrease the Th1 response respectively above and below the levels prevailing without said treatment.

In one highly preferred embodiment, the disorder associated with an imbalance in lymphokine expression is an inflammatory autoimmune disease with a Th1 immune profile.

## PHARMACEUTICAL COMPOSITION

Another aspect relates to a pharmaceutical composition comprising a peptide or a conjugate according to the invention, admixed with a pharmaceutically acceptable diluent, excipient or carrier.

Even though the peptide/conjugate of the present invention (including their pharmaceutically acceptable salts, esters and pharmaceutically acceptable solvates) can be administered alone, they will generally be administered in admixture with a pharmaceutical carrier, excipient or diluent, particularly for human therapy. The pharmaceutical compositions may be for human or animal usage in human and veterinary medicine.

Examples of such suitable excipients for the various different forms of pharmaceutical compositions described herein may be found in the "Handbook of Pharmaceutical Excipients, 2nd Edition, (1994), Edited by A Wade and PJ Weller.

Acceptable carriers or diluents for therapeutic use are well known in the pharmaceutical art, and are described, for example, in Remington's Pharmaceutical Sciences, Mack Publishing Co. (A. R. Gennaro edit. 1985).

Examples of suitable carriers include lactose, starch, glucose, methyl cellulose, magnesium stearate, mannitol,

sorbitol and the like. Examples of suitable diluents include ethanol, glycerol and water.

The choice of pharmaceutical carrier, excipient or diluent can be selected with regard to the intended route of administration and standard pharmaceutical practice. The pharmaceutical compositions may comprise as, or in addition to, the carrier, excipient or diluent any suitable binder(s), lubricant(s), suspending agent(s), coating agent(s), solubilising agent(s).

Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol.

Examples of suitable lubricants include sodium oleate, sodium stearate, magnesium stearate, sodium benzoate, sodium acetate, sodium chloride and the like. Preservatives, stabilizers, dyes and even flavoring agents may be provided in the pharmaceutical composition. Examples of preservatives include sodium benzoate, sorbic acid and esters of p-hydroxybenzoic acid. Antioxidants and suspending agents may be also used.

## SALTS/ESTERS

The peptides/conjugates of the invention can be present as salts or esters, in particular pharmaceutically acceptable salts or esters.

Pharmaceutically acceptable salts of the peptides/conjugates of the invention include suitable acid addition or base salts thereof. A review of suitable pharmaceutical salts may be found in Berge et al, J Pharm Sci, 66, 1-19 (1977). Salts are formed, for example with strong inorganic acids such as mineral acids, e.g. sulphuric acid, phosphoric acid or hydrohalic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as ($C_1$-$C_4$)-alkyl- or aryl-sulfonic acids which are unsubstituted or substituted (for example, by a halogen) such as methane- or p-toluene sulfonic acid.

Esters are formed either using organic acids or alcohols/hydroxides, depending on the functional group being esterified. Organic acids include carboxylic acids, such as alkanecarboxylic acids of 1 to 12 carbon atoms which are unsubstituted or substituted (e.g., by halogen), such as acetic acid; with saturated or unsaturated dicarboxylic acid, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as ($C_1$-$C_4$)-alkyl- or aryl-sulfonic acids which are unsubstituted or substituted (for example, by a halogen) such as methane- or p-toluene sulfonic acid. Suitable hydroxides include inorganic hydroxides, such as sodium hydroxide, potassium hydroxide, calcium hydroxide, aluminium hydroxide. Alcohols include alkanealcohols of 1-12 carbon atoms which may be unsubstituted or substituted, e.g. by a halogen).

## ENANTIOMERS/TAUTOMERS

In all aspects of the present invention previously discussed, the invention includes, where appropriate all enantiomers and tautomers of the peptides/conjugates. The man skilled in the art will recognise compounds that possess an optical properties (one or more chiral carbon atoms) or tautomeric characteristics. The corresponding enantiomers and/or tautomers may be isolated/prepared by methods known in the art.

## STEREO AND GEOMETRIC ISOMERS

Some of the peptides/conjugates of the invention may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers, and mixtures thereof. The terms used in the claims encompass these forms, provided said forms retain the appropriate functional activity (though not necessarily to the same degree).

The present invention also includes all suitable isotopic variations of the peptides/conjugates or pharmaceutically acceptable salts thereof. An isotopic variation is defined as one in which at least one atom is replaced by an atom having the same atomic number but an atomic mass different from the atomic mass usually found in nature. Examples of isotopes that can be incorporated into the peptides/conjugates and pharmaceutically acceptable salts thereof include isotopes of hydrogen, carbon, nitrogen, oxygen, phosphorus, sulphur, fluorine and chlorine such as $^{2}H$, $^{3}H$, $^{13}C$, $^{14}C$, $^{15}N$, $^{17}O$, $^{18}O$, $^{31}P$, $^{32}P$, $^{35}S$, $^{18}F$ and $^{36}Cl$, respectively. Certain isotopic variations of the peptides/conjugates and pharmaceutically acceptable salts thereof, for example, those in which a radioactive isotope such as $^{3}H$ or $^{14}C$ is incorporated, are useful in drug and/or substrate tissue distribution studies. Tritiated, i.e., $^{3}H$, and carbon-14, i.e., $^{14}C$, isotopes

are particularly preferred for their ease of preparation and detectability. Further, substitution with isotopes such as deuterium, i.e., $^{2}H$, may afford certain therapeutic advantages resulting from greater metabolic stability, for example, increased *in vivo* half-life or reduced dosage requirements and hence may be preferred in some circumstances. Isotopic variations of the peptides/conjugates of the present invention and pharmaceutically acceptable salts thereof of this invention can generally be prepared by conventional procedures using appropriate isotopic variations of suitable reagents.

**SOLVATES**

The present invention also includes the use of solvate forms of the peptides/conjugates of the present invention. The terms used in the claims encompass these forms.

**POLYMORPHS**

The invention furthermore relates to the peptides/conjugates of the present invention in their various crystalline forms, polymorphic forms and (an)hydrous forms. It is well established within the pharmaceutical industry that chemical compounds may be isolated in any of such forms by slightly varying the method of purification and or isolation form the solvents used in the synthetic preparation of such compounds.

**PRODRUGS**

The invention further includes the peptides/conjugates of the present invention in prodrug form. Such prodrugs are generally peptides/conjugates wherein one or more appropriate groups have been modified such that the modification may be reversed upon administration to a human or mammalian subject. Such reversion is usually performed by an enzyme naturally present in such subject, though it is possible for a second agent to be administered together with such a prodrug in order to perform the reversion in vivo. Examples of such modifications include ester (for example, any of those described above), wherein the reversion may be carried out be an esterase etc. Other such systems will be well known to those skilled in the art.

**ADMINISTRATION**

The pharmaceutical compositions of the present invention may be adapted for oral, rectal, vaginal, parenteral, intramuscular, intraperitoneal, intraarterial, intrathecal, intrabronchial, subcutaneous, intradermal, intravenous, nasal, buccal or sublingual routes of administration.

For oral administration, particular use is made of compressed tablets, pills, tablets, gellules, drops, and capsules.

Other forms of administration comprise solutions or emulsions which may be injected intravenously, intraarterially, intrathecally, subcutaneously, intradermally, intraperitoneally or intramuscularly, and which are prepared from sterile or sterilisable solutions. The pharmaceutical compositions of the present invention may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

Compositions may be formulated in unit dosage form, i.e., in the form of discrete portions containing a unit dose, or a multiple or sub-unit of a unit dose.

**DOSAGE**

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention.

## COMBINATIONS

In a particularly preferred embodiment, one or more peptides/conjugates of the invention are administered in combination with one or more other therapeutically active agents, for example, existing drugs available on the market. In such cases, the compounds of the invention may be administered consecutively, simultaneously or sequentially with the one or more other agents.

Combination therapy is desirable in order to avoid an overlap of major toxicities, mechanism of action and resistance mechanism(s). Furthermore, it is also desirable to administer most drugs at their maximum tolerated doses with minimum time intervals between such doses. The major advantages of combining chemotherapeutic drugs are that it may promote additive or possible synergistic effects through biochemical interactions and also may decrease the emergence of resistance in early tumor cells which would have been otherwise responsive to initial chemotherapy with a single agent.

## ASSAY

Another aspect relates to the use of a peptide or a conjugate according to the invention, in an assay for elucidating agents capable of regulating experimental autoimmune encephalomyelitis (EAE) or regulating multiple sclerosis.

## VACCINES

Another aspect of the invention relates to a vaccine composition comprising a peptide or a conjugate as described above. Preferably, the vaccine composition further comprises one or more adjuvants. The term "adjuvant" as used herein includes an agent having the ability to enhance the immune response of a vertebrate subject's immune system to an antigen or antigenic determinant.

The preparation of vaccines which contain an immunogenic polypeptide as active ingredient will be familiar to one skilled in the art. Typically, such vaccines are prepared as injectables, either as liquid solutions or suspensions; solid forms suitable for solution in, or suspension in, liquid prior to injection may also be prepared. The preparation may also be emulsified, or the protein encapsulated in liposomes. The active immunogenic ingredients are often mixed with excipients which are pharmaceutically acceptable and compatible with the active ingredient. Suitable excipients are, for example, water, saline, dextrose, glycerol, ethanol, or the like and combinations thereof.

In addition, if desired, the vaccine may contain minor amounts of auxiliary substances such as wetting or emulsifying agents, pH buffering agents, and/or adjuvants which enhance the effectiveness of the vaccine. Examples of adjuvants which may be effective include but are not limited to: aluminum hydroxide, N-acetyl-muramyl-L-threonyl-D-isoglutamine (thr-MDP), N-acetyl-nor-muramyl-L-alanyl-D-isoglutamine (CGP 11637, referred to as nor-MDP), N-acetylmuramyl-L-alanyl-D-isoglutaminyl-L-alanine-2-(1'-2'-dipalmitoyl-sn-glycero-3-hydroxyphosphoryloxy)-ethylamine (CGP 19835A, referred to as MTP-PE), and RIBI, which contains three components extracted from bacteria, monophosphoryl lipid A, trehalose dimycolate and cell wall skeleton (MPL+TDM+CWS) in a 2% squalene/Tween 80 emulsion.

Further examples of adjuvants and other agents include aluminum hydroxide, aluminum phosphate, aluminum potassium sulfate (alum), beryllium sulfate, silica, kaolin, carbon, water-in-oil emulsions, oil-in-water emulsions, muramyl dipeptide, bacterial endotoxin, lipid X, *Corynebacterium parvum* (*Propionobacterium acnes*), *Bordetella pertussis*, polyribonucleotides, sodium alginate, lanolin, lysolecithin, vitamin A, saponin, liposomes, levamisole, DEAE-dextran, blocked copolymers or other synthetic adjuvants. Such adjuvants are available commercially from various sources, for example, Merck Adjuvant 65 (Merck and Company, Inc., Rahway, N.J.) or Freund's Incomplete Adjuvant and Complete Adjuvant (Difco Laboratories, Detroit, Michigan).

Typically, adjuvants such as Amphigen (oil-in-water), Alhydrogel (aluminum hydroxide), or a mixture of Amphigen and Alhydrogel are used.

The proportion of immunogen and adjuvant can be varied over a broad range so long as both are present in effective amounts. For example, aluminum hydroxide can be present in an amount of about 0.5% of the vaccine mixture ($Al_2O_3$ basis). Conveniently, the vaccines are formulated to contain a final concentration of immunogen in the range of from 0.2 to 200 μg/ml, preferably 5 to 50 μg/ml, most preferably 15 μg/ml.

The effectiveness of an adjuvant may be determined by measuring the amount of antibodies directed against an immunogenic agent resulting from administration of this agent in vaccines which are also comprised of the various adjuvants.

The vaccines are conventionally administered parenterally, by injection, for example, either subcutaneously or intramuscularly. Additional formulations which are suitable for other modes of administration include suppositories and, in some cases, oral formulations. For suppositories, traditional binders and carriers may include, for example, polyalkylene

glycols or triglycerides; such suppositories may be formed from mixtures containing the active ingredient in the range of 0.5% to 10%, preferably 1% to 2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10% to 95% of active ingredient, preferably 25% to 70%. Where the vaccine composition is lyophilised, the lyophilised material may be reconstituted prior to administration, e.g. as a suspension. Reconstitution is preferably effected in buffer.

Another aspect of the invention relates to the use of a peptide or a conjugate as described above in the preparation of a vaccine composition.

The present invention is further described by way of the following non-limiting examples, and with reference to the figures below wherein:

Figure 1 is a schematic representation of synthesis of cyclic peptides.

Figure 2 is a schematic representation of the conjugation between $MBP_{83-99}$-KLH peptides and oxidised mannan and reduction of the complex to result in reduced mannan-$MBP_{83-99}$-KLH conjugates used for immunization.

Figure 3 shows IFN-γ responses in SJL/J mice immunized with $MBP_{83-99}$ peptide analogues. Immunizations were given twice intradermally of 50μg peptide conjugated to reduced mannan and 14 days post-immunization spleen cells were isolated for ELISpot analysis. IFN-γ responses are shown as spot forming units (sfu) per $0.5x10^6$ cells. The data is representative of two experiments with three mice per group.

Figure 4 shows IL-4 responses in SJL/J mice immunized with $MBP_{83-99}$ peptide analogues. Immunizations were given twice intradermally of 50μg peptide conjugated to reduced mannan and 14 days post-immunization spleen cells were isolated for ELISpot analysis. IL-4 responses are shown as spot forming units (sfu) per $0.5x10^6$ cells. The data is representative of two experiments with three mice per group.

Figure 5 shows (a) IL-10 responses in SJL/J mice immunized with $[Y^{91}]MBP_{83-99}$ or $MBP_{83-99[}F^{91}]$ peptide analogues. Immunizations were given twice intradermally of 50μg peptide conjugated to reduced mannan and 14 days post-immunization spleen cells were isolated for ELISpot analysis. IL-10 responses are shown as spot forming units (sfu) per $0.5x10^6$ cells. No peptide was used as a negative control (grey bars) and recall peptide is shown as black bars; (b) Proliferation of spleen cells in response to recall peptide $[F^{91}]MBP_{83-99}$ or (c, d) $[Y^{91}]MBP_{83-99}$ after immunization of SJL/J mice with reduced mannan-KLH-$[F^{91}]MBP_{83-99}$ or reduced mannan-KLH-$[Y^{91}]MBP_{83-99}$ respectively; (d) ConA removed from figure. ConA was used as an internal positive control and no peptide as negative control. The data is representative of three different mouse experiments.

Figure 6 shows SJL/J mice immunized with (a) linear or cyclic $MBP_{83-99}$ peptides or (b) mutant $MBP_{83-99}$ peptide analogues $[[A^{91}]MBP_{83-99}$, cyclo(83-99)$[A^{91}]MBP_{83-99}$, $[E^{91}]MBP_{83-99}$, $[F^{91}]MBP_{83-99}$, $[A^{91}$, $R^{96}]MBP_{83-99}$ and $[Y^{91}]MBP_{83-99}]$. Total IgG antibody levels were measured by ELISA coating with each respective peptides conjugated to BSA; (c) IgG1, IgG2a, IgGM antibody subclass; (d) Total IgG antibody levels were measured coating with native bovine MBP protein. The data is representative of two experiments. Individual mouse curves are shown.

Figure 7 shows a schematic representation for the synthesis of cyclic peptides.

Figure 8 shows IFN-γ antagonism. Mice were immunized with native $MBP_{83-99}$ peptide emulsified in CFA, and spleen cells were isolated 25 days later. The ability of cyclic mutant $MBP_{83-99}$ peptide analogues to antagonize IFN-γ production was assessed by ELISpot analysis. The native $MBP_{83-99}$ peptide was added together with cyclic mutated $MBP_{83-99}$ analogues, and % IFN-γ inhibition is shown.

Figure 9 shows (A) IFN-γ responses and (B) IL-4 responses in SJL/J mice immunized with linear agonist $MBP_{83-99}$, linear antagonist $[A^{91}]MBP_{83-99}$, cyclo(83-99)$MBP_{83-99}$ and cyclo(83-99)$[A^{91}]MBP_{83-99}$ peptide analogues emulsified in CFA. IFN-γ or IL-4 responses are shown as SFU (SEM per 0.5 million cells minus background (negative control). (C) Total IgG antibody levels were measured by ELISA coating with each respective peptide conjugated to KLH. (D) Mice were immunized with native $MBP_{83-99}$ peptide emulsified in CFA and proliferation of spleen cells determined in response to recall peptides. Data are shown as mean counts per minute (cpm) of triplicate wells (SEM over 6 days. Results are representative of two experiments with three mice per group.

Figure 10 shows (A) IFN-γ responses and (B) IL-4 responses in SJL/J mice immunized with linear (agonist) MBP83-99,

linear mutant (antagonist) [A91]MBP83-99, cyclo(83-99)MBP83-99 and mutant cyclo(83-99)[A91]MBP83-99 peptide analogues conjugated to reduced mannan. IFN-$\gamma$ or IL-4 responses are shown as SFU (SEM per 0.5 million cells minus background (negative control). (C) IL-4 responses in SJL/J mice immunized with cyclo(83-99)$MBP_{83-99}$ and cyclo(83-99)[$A^{91}$]$MBP_{83-99}$ peptide analogues conjugated to reduced mannan. (D) Total IgG antibody levels were measured by ELISA coating with each respective peptides conjugated to BSA. Results are representative of two experiments with three mice per group.

Figure 11 shows models of MHC class II I-$A^s$ with bound MBP peptide ligands. MHC binding grooves are shown as ribbons ($\alpha$-chain, pale green; $\beta$-chain, grey) with the bound peptides as stick representations: (a) MBP wild type ($K^{91}$) peptide in cyan; (b) [$R^{91}$, $A^{96}$]$MBP_{87-99}$ mutant analog in magenta; (c) [$A^{91}$, $A^{96}$]$MBP_{87-99}$ mutant analog in orange. (d) Peptides are shown as overlays with the corresponding MBP residue positions (87-96) indicated.

Figure 12 shows (a) IFN-$\gamma$ and (b) IL-4 responses in SJL/J mice immunized with either $MBP_{87-99}$ or [$R^{91}$, $A^{96}$]$MBP_{87-99}$ peptides. Immunization with 50 $\mu$g peptide emulsified in CFA (black bar) or conjugated to reduced mannan (white bar). Negative (background levels) are indicated as grey bar. IFN-$\gamma$ or IL-4 responses are shown as spot forming units (SFU) per 0.5 million cells $\pm$ standard error of mean. Representative of 2 experiments with 3 mice per group. **($p<0.01$).

Figure 13 shows IFN-$\gamma$ responses in SJL/J mice immunized with either $MBP_{87-99}$ or [$A^{91}$, $A^{96}$]$MBP_{87-99}$ peptides. (a) Immunization with 50$\mu$g peptide emulsified in CFA and (b) immunization with 50 $\mu$g peptide conjugated to reduced mannan. IFN-$\gamma$ responses are shown as spot forming units (SFU) per 0.5 million cells $\pm$ standard error of mean. Representative of 2 experiments with 3 mice per group.

Figure 14 shows IL-4 responses in SJL/J mice immunized with either $MBP_{87-99}$ or [$A^{91}$, $A^{96}$]$MBP_{87-99}$ peptides. (a) Immunization with 50$\mu$g peptide emulsified in CFA and (b) immunizations with 50 $\mu$g peptide conjugated to reduced mannan. IL-4 responses are shown as spot forming units (SFU) per 0.5 million cells $\pm$ standard error of mean. Representative of 2 experiments with 3 mice per group.

Figure 15 shows IFN-$\gamma$ production by T cells from SJL/J mice immunized with the mutant peptide [$A^{91}$, $A^{96}$]$MBP_{87-99}$ (a) emulsified in CFA or (b) conjugated to reduced mannan. Recall peptides were either $MBP_{87-99}$, [$A^{91}$, $A^{96}$]$MBP_{87-99}$ or [$R^{91}$, $A^{96}$]$MBP_{87-99}$ or no peptide (negative) (x-axis). IFN-$\gamma$ production is shown as spot forming units (SFU) / 0.5 million cells $\pm$ standard error of mean. Representative of 2 experiments with 3 mice per group.

Figure 16 shows SJL/J mice immunized with (a) $MBP_{87-99}$ or [$A^{91}$, $A^{96}$]$MBP_{87-99}$ peptide analogs emulsified in CFA, or, (b) $MBP_{87-99}$ or [$A^{91}$, $A^{96}$]$MBP_{87-99}$ peptide analogs conjugated to reduced mannan. Total IgG antibody levels were measured by ELISA coating with each respective peptides conjugated to BSA or KLH. (c,d) Cross-reactive IgG antibody levels were measured coating with native $MBP_{87-99}$ peptide and using sera from SJL/J mice immunized with $MBP_{87-99}$ or [$A^{91}$, $A^{96}$]$MBP_{87-99}$ peptides (c) emulsified in CFA or (d) conjugated to reduced mannan. Error bars depict standard error of the mean. Representative of 2 experiments with 3 mice per group is shown.

Figure 17 shows (a) Analytical RP-HPLC of purified linear [$A^{91}$, $A^{96}$]$MBP_{87-99}$ analog after its purification by semi-preparative RP-HPLC and lyophilization. Column: Nucleosil C18, 250x4.6 mm, 5 $\mu$m. $T_R$: 17.3 min. Conditions: gradient 5% (B)-100% (B), in 35 min, flow rate 1 ml/min. [Eluents (A): Solution TFA in $H_2O$ 0.08% (v/v), (B): Solution TFA in AcN 0.08% (v/v)]. (b) ESI-MS of linear [$A^{91}$, $A^{96}$]$MBP_{87-99}$ analog. $M^+$:1473.31, $M+2H^+/2$:737.29.

Figure 18 shows hydrogen bonding interactions in the modeled complexes of $MBP_{87-96}$ peptide and mutant analogs with H2 I-$A^s$.

Figure 19 shows Van der Waals interactions in the modeled complexes of $MBP_{87-96}$ peptide and mutant analogs with H2 I-$A^s$.

Figure 20 shows IFN-$\gamma$ (A, B) and IL-4 (C, D) responses in SJL/J mice immunized with either $MBP_{87-99}$ or cyclo(87-99)[$A^{91}$, $A^{96}$]$MBP_{87-99}$ peptide analogs. (A, C) Immunization was given once of 50 $\mu$g peptide emulsified in complete Freund's adjuvant and 28 days post-immunization spleen cells were isolated for ELISpot analysis. (B, D) Immunizations were given twice intradermally of 50 $\mu$g peptide conjugated to reduced mannan-KLH and 14 days post-immunization spleen cells were isolated for ELISpot analysis. IFN-$\gamma$ and IL-4 responses (background was subtracted for IL-4 responses) are shown as spot forming units (SFU) per 0.5 million cells +/- standard error of the mean of triplicate wells. Representative of two experiments with three mice per group (**$p<0.01$)

Figure 21 shows SJL/J mice were immunized with, (A) $MBP_{87-99}$ or cyclo(87-99)[$A^{91}$, $A^{96}$]$MBP_{87-99}$ peptide analogs emulsified in complete Freund's adjuvant, or, (B) $MBP_{87-99}$ or cyclo(87-99)[$A^{91}$, $A^{96}$]$MBP_{87-99}$ peptide analogs conjugated to reduced mannan. Spleen cells were isolated for ELISpot analysis and were cultured with native $MBP_{87-99}$ peptide *in vitro.* IFN-$\gamma$ responses are shown as spot forming units (SFU) per 0.5 million cells +/- standard error of the mean of triplicate wells. Representative of two experiments with three mice per group

Figure 22 shows SJL/J mice were immunized with, (A) $MBP_{87-99}$ or cyclo(87-99)[$A^{91}$, $A^{96}$]$MBP_{87-99}$ peptide analogs emulsified in complete Freund's adjuvant, or, (B) $MBP_{87-99}$ or cyclo(87-99)[$A^{91}$, $A^{96}$]$MBP_{87-99}$ peptide analogs conjugated to reduced mannan-KLH. Total IgG antibody levels were measured by ELISA coating with each respective peptides conjugated to BSA or KLH. (C) Total IgG antibody levels were measured coating with native $MBP_{87-99}$ peptide and using sera from SJL/J mice immunized with $MBP_{87-99}$ or cyclo(87-99)[$A^{91}$, $A^{96}$]$MBP_{87-99}$ emulsified in complete Freund's adjuvant. (D) Total IgG antibody levels were measured coating with native $MBP_{87-99}$ peptide and using sera from SJL/J mice immunized with $MBP_{87-99}$ or cyclo(87-99)[$A^{91}$, $A^{96}$]$MBP_{87-99}$ conjugated to reduced mannan-KLH. Representative of two experiments with three mice per group.

## EXAMPLE 1

## Materials and Methods

### Solid-phase peptide synthesis of linear and cyclic analogues

Peptides (Table 1) were prepared on 2-chlorotrityl chloride resin (CTLR-Cl) using Fmoc/tBu methodology. The cyclization was achieved with TBTU/HOAt and 2,4,6-collidine as base, as previously described. Preparative HPLC for peptide analogues were performed using a Lichrosorb RP-18 reversed phase semipreparative column with 7$\mu$m packing material. The peptides were >95% pure as analysed by mass spectrometry.

**Table 1:** $MBP_{83-99}$ peptide analogues used in this study

| | Sequence | Peptide analogues |
|---|---|---|
| P1 | ENPVVHFFKNIVTPRTP | $MBP_{83-99}$ |
| P2 | cyclo(83-99)ENPVVHFFKNIVTPRTP | cyclo(83-99)$MBP_{83-99}$ |
| P3 | ENPVVHFFANIVTPRTP | [$A^{91}$]$MBP_{83-99}$ |
| P4 | cyclo(83-99)ENPVVHFFANIVTPRTP | cyclo(83-99)[$A^{91}$]$MBP_{83-99}$ |
| P5 | ENPVVHFFENIVTPRTP | [$E^{91}$]$MBP_{83-99}$ |
| P6 | ENPVVHFFFNIVTPRTP | [$F^{91}$]$MBP_{83-99}$ |
| P7 | ENPVVHFFRNIVTARTP | [$R^{91}$,$A^{96}$]$MBP_{83-99}$ |
| P8 | ENPVVHFFYNIVTPRTP | [$Y^{91}$]$MBP_{83-99}$ |
| P9 | ENPVVHFFANIVTARTP | [$A^{91}$,$A^{96}$]$MBP_{83-99}$ |

### Examples of Cyclization

Cyclization of linear $MBP_{83-99}$ protected analogue was achieved using O-benzotriazol-1-yl-N,N,N',N'-tetramethyluronium tetrafluoroborate (TBTU) and 1-hydroxy-7-azabenzotriazole, 2,4,6 collidine in DMF solution, allowing fast reactions and high yield cyclization products. Recent results indicate that HOAt, a 4-nitrogen containing variant, is a very effective coupling additive, more efficient than HOBt for solution or solid phase synthesis leading in high yield products.

### Synthesis of human MBP cyclic peptides

Cyclo(83-99)[$Arg^{91}$, $Ala^{96}$] $MBP_{83-99}$ (Glu-Asn-Pro-Val-$Val^{87}$-His-Phe-Phe-$Arg^{91}$-Asn-Ile-Val-Thr-$Ala^{96}$-Arg-Thr-$Pro^{99}$)

Cyclo(91-99)[$Ala^{96}$] $MBP_{83-99}$ (Glu-Asn-Pro-Val-$Val^{87}$-His-Phe-Phe-$Lys^{91}$-Asn-Ile-Val-Thr-$Ala^{96}$-Arg-Thr-$Pro^{99}$)

Human MBP cyclic analogues were prepared on 2-chlorotrityl chloride resin using Fmoc/tBu methodology. The peptide synthesis was achieved using DIC/HOBt in DMF and the $N^{\alpha}$-$NH_2$ of amino acids was protected with the Fmoc group. The side chain of peptides was protected as following: Trt for His, Pbf for Arg, tBu for Ser, Thr, Asp, Glu, Boc for Lys, as regarding the cyclic analogue cyclo(91-99)[$Ala^{96}$] $MBP_{83-99}$ (by $N\varepsilon$-$NH_2$ of Lys and C-terminous) Mtt protected

group was used, because it can easily be removed using mixture HFIP(1,1,1,3,3,3 hexafluoro-2-propanol)/DCM (2/8), which cleaves peptides from the resin. Otherwise, the side chain of Lys of the cyclo(87-99)[$Arg^{91}$, $Ala^{96}$]$MBP_{87-99}$ was protected with Boc group. The final protected linear peptides on resin were dried in vacuo and then treated with the splitting mixture DCM/HFIP (8/2) for 7h at room temperature to release the peptide from the resin and deprotect Lys from Mtt of the cyclo(91-99)[$Ala^{96}$] $MBP_{83-99}$. Each one of the linear protected peptide was dissolved in DMF and collidine/HOAt was added. This mixture was added dropwise in a solution of TBTU in DMF for 8 hours. The cyclization was determined by TLC and analytical reversed phase HPLC (RP-HPLC). The solvent was removed under reduced pressure affording a light yellow oily residue. The cyclic protected peptide (purity ≥90%) was precipitated from $H_2O$ and dried in vacuo for 16h. The cyclic protected peptide was treated with 65% TFA in DCM and 3% ethanodithiol as scavanger for 4 hours at room temperature. The resulting solution was concentrated to a small volume and the final free peptide was precipitated as a light yellow amorphous solid added diethylether (purity ≥80%). Peptide purity was assessed by analytical HPLC reruns, thin layer chromatography (TLC) and mass spectrometry (ESIMS) (Fig. 1).

Conjugation of reduced mannan to $MBP_{83-99}$ peptide analogues via a KLH linker

$MBP_{83-99}$ peptide analogues (Table 1) were conjugated to keyhole limpet hemocyanin (KLH) via glutaraldehyde (Figure 2); KLH acts as a linker between mannan and peptide. 14mg mannan (from Saccharomyces cerevisiae, SIGMA, St Louis, USA) was dissolved in 1ml pH 6.0 sodium phosphate buffer, followed by the addition of 100μl 0.1 M sodium periodate (dissolved in pH 6.0 phosphate buffer) and incubated on ice for 1 hour in the dark. 10 μL ethanediol was added to the mixture and incubated for a further 30 mins on ice. The resultant mixture (oxidized mannan) was passed through a PD-10 column (Sephadex G-25 M column, Amersham Biosciences, Sweden) pre-equilibrated in pH 9.0 phosphate buffer, to exclude out sodium periodate and ethanediol. Oxidized mannan (7.0 mg/ml) was eluted with 2.0 ml of pH 9.0 phosphate buffer, to which 1.0 mg linear or cyclic peptide $MBP_{83-99}$-KLH analogues (Table 1) were added and allowed to react overnight at room temperature in the dark. Conjugation occurs via Schiff base formation between free amino groups of KLH and oxidized mannan (Figure 2). Reduced mannan-KLH-$MBP_{83-99}$ complexes were prepared by adding 1.0 mg sodium borohydride to each mixture for 6-8 hours at RT in the dark and were used without further purification as previously described. Samples were aliquoted and stored at -20° C until used. MBP peptide analogues were previously characterized by capillary electropheresis for conjugation to mannan and by SDS PAGE gel, staining with Coomassie, Silver or Schiff's reagent (data not shown). Peptides were 100% conjugated to reduced mannan.

Mice and immunizations

Female 6-8 week old SJL/J mice used in all experiments, were purchased from Walter and Eliza Hall Institute (VIC Australia) and housed at the Biological Research Laboratory at Burnet Institute at Austin, Heidelberg, Australia. SJL/J mice were immunized twice on days 0, 14 intradermally (base of tail) with 50 μg $MBP_{83-99}$ peptide analogue-KLH-reduced mannan.

Immunological assays

ELISpot analyses specific T cell responses to antigens by detecting the secretion of specific cytokines. Spleen cells from immunized SJL/J mice were isolated 14 days after immunization and assessed by ELISpot for IFN-γ, IL-4 or IL-10 secretion by T cells. IFN-γ ELISpot assay was performed on MultiScreen-IP Filter Plate (MAIP S4510) with hydrophobic PVDF filters (Millipore, UK) while IL-4 and IL-10 ELISpot was performed on MultiScreen-HA Filter Plate (MAHA S4510) with mixed cellulose esters filters (Millipore, UK). MAIP S4510 plates were pre-wet with 50 μl of 70% ethanol, washed 5 times with 200 μl sterile PBS and coated with 70 μl of 5 μg/ml anti-IFN-γ capture antibody, AN18 (Mabtech, Australia) in PBS and left to incubate at 4°C O/N. While 70 μl of 5 μg/ml anti-IL-4 and anti-IL-10 capture antibody (Mabtech, Australia) was added directly to MAHA S4510 plates and incubated at 4°C O/N without 70% ethanol treatment. Following 5 washes with PBS, plates were blocked by adding 200μl of culture media (supplemented with 2.5% FCS) and incubated for 2 hours at 37° C. The blocking media was discarded and 10 μg/ml recall peptides ($MBP_{83-99}$ peptide analogues not conjugated to KLH-reduced mannan) were added into each defined well. Con A (1.0 μg/ml; IL-4 and IFN-γ) or LPS (1.0 μg/ml; IL-10) were used as an internal positive controls and no peptide was used as negative control. Triplicate wells were set up for each condition. 5 x $10^5$ spleen cells in 100μl culture media were seeded into each well and incubated for 18 hours for IFN-γ, 24 hours for IL-4 and 48 hours for IL-10 at 37° C. Plates were washed 5 times PBS/0.05% Tween 20 and 5 times PBS and incubated for 2 hours at RT with anti-murine IFN-γ, IL-4 or IL-10 monoclonal antibody-biotin. Plates were washed and streptavidin-ALP was added at 1.0 μg/ml and incubated for 2.0 hours at RT. Spots of activity were detected using a colorimetric AP kit (Biorad, Hercules, CA USA) and counted using an AID ELISpot plate reader (Autoimmun Diagnostika GmbH, Germany). Data are presented as mean spot forming units (sfu) per 0.5x$10^6$ cells +/- standard error of the mean (SEM).

Proliferation

Spleen cells from immunized SJL/J mice were isolated 14 days after immunization and assessed by T cell proliferation assay. 1 x $10^5$ spleen cells in 100μl culture media were seeded into 96 U-bottom plates and incubated for 1-6 days at 37°C in the presence of recall peptide (10μg/ml), ConA (positive control) or no peptide (negative control). Proliferation was assessed by adding 1 μCi of [$^3$H]-thymidine per well to one plate per time point (days 1-6). Cells were incubated for a further 6 h before harvesting onto glass fiber filters. [$^3$H] uptake was measured using a β-scintillation counter (Top Count Gamma Counter, Packard, USA).

ELISA

Blood was collected and sera isolated from mice prior to and 2 weeks after immunization. $MBP_{83-99}$ peptides conjugated to BSA were coated onto polyvinyl chloride (PVC) microtiter plates at 10 μg/ml or with 2.5 μg/ml native MBP bovine protein in 0.2 M $NaHCO_3$ buffer, pH 9.6, overnight at 4°C and non-specific binding was blocked with 2% BSA for 1.0 hour at room temperature. After washing (0.05%Tween 20/PBS), serial dilutions of sera were added and incubated for a further 2.0 hours at room temperature. The plates were washed and bound antibody was detected using HRP-conjugated sheep anti-mouse antibody (1:1000 in PBS) (Amersham, UK) and developed using 2,2"-azino-di(3-ethylbenzthiazoline) sulphonate (ABTS) (Sigma, UK). Absorption at 405nm was recorded using an ELISA microplate reader.

Induction-inhibition and assessment of Lewis rats MBP-EAE

Inbred Lewis rats bred and maintained in the animal facility of the Hellenic Pasteur Institute were used in all experiments. Female rats (220g) were immunized with linear $MBP_{72-85}$ (30μg) (n=10, as positive control), or $MBP_{72-85}$ (30μg) plus the cyclic analogue c-$Ala^{81}MBP_{72-85}$ (500μg) or [$Arg^{91}$,$Ala^{96}$]$MBP_{87-99}$ (n=5) in 200μl of an emulsion containing equal volumes of peptide diluted in sterile saline and Freund's complete adjuvant (Difco, USA) containing 4 mg/ml heat-killed *M. tuberculosis* (H37Ra) (Difco). Immunization was performed subcutaneously in the two hind foot pads and repeated 7 days later with the same dosage. Rats were examined daily for clinical signs of EAE and scored as following: 0, no clinical disease; 0.5, weight loss; 1, tail weakness; 2, paraparesis of hindlimbs; 3, paraplegia of hindlimbs; 4, paraplegia with forelimb weakness, moribund; 5, death. PBS/CFA-injected animals served as negative controls. For histological analyses, mice were anaesthetized with ether, bled and perfused with PBS (pH 7.4) (PBS) followed by 4% paraformaldehyde in PBS. Spinal cord was dissected out and fixed overnight in 4 % paraformaldehyde in PBS at 4°C before been embedded in paraffin. Paraffin sections were stained with Luxol fast blue and Nuclear Fast Red for visualization of demyelination and inflammation respectively.

Induction-inhibition and assessment of C57/bl mice MOG-EAE

a. Animal handling

Female C57BL/6 pathogen-free mice were purchased from the Hellenic Pasteur Institute, Athens, and housed in the animal facility of the B' Neurological Department of AHEPA University Hospital, Aristotle University Medical School, Thessaloniki, Greece. All experimental procedures were in accordance with the National Institute of Health guidelines. Mice were fed a regular diet and given water without antibiotics.

b. EAE induction and evaluation of clinical disease.

Induction of chronic $MOG_{35-55}$ EAE

Female C57BL/6 female mice, 10 weeks-old, were divided into 2 different groups depending on the peptides used to prepare the emulsion of injection: group A (or control group, n=9) was immunized using the linear myelin oligodendrocyte glycoprotein 35-55 peptide ($MOG_{35-55}$) (Sigma) and group B (n=8) was immunized using a 1:1 mixture of the linear $MOG_{35-55}$ and the cyclo(83-99)$MBP_{83-99}$ peptide analog. Both groups were immunized with subcutaneous injections at the left para-lumbar region of emulsions composed of either 300μg of linear $MOG_{35-55}$ (group A) or 300μg of linear $MOG_{35-55}$ plus 300μg of cyclo(83-99)$MBP_{83-99}$ (group B) emulsified in 200μl of a solution containing 100μl complete Freund's adjuvant (CFA) containing 4 mg/ml Mycobacterium tuberculosis H37RA and 100μl filtered phosphate buffered saline (PBS) (day 0). Additionally, on day 0, both mice groups were also given i.p. 400ng of Pertussis toxin (Sigma) diluted in 0,5ml of filtered PBS and 48 hours later, on day 2, another 200ng of Pertussis toxin diluted again in 0,5ml of PBS. An additional injection of the emulsified peptides, as described previously, was also delivered 7 days later into the right para-lumbar region.

Clinical evaluation

All animals were examined daily and evaluated for clinical signs of disease. The first clinical signs appear on day 12-16 post-immunization, depending on the development and severity of EAE disease. The clinical status of the mice was graded as follows: 0: without clinical disease; 1: flail tail; 2: tail paralysis; 3: hind limb weakness sufficient to impair righting; 4: paraplegia; 5: paraplegia with forelimb paresis or plegia; 6: death from EAE.

Two experimental sets were conducted using exactly the same experimental procedures: the first experiment evaluated the acute phase of the disease and animals were sacrificed at day 17 post disease induction and the second experiment evaluated the chronic clinical course of the disease and thus animals were sacrificed at day 46 post disease induction.

Tissue collection - Histopathology

On day 17 (acute phase of EAE) and day 46 post-EAE-induction (chronic phase of EAE) animals were subjected to transcardial perfusion with 4% paraformaldehyde in PBS. Brains and spinal cords were removed, post-fixed in the same fixative for approximately 20 hours, routinely processed for paraffin embedding and sectioned at 6 $\mu$m. Sections from animals of acute and chronic phases of the disease, were then stained using the following methods: a) a modified Bielschowsky silver impregnation staining method combined with haematoxylin, for the simultaneous evaluation of axonal injury, axonal loss and inflammatory processes in EAE as previously described in detail [Lourbopoulos *et, al.*, 2007]; b) Luxol fast blue staining counterstained with Nuclear fast Red for the detection of demyelinating areas within the CNS of animals, using routine histopathological protocols.

Pathological evaluation was performed under a light microscope (Olympus Axioplan-2) by two blinded investigators and photos were taken using a CCD camera (Nikon). Five randomly selected longitudinal sections per tissue (brains and spinal cords) were evaluated as follows: for each animal, each section was evaluated under 20x or 40x optical fields (depending on the object of study) so as to cover the entire area of the section. Initial study of pathology revealed that spinal cords had the majority of lesions (compared to brains) and thus further detailed study was focused on the spinal cord sections of the animals.

Depending on the object of interest the following scales were used to perform the evaluation: (a) For axonal injury (AI), the following scale was used: 0 = no AI, 1+ = scattered dystrophic injured axons without any spheroid or ovoid, 2+ = mild AI with the presence of at least one spheroid or ovoid, 3+ = moderate AI, 4+ = severe AI; generally injured axons were defined and identified as spheroids and ovoids (which represent axonotmesis), and dilated (dystrophic) axons which represent injured axons not yet being cut. (b) For axonal loss (AL): 0 = normal axonal density, 1+ = <25% AL, 2+ = mild AL (26-50%), 3+ = moderate to severe (51-75%) AL, 4+ = severe AL (>75%); various degrees of decreased density of axons was attributed to axonal loss and evaluated accordingly. (c) For infiltrations: number of infiltrating cells per optical field and number of infiltrating cells per infiltration. (d) Demyelination was evaluated under 40x optical fields; using a prefrontal grid, we measured the area of demyelination and the total area of white matter in each optical field and then subtracted the % of demyelination present in each optical field.

Statistical analysis

Statistical analysis of the data was performed using the SPSS 11.5 software. For scale clinical, histopathological and *in vitro* proliferation data we initially tested their normality using Shapiro-Wilk and Kolmogorov-Smirnov tests in order to assess their validity for student's t-test application. Wherever data were found to violate the normality assumption and a logarithmic transformation could not be applied, we used the non-parametric equivalent Mann-Whitney U test for comparison of the two groups (treated and control in each EAE). Values within the text are expressed as mean$\pm$SD. For nominal or ordinal data a Pearson chi-square test or Fisher's exact test was used for comparison of the two groups, depending on the tables' properties. Kaplan-Meier survival analysis was performed in the clinical course of EAE with "disease onset" as the end-point of the analysis; disease onset was defined as the first day with clinical score of "1". Mean Maximal Score (MMS) was calculated for each group using the maximal scores from each animal of each group. Area Under the Curve (AUC) was calculated for each animal using the formula:

$$AUC = \left(\frac{score_1}{2}\right) + \left(\sum_{i=2}^{k-1} score_i\right) + \left(\frac{score_k}{2}\right)$$

[Fleming, *et. al*., 2005]

**Results**

T cell responses to linear and cyclic $MBP_{83-99}$ eptides conjugate to reduced mannan

The ability of linear $MBP_{83-99}$, $[A^{91}]MBP_{83-99}$, $[E^{91}]MBP_{83-99}$, $[F^{91}]MBP_{83-99}$, $[Y^{91}]MBP_{83-99}$, $[R^{91},A^{96}]MBP_{83-99}$, cyclo(83-99)$MBP_{83-99}$ and cyclo(83-99)$[A^{91}]MBP_{83-99}$ peptides conjugated to reduced mannan, to induce T cell responses after 2 injections was measured using IFN-γ and IL-4 cytokine secretion by ELISpot analysis. Mice immunized with linear and cyclo(83-99)$MBP_{83-99}$ and linear $[F^{91}]MBP_{83-99}$ generated weak IFN-γ secreting T cells; all other peptides were negative (Figure 3). However, high levels of IL-4 were induced to all linear analogues, $[A^{91}]MBP_{83-99}$, $[E^{91}]MBP_{83-99}$, $[F^{91}]MBP_{83-99}$, $[Y^{91}]MBP_{83-99}$, and $(R^{91},A^{96}]MBP_{83-99}$ (Figure 4). Interestingly, linear and cyclic native $MBP_{83-99}$ peptides did not stimulate IL-4 secreting T cells; cyclo(83-99)$[A^{91}]MBP_{83-99}$ was also negative (Figure 4, Table 2). We selected $[Y^{91}]MBP_{83-99}$ and $[F^{91}]MBP_{83-99}$ analogues to immunize mice and measure IL-10 secretion, and both peptide analogues induced moderate levels of IL-10 production by T cells (Figure 5a, Table 2). No peptide was used as a negative control (Figures 3-5) and ConA was used as an internal positive control which consistently induced >1,000 sfu/$0.5x10^6$ cells for both IFN-γ and IL-4 and LPS was used for IL-10 (not shown). Furthermore, immunization of mice with reduced mannan-KLH-$[F^{91}]MBP_{83-99}$ induced high levels of proliferative T cells in a T cell proliferation assay whereas, immunization with reduced mannan-KLH-$[Y^{91}]MBP_{83-99}$ induced very low levels of T cell proliferation (Figure 5b-d, Table 2).

Antibody responses to linear and cyclic $MBP_{83-99}$ peptides conjugated to reduced mannan

The production of total IgG antibody responses in mice immunized with linear $NOP_{83-99}$, $[A^{91}]MBP_{83-99}$, $[E^{91}]MBP_{83-99}$, $[F^{91}]MBP_{83-99}$, $[Y^{91}]MBP_{83-99}$, $[R^{91},A^{96}]MBP_{83-99}$ and cyclo(83-99)$MBP_{83-99}[A^{91}]MBP_{83-99}$ peptides conjugated to reduced mannan, were measured using ELISA. High IgG antibody levels were generated in mice immunized with both linear and cyclo(83-99)$NMP_{83-99}$ peptides (titer >1/25,600) (Figure 6a, Table 2). In addition, high antibody levels were induced to peptides $[E^{91}]MBP_{83-99}$, $[Y^{91}]MBP_{83-99}$, $[R^{91},A^{96}]MBP_{83-99}$ (titer >1/25,600), medium antibody levels to $[A^{91}]MBP_{83-99}$ (titer 1/6,400) and low antibody levels were induced in mice immunized with cyclo(83-99)$[A^{91}]MBP_{83-99}$ and $[F^{91}]MBP_{83-99}$ (titer 1/800) (Figure 6b, Table 2). The subclass of the antibodies induced were IgG1 and not IgM or IgG2a (Figure 6c).

Since high levels of antibodies were generated to $MBP_{83-99}$ peptides and its mutant analogues, we determined whether sera from these mice cross reacted with the native MBP protein. Mice immunized with linear or cyclo(83-99)$MBP_{83-99}$ and $[A^{91}, R^{96}]MBP_{83-99}$, peptides highly cross reacted with native MBP protein, mice immunized with linear or cyclo(83-99)$[A^{91}]MBP_{83-99}$ and $[E^{91}]MBP_{83-99}$ reacted moderately, whereas mice immunized with $[F^{91}]MBP_{83-99}$ or $[Y^{91}]MBP_{83-99}$ did not react with native MBP protein at 1/200 and 1/1,600 sera dilution (Figure 6d, Table 2).

Cyclic MBP83-99 single and double mutant analogues are able to inhibit IFN-γ responses induced by the native MBP83-99 peptide

ELISpot assays are an *ex vivo* 18 h assay that measures IFN-γ secretion by T cells. It does not require expansion of cell cultures, as it detects specifically activated effector cells (both CD4 and CD8 cytokine producing terminal effectors). The sensitivity of the assay is higher than limiting dilution analysis, FACscan analysis, or ELISA methods and can reliably detect precursor frequencies of antigen specific effectors of 1 in every 0.5 million cells. It is therefore an appropriate method to detect antigen specific cells. Here, we measured IFN-γ production generated by the native $MBP_{83-99}$ peptide and assessed the % inhibition (antagonize) of IFN-γ produced in the presence of mutant peptide analogues.

Cyclic head-to-tail $MBP_{83-99}$ single and double mutant analogues were tested for their ability to inhibit IFN-γ responses induced by the native agonist $MBP_{83-99}$ peptide. Mice were immunized with the native agonist $MBP_{83-99}$ peptide emulsified in complete Freund's adjuvant (CFA). Spleen cells were isolated 25 days later, and the ability of cyclic peptides to inhibit (antagonize) IFN-γ production was assessed in in vitro antagonism ELISpot assays (Figure 8). The results are shown as % IFN-γ inhibition of each cyclic mutant peptide analogue, in comparison to the native $MBP_{83-99}$ peptide alone. All cyclic peptide analogues inhibited IFN-γ production between 30% and 90%. The single mutant analogues cyclo(83-99)-$[F^{91}]MBP_{83-99}$ and cyclo(83-99)$[Y^{91}]MBP_{83-99}$ and the double mutant analogue cyclo(83-99)$[Y^{91},A96]MBP_{83-99}$ were found to inhibit IFN-γ between 60% and 80%. However, the single mutant analogue cyclo(83-99)$[A^{91}]MBP_{83-99}$ was the most efficient in inhibiting IFN-γ production induced by the native $MBP_{83-99}$ peptide, up to 92%. On the basis of the ability of cyclo(83-99)$[A^{91}]MBP_{83-99}$ peptide to inhibit (antagonize) IFN-γ production by T cells, we chose to examine its *in vivo* efficacy in SJL/J mice after emulsification in CFA or conjugation to reduced mannan.

Cyclo(83-99)NOP83-99 and cyclo(83- 9)[$A^{91}$]$NMP_{83-99}$ compared to their linear counterparts enhance IL-4 production when emulsified in CFA

The ability of the antagonist cyclo(83-99)[$A^{91}$]$MBP_{83-99}$ and the native agonist cyclo(83-99)$MBP_{83-99}$ analogues to induce T cell responses after one injection, in comparison to linear [$A^{91}$]$MBP_{83-99}$ and linear $NMP_{83-99}$, was determined in SJL/J mice. Spleen cells were isolated 28 days after injection, and ELISpot assay was performed to measure IFN-$\gamma$ and IL-4 secretion (Figure 9A and Figure 9B). Linear and cyclic $MBP_{83-99}$ native agonist peptides induced IFN-$\gamma$responses that were lower in linear and cyclic antagonist [$A^{91}$]$MBP_{83-99}$ mutant analogues (Figure 9A). However, no differences in IFN-$\gamma$ production were noted between linear and cyclic analogues (Figure 9A). Interestingly, linear $MBP_{83-99}$ did not induce IL-4; however, cyclo(83-99)$MBP_{83-99}$ induced IL-4 (Figure 9B). Likewise, linear [$A^{91}$]$MBP_{83-99}$ was not able to generate IL-4; however, cyclo(83-99)[$A^{91}$]$MBP_{83-99}$ did generate IL-4 (Figure 9B). It is clear that cyclization of native agonist $MBP_{83-99}$ and antagonist [$A^{91}$]$MBP_{83-99}$ peptides enhances IL-4 production *in vivo.* No peptide (cells alone) was used as negative control, and ConA was used as an internal positive control that consistently induced > 1000 SFU/0.5 million cells (not shown).

Cvclo(83-99)$MBP_{83-99}$ and cyclo(83-99)[$A^{91}$]$MBP_{83-99}$ compared to their linear counterparts enhance antibody production when emulsified in CFA

The production of specific IgG antibody responses in mice immunized with linear $MBP_{83-99}$, linear [$A^{91}$]$MBP_{83-99}$, cyclo(83-99)$MBP_{83-99}$, and cyclo(83-99)[$A^{91}$]$MBP_{83-99}$ peptides emulsified in CFA was measured using ELISA. Linear $MBP_{83-99}$ peptide did not generate IgG antibodies in SJL/J mice; however, antibodies were generated to cyclo(83-99)$MBP_{83-99}$ (Figure 9C). The mutated linear analogue was able to induce IgG antibody responses that were enhanced by its cyclic counterpart cyclo(83-99)-[$A^{91}$]$MBP_{83-99}$ (Figure 9C). Thus, cyclization of agonist $MBP_{83-99}$ and antagonist [$A^{91}$]$MBP_{83-99}$ enhanced antibody production in SJL/J mice.

Cyclization does not affect T cell proliferation to native $MBP_{83-99}$ peptide

Proliferation assays were used to detect the level of antigen specific T cells by measuring the [3H]thymidine uptake of T cells proliferating in the presence of peptides on days 1 - 6. Spleen cells from mice immunized with linear agonistMBP83-99 peptide were isolated 28 days after immunization and assessed by T cell proliferation assay. T cells proliferated to MBP83-99 peptide, reaching a peak by day 5 of up to 25 000 cpm (Figure 9D). In addition, the $MBP_{83-99}$ specific T cells cross-reacted with linear [$A^{91}$]$MBP_{83-99}$ and mutant cyclo(83-99)[$A^{91}$]$MBP_{83-99}$ peptide analogues (Figure 9D). The cyclo(83-99)[$A^{91}$]$MBP_{83-99}$ peptide proliferated similarly to linear [$A^{91}$]$MBP_{83-99}$ peptide, reaching a peak on day 5 (Figure 9D). ConA (internal control) yielded proliferation of more than 75 000 cpm peaked on day 2 and was excluded from the figures, and no peptide (cells alone) was used as background negative control. Interestingly, although cyclization enhanced IL-4 responses and antibody production, it did not affect the proliferation of $MBP_{83-99}$ specific T cell proliferation.

Reduced mannan conjugated to linear [A91]$MBP_{83-99}$ and cyclo(83-99)[$A^{91}$]$MBP_{83-99}$ analogues enhances IL-4 production

The ability of linear and cyclic agonist $MBP_{83-99}$, and linear and cyclic antagonist [$A^{91}$]$MBP_{83-99}$ peptides conjugated to reduced mannan, to induce T cell responses after two injections was measured using IFN-$\gamma$ and IL-4 cytokine secretion by ELISpot analysis. Mice immunized with linear and cyclic agonist $MBP_{83-99}$ peptides generated IFN-$\gamma$secreting T cells; however, both linear and cyclic antagonist [$A^{91}$]$MBP_{83-99}$ peptides were negative (Figure 10A). Linear $MBP_{83-99}$ and cyclo(83-99)$MBP_{83-99}$ generated weak or no IL-4 cytokine secretion by T cells (Figure 10B). However, very strong IL-4 responses were induced to linear [$A^{91}$]$MBP_{83-99}$ peptide (up to 600 SFU/0.5 million cells) and moderate levels by cyclo(83-99)[$A^{91}$]$MBP_{83-99}$ (up to 70 SFU/0.5 million cells) (Figure 10B and Figure 10C). Thus, [$A^{91}$] mutation to the agonist $MBP_{83-99}$ peptide diverts immune responses from Th1 (IFN-$\gamma$) to Th2 (IL-4) when conjugated to reduced mannan. No peptide (cells alone) was used as negative control, and ConA was used as an internal positive control that consistently induced > 1000 SFU/0.5 million cells (not shown).

Cyclization does not affect antibody responses when cyclic $MBP_{83-99}$ peptide analogues are conjugate to reduced mannan

The production of total IgG antibody responses in mice immunized with linear agonist $MBP_{83-99}$, linear antagonist [$A^{91}$]$MBP_{83-99}$, cyclo(83-99)$MBP_{83-99}$, and cyclo(83-99)[$A^{91}$]$MBP_{83-99}$ peptide analogues, conjugated to reduced mannan, were measured using ELISA. High IgG antibody levels were generated in mice immunized with linear $MBP_{83-99}$ and cyclo(83-99)$MBP_{83-99}$ peptide analogues (titer up to at least 1/25600) (Figure 10D). Lower antibody levels were

induced by $[A^{91}]MBP_{83-99}$ (titers out to 1/640) and very weak antibodies to cyclo(83-99)$[A^{91}]MBP_{83-99}$ peptide analogue (titers out to 1/1600) (Figure 10D).

Clinical course of C57BL/6 MOG EAE

To test the effect of a preventive administration of cyclo(83-99)$MBP_{83-99}$ at EAE induction 2 experimental sets were designed, each one with 2 experimental groups as previously described at materials and methods: the first experiment was designed to study the effect of cyclo(83-99)$MBP_{83-99}$ at the pathological procedures at acute phase of EAE and the second one to study the effect of the peptide up to the chronic phase of EAE.

For the study the acute phase, EAE was induced as described previously. The animals developed a severe and aggressive disease with and with fast and steep disease onset. The developed EAE disease was a highly homogenous one since variability in clinical scores was very low. All animals were sacrificed at day 17, i.e. 5 days post their first clinical sign of EAE (acute phase). The mean clinical score was statistical significant different at days 15, 16 and 17 (Student's t-test, $p<0,05$). No animals died from EAE, in either group, until the day of sacrifice. Mean Maximal Clinical Scores (MMS) were not calculated for this experiment since animals were not allowed to live long enough to reach their maximal scores. On the other hand, mean Areas Under the Curve (mAUC) for each group have been calculated and are significantly different between the 2 groups: group B appeared to have a lower mAUC (group B mAUC=13,32±2,55 compared to control group mAUC=18,042±3,34; Mann-Whitney U test p=0,014). Mean day of disease onset (first definite clinical sign or score "1") was not different between the 2 groups (mean day of disease onset for control = 11,67±1,21 and Group B = 12,00±0,81; Kaplan-Meier survival analysis, log rank p=0,7309). Thus, clinical data from acute phase indicate that $MBP_{83-99}$ preventive co-administration conferred a mild clinical benefit in a severe and aggressive form of MOG-EAE.

For the study the chronic phase of EAE, animals were allowed to survive until day 46 post EAE induction (chronic phase). This second EAE experiment was slightly different from the first one since the developed EAE was a moderate one, with less steep slope of onset. Moreover, this EAE had a higher variation concerning the clinical scores of each animal in either group. Animals that received the cyclo(83-99)$MBP_{83-99}$ peptide had a lower mean clinical score throughout the entire observation period but this failed to reach statistical significance (Student's t-test, $p>0,05$). No animals died from EAE in either group. Mean Maximal Clinical Scores (MMS) were not statistically different between the 2 groups despite a lower MMS of group B (control group MS=3,125±1,45 and group B MS=2,375±1,45; Student's t-test p=0,32); mean Areas Under the Curve (mAUC) for each group were also not significantly different although group B had a lower AUC (group B mAUC=51,08±36,58 compared to control group mAUC=66,54±39,38; Student's t-test p=0,429). Mean day of disease onset (first definite clinical sign or score "1") was not significant different between the 2 groups (mean day of disease onset for control = 18,5±11,23 and Group B = 17,00±11,74; Kaplan-Meier survival analysis, log rank p=0,1925).

Histopathological evaluation

Inflammatory process

Study of inflammatory processes at acute phase of EAE showed no quantitative differences of inflammatory processes the between the control group and group B when the following parameters were evaluated: the number of infiltrating cells per optical field (control group: 309,4±219,9, group B: 290,8±277, 7. Student's t-test, p=0,593) and inflammatory cells per infiltration (control group: 55,75±37,4, group B: 53,72±62,0. Student's t-test, p=0,780).

However, in the experiment of chronic phase, the inflammatory process in the spinal cords of each group were different: both groups reduced there inflammatory burden within their tissues but the residual inflammation was significantly more in the control group as this was depicted by the infiltrating cells per optical field (control group: 33,18±67,03, group B: 10,71±39,0. Student's t-test, p=0,001) and the numbers of inflammatory cells per infiltration (control group: 15,76±28,5, group B: 6,0±15,8. Student's t-test, p=0,000).

These data indicate that the cyclo(83-99)$MBP_{83-99}$ co-administration at disease onset produces a quantitatively similar inflammatory load within the spinal cords of the animals, which does not remain during the chronic phase since we observed significantly less inflammatory cells within the spinal cord parenchyma of the cyclo(83-99)$MBP_{83-99}$ treated animals.

Axonopathy

Study of axonal pathology revealed a mild but statistical significant beneficial effect of cyclo(83-99)$MBP_{83-99}$ treatment on axonal injury and a mild effect on axonal loss at the acute phase of disease (Pearson's chi-square tests for axonal injury and axonal loss respectively: p=0,006 and p=0,000, refer to tables below).

**Acute phase: table of axonal Injury**

| | | | axonal injury per 20x field | | | | |
|---|---|---|---|---|---|---|---|
| | | | 0 | 1+ | 2+ | 3+ | 4+ |
| GROUP | control | % within GROUP | 4,3% | 42,4% | 17,4% | 20,7% | 15,2% |
| | +c.$MBP_{83-99}$ | % within GROUP | 14,3% | 45,1% | 22,3% | 11,3% | 6,0% |

**Acute phase: table of Axonal Loss**

| | | | axonal loss per 20x field | | | | |
|---|---|---|---|---|---|---|---|
| | | | 0 | 1+ | 2+ | 3+ | 4+ |
| GROUP | control | % within GROUP | 4,3% | 22,8% | 29,3% | 30,4% | 13,0% |
| | +c.$MBP_{83-99}$ | % within GROUP | 16,5% | 36,1% | 31,6% | 12,0% | 3,8% |

However, during chronic phase axonal injury was not different between the 2 groups (Fisher's exact test, p=0,104), but axonal loss was found to be less in group B compared to control ((Fisher's exact test, p=0,000).

**Chronic phase: table of axonal Injury**

| | | | axonal injury per 20x field | | | | |
|---|---|---|---|---|---|---|---|
| | | | 0 | 1+ | 2+ | 3+ | 4+ |
| GROUP | control | % within GROUP | 48,7% | 24,7% | 22,7% | 4,0% | 0% |
| | +c.$MBP_{83-99}$ | % within GROUP | 49,3% | 29,0% | 13,0% | 8,0% | 0,7% |

**Chronic phase: table of axonal Loss**

| | | | axonal loss per 20x field | | | | |
|---|---|---|---|---|---|---|---|
| | | | 0 | 1+ | 2+ | 3+ | 4+ |
| GROUP | control | % within GROUP | 24,0% | 24,7% | 31,3% | 16,0% | 4,0% |
| | +c.$MBP_{83-99}$ | % within GROUP | 51,4% | 26,8% | 10,9% | 10,1% | 0,7% |

These data indicate that cyclo(83-99)$MBP_{83-99}$ co-administration at disease onset slightly deviates the axonopathic process within the spinal cords of the animals, resulting in a mild, but statistical significant, reduction of axonal injury during acute phase. This reduced axonal injury is followed by less axonal loss within the white bundles of the spinal cord during the acute phase of EAE and is additionally contributing to less axonal loss during the chronic phase of the disease.

Demyelination

Study of demyelinating process revealed a significant protective effect of the cyclo(83-99)$MBP_{83-99}$ treatment on myelin integrity. Demyelination was minimal in group B during acute and chronic phase of EAE compared to control group which exhibited a high percentage of demyelination of the white matter of spinal cords (Acute phase: control group=20,73$\pm$16,53 and group B=9,58$\pm$10,5 Student's t-test, p=0,000; Chronic phase: control group=14,92$\pm$15,31 and group B=7,65$\pm$11,4 Student's t-test, p=0,000).

**Discussion**

Multiple Sclerosis (MS) is a demyelinating and inflammatory disease of the central nervous system with autoimmune pathology. There is increasing evidence that T cell responses to candidate myelin antigens, such as myelin basic protein (MBP), may play an important role in the pathogenesis of the disease. It has been demonstrated that T cell responses to MBP are associated with preferential recognition of the 83-99 immunodominant region of $MBP_{83-99}$

(ENPVVHFFKNIVTPRTP).

The peptide analogues of the invention are based on the encephalitogenic peptide $MBP_{83-99}$, (ENPVVHFF<u>K</u>$^{91}$NIVT<u>P</u>$^{96}$RTP) with the principal TCR contact residues at positions 91 and/or 96 replaced to give $[A^{91}]MBP_{83-99}$, $[E^{91}]MBP_{83-99}$, $[F^{91}]MBP_{83-99}$, $[Y^{91}]MBP_{83-99}$, $[R^{91},A^{96}]MBP_{83-99}$. Cyclo(83-99)$MBP_{83-99}$ and cyclo$[A^{91}]MBP_{83-99}$ derivatives were also synthesized. Peptides were conjugated to reduced mannan via a KLH linker and injected into mice. Reduced mannan was used as a carrier to divert immune responses to Th2. The use of reduced mannan to divert the immune responses towards Th2 to MBP peptides constitutes a novel strategy for the immunotherapy of MS. The immune responses induced in mice are summarized in Table 2.

**Table 2:** Summary of immune responses induced in SJL/J mice to $MBP_{83-99}$ peptide analogues conjugated to reduced mannan-KLH

| Peptide analogue | IFN-γ | IL-4 | IL-10 | Proliferation | Antibody | Cross reaction with native MBP |
|---|---|---|---|---|---|---|
| <u>Linear</u> | | | | | | |
| $MBP_{83-99}$ | ++ | - | | | +++ | +++ |
| $[A^{91}]MBP_{83-99}$ | - | +++ | | | + | + |
| $[E^{91}]MBP_{83-99}$ | - | +++ | | | +++ | + |
| $[F^{91}]MBP_{83-99}$ | ++ | +++ | ++ | +++ | +/- | |
| $[Y^{91}]MBP_{83-99}$ | - | +++ | ++ | +/- | +++ | |
| $[R^{91}, A^{96}]MBP_{83-99}$ | - | +++ | | | +++ | +++ |
| <u>Cyclic</u> | | | | | | |
| $MBP_{83-99}$ | + | - | | | +++ | +++ |
| $[A^{91}]MBP_{83-99}$ | - | - | | | +/- | + |

- negative; +/- very weak; + weak; ++ moderate; +++ strong

The agonist peptide $MBP_{83-99}$ conjugated to reduced mannan induced high levels of IFN-γ, low levels of IL-4 and generated antibodies which were cross reactive with the native MBP protein. Substitution of $K^{91}$ to $A^{91}$, $E^{91}$ or $Y^{91}$ diverted IFNγ responses to high IL-4 responses in immunized mice. In addition, immunization with reduced mannan-$[Y^{91}]MBP_{83-99}$ analogue induced moderate levels of IL-10 cytokine production and very weak T cell proliferative responses. Immunization with $[A^{91}]MBP_{83-99}$, $[E^{91}]MBP_{83-99}$ or $[Y^{91}]MBP_{83-99}$ analogues generated IgG1 antibodies, and antibodies generated to $[A^{91}]MBP_{83-99}$ and $[E^{91}]MBP_{83-99}$ cross reacted weakly with native MBP protein. Howeveer, antibodies produced to $[Y^{91}]MBP_{83-99}$ peptide did not cross react with native MBP protein. $[F^{91}]MBP_{83-99}$ peptide conjugated to reduced mannan induced IFN-γ, IL-4 and IL-10 responses, strong T cell proliferative responses and weak antibody responses. Double mutations in the peptide analogue $[R^{91}, A^{96}]MBP_{83-99}$ when conjugated to reduced mannan was able to generate high levels of IL-4 and no IFN-γ, however the IgG1 antibodies generated were cross reactive with the native protein. Cyclo(83-99)$MBP_{83-99}$ and cyclo(83-99)$[A^{91}]MBP_{83-99}$ did not induce desired responses. It is clear that $[A^{91}]MBP_{83-99}$, $[E^{91}]MBP_{83-99}$ and $[Y^{91}]MBP_{83-99}$ peptides gave the best cytokine profile with $[Y^{91}]MBP_{83-99}$ being the most promising, (since, in addition to cytokine, had no reactivity to native MBP protein) as a therapeutic peptide against MS.

<u>Reduced mannan is preferred to generate the appropriate immune response</u>

It is of interest that when all peptide analogues were conjugated to oxidized mannan, no IFN-γ or IL-4 responses were generated. However, moderate antibody levels were induced (not shown). Oxidized mannan has been shown to preferentially generate CD8 responses, and thus, was unable to induce responses to the CD4 epitope of $MBP_{83-99}$ and its analogues. In addition, immunization of SJL/J mice with linear $MBP_{83-99}$, $[A^{91}]MBP_{83-99}$, $[E^{91}]MBP_{83-99}$, $[F^{91}]MBP_{83-99}$, $[Y^{91}]MBP_{83-99}$, $[R^{91},A^{96}]MBP_{83-99}$, cyclo(83-99)$MBP_{83-99}$ and cyclo(83-99)$[A^{91}]MBP_{83-99}$ induced either IFN-γ and IL-4 secretion or antibody production. In addition, T cells against the agonist peptide only weakly proliferated in the presence of $[Y^{91}]MBP_{83-99}$ or $[R^{91}, A^{96}]MBP_{83-99}$ analogues and all peptide analogues were able to inhibit (antagonize) IFN-γ production *in vitro* by T cells against the agonist $MBP_{83-99}$ peptide. Together, the cytokine profile, antibody data, proliferation and antagonism experiments indicate that the $[R^{91}, A^{96}]MBP_{83-99}$ peptide analogue shows promise for further therapeutic studies when emulsified in complete Freund's adjuvant (Table 3). As can be seen in the summary table (Table 3), emulsifying all the peptide analogues in complete Freund's adjuvant was able to downregulate IFN-γ secretion but was unable to induce IL-4 cytokine (except $[R^{91}, A^{96}]MBP_{83-99}$), However, when the peptide analogues were conjugated to reduced mannan-KLH very strong IL-4 cytokine and little to no IFN-γ was generated, in addition to IL-10 (Table

2). Thus, conjugating $MBP_{83-99}$ peptide analogues to reduced mannan was able to divert the immune response from Th1 to Th2 and thus constitutes a novel approach to the immunotherapy of MS.

**Table 3:** Summary of immune responses induced in SJL/J mice to $MBP_{83-99}$ peptide analogues emulsified in complete Freund's adjuvant

| Peptide analogue | IFN-γ | IL-4 | Proliferation cross reaction | Antagonism | Antibody | Ab Cross reaction with native MBP |
|---|---|---|---|---|---|---|
| <u>Linear</u> | | | | | | |
| $MAP_{83-99}$ | + | - | | | - | - |
| $[A^{91}]MBP_{83-99}$ | - | - | ++ | ++ | ++ | ++ |
| $[E^{91}]$ $MBP_{83-99}$ | - | - | ++ | ++ | ++ | - |
| $[F^{91}]$ $MBP_{83-99}$ | ++ | - | ++ | ++ | ++ | - |
| $[Y^{91}]MBP_{83-99}$ | | | +/- | ++ | - | - |
| $[R^{91}, A^{96}]MBP_{83-99}$ | ++ | ++ | +/- | ++ | ++ | - |
| <u>Cyclic</u> | | | | | | |
| $MBP_{83-99}$ | + | - | | | ++ | - |
| $[A^{91}]MBP_{83-99}$ | - | - | ++ | ++ | + | - |
| - negative; +/- very weak; + weak; ++ moderate; +++ strong | | | | | | |

<u>Clinical and histopathological effect of co-administrating cyclo(83-99)$MBP_{83-99}$ peptide as a preventive treatment in the chronic model of EAE in C57BL/6 mice.</u>

Clinical data suggest that the preventive co-administration of the cyclo(83-99)$MBP_{83-99}$ peptide in 1:1 ratio has a mild beneficial effect, which is not as strong as the one observed in analogous experiments in Lewis rats' MBP-EAE [Tselios T *et al*., 2002, Tselios, *et.al*., 2000]. Results suggest that the observed benefit in C57BL/6 mice' MOG-EAE is not always statistically significant and is dependent on the clinical characteristics of the produced EAE (inter-group variability, disease severity and aggressiveness).

Despite the fact that the cyclo(83-99)$MBP_{83-99}$ peptide seems to lower the disease severity and clinical score, this effect does not always reach statistical significance. Careful study of both experimental sets (acute and chronic) lead to the conclusion that cyclo(83-99)$MBP_{83-99}$ co-administration with the linear $MOG_{35-55}$ peptide has a mild beneficial effect which is strongly affected by the group size and variance of the clinical scores of each EAE experiment [Fleming, *et. al*., 2005]. Thus, when the induced EAE is highly homogenous and has a sufficient sample size then the mild benefit from the cyclo(83-99)$MBP_{83-99}$ peptide administration is statistically significant; on the other hand, when the induced EAE is not very homogenous and has high variability concerning the clinical scores (as this was the case with the chronic experimental set), then the mild benefit from the cyclo(83-99)$MBP_{83-99}$ peptide administration does reach statistical significance.

As far as histopathology is concerned, the results indicate that there is a quantitative similar inflammatory burden within the spinal cords of the 2 groups (control and cyclo(83-99)$MBP_{83-99}$ or group B) during the acute phase; this inflammatory load is cleared in both groups, as they progress to chronic phase, but the clearance seems to be more efficiently in group B and leads to reduced inflammatory cells within their spinal cords. When demyelination was studied, the animals in group B were found to have significant less demyelinated axons compared to controls, both in acute and chronic phases of the disease. When axonopathic processes were studied, axonal injury and axonal loss were found to be significantly milder in group B during acute phase, a fact that seems to lead to an increased preservation of axons in group B, through progression of EAE to chronic phase, as this is indicated by the reduced axonal loss in group B at chronic phase. Thus, a relatively preserved axonal density was observed in group B during chronic phase which seems to conform to the mild clinical benefit of cyclo(83-99)$MBP_{83-99}$ co-administration during chronic phase.

The aforesaid results cannot be explained simply through the inflammatory load of the spinal cords of the animals since there is no difference between the absolute numbers of infiltrating cells within the CNS parenchyma during acute phase of EAE. However, it is probable that the equal numbers of infiltrating cells during acute phase of the disease do not necessary correspond to equal numbers of "reactive" MOG-specific T-lymphocytes within the parenchyma. If this is to be true and there are less MOG-reactive T-cells and more cyclo(83-99)$MBP_{83-99}$ specific but not reactive T-cells, then it could probably result in reduced demyelination, reduced axonopathic processes and increased clearance of the inflammatory cells as the disease progressed to chronicity [Lassmann *et. al*., 2007, Abromson-Leeman, *et al*., 2005, Bauer J *et al*., 1998, Berger T *et al*., 1997, Suvannavejh GC *et al*., 2000, Pender MP and Rist MJ 2001].

Thus, as a conclusion, the cyclo(83-99)$MBP_{83-99}$ peptide does indeed produce some beneficial effect in the C57BL/6 MOG-EAE. However, this effect is not as strong as in MBP-EAE in Lewis rats.

## EXAMPLE 2

### Results for linear [$A^{91}$, $A^{96}$]$MBP_{83-99}$ and comparison with [$R^{91}$, $A^{96}$]$MBP_{83-99}$

Interactions of [$A^{91}$, $A^{96}$]$MBP_{87-99}$ and [$R^{91}$, $A^{96}$]$MBP_{87-99}$ peptides in complex with MHC class II, I-$A^s$

In the modeled H2 I-$A^s$ complex of the MBP peptide, the residues $V^{87}$, $F^{90}$, $N^{92}$, and $T^{95}$ are anchored in MHC pockets P1, P4, P6 and P9, respectively [11, 12]. The residue $K^{91}$ is pointing up from the MHC groove. The models indicate that mutations of the $K^{91}$ residue do not cause major disruptions to the structures and to the intermolecular interactions between the peptide and the MHC cleft (Fig. 7). The effect of mutation at position 91 has been studied by analyzing intermolecular interactions in the mutated complexes. These are summarized in the tables in Figures 14 and 15. The intermolecular interactions in the modeled complex with ([$R^{91}$, $A^{96}$]$MBP_{87-96}$) have been described previously [11]. The analog ([$A^{91}$, $A^{96}$]$MBP_{87-96}$) shows similar trends as described below.

With respect to hydrogen bonding interactions, the most pronounced effect includes the loss of contact made by the nitrogen atoms of $K^{91}$ and $F^{90}$ (MBP). Also, [$A^{91}$, $A^{96}$]$MBP_{87-96}$ does not display the contacts to the MHC residues $N^{82}$(B), $Y^{67}$(B), and $Y^{68}$(A), observed in the complex with the wild type peptide. However, compensatory interactions are observed to the MHC residues $T^{77}$(B) and $D^{57}$(B). It must be noted that [$A^{91}$, $A^{96}$]$MBP_{87-96}$ displays the greatest deviation from the intermolecular hydrogen bond interactions of the wild type complex, compared to all other mutants we have studied previously [11, 12]. Interestingly, the differences in the van der Waals (vdW) interactions made by the native peptide *vs.* [$A^{91}$, $A^{96}$]$MBP_{87-96}$ were less pronounced than was observed with other analogs [11, 12]. In this case, the loss of contact made by $K^{91}$ is once more observed. To balance, the analog exhibits the $A^{91}$ (MBP) interactions with $F^{11}$(B) (MHC). Other losses of contacts by [$A^{91}$, $A^{96}$]$MBP_{87-96}$ include $V^{87}$ to $Y^9$(A), $H^{81}$(B) and $N^{82}$(B), $F^{89}$ to $Y^9$(A), and $V^{94}$ to $T^{65}$(A). All these are compensated by the interactions, observed in the mutant complex, but not exhibited by the native MBP peptide itself: $V^{87}$ to $F^{54}$(A), $F^{89}$ to $V^{78}$(B), and $V^{94}$ to $Y^{61}$(B), respectively. Based on the novel interactions noted by [$A^{91}$, $A^{96}$]$MBP_{87-96}$ and [$R^{91}$, $A^{96}$]$MBP_{87-96}$ peptide with H2 I-$A^s$, we assessed their immunological profile in SJL/J ($H2^s$) mice compared to the native peptide.

[$R^{91}$, $A^{96}$]$MBP_{87-99}$ peptide conjugated to reduced mannan decreases IFN-γ production and generates high levels of IL-4

The ability of the native $MBP_{87-99}$ peptide and the double mutant analog [$R^{91}$, $A^{96}$]$MBP_{87-99}$ to induce immune responses in SJL/J mice was assessed by IFN-γ and IL-4 ELISpot assay. High levels of IFN-γ were generated to $MBP_{87-99}$ peptide which were decreased by 30 % ($p<0.01$) when mice were immunized with [$R^{91}$, $A^{96}$]$MBP_{87-99}$ peptide emulsified in CFA (Fig. 2a). However, the levels of IFN-γ were further decreased (70 %) when [$R^{91}$, $A^{96}$]$MBP_{87-99}$ peptide was conjugated to reduced mannan ($p<0.01$) (Fig. 8a). Furthermore, immunization of mice with the native $MBP_{87-99}$ peptide either emulsified in CFA or conjugated to reduced mannan did not induce IL-4 cytokine secreting T cells (Fig. 8b). It was of interest however, that immunization of mice with [$R^{91}$, $A^{96}$]$MBP_{87-99}$ peptide emulsified in CFA induced low levels of IL-4 (which was significant above background, $p<0.01$) and very high IL-4 levels when conjugated to reduced mannan ($p<0.01$) (Fig. 8b). It is clear that [$R^{91}$, $A^{96}$]$MBP_{87-99}$ significantly decreases IFN-γ levels and generates high levels of IL-4 when conjugated to reduced mannan.

[$A^{91}$, $A^{96}$]$MBP_{87-99}$ peptides conjugated to reduced mannan diverts immune responses from Th1 (IFN-γ) to Th2 (IL-4)

The ability of the linear $MBP_{87-99}$ or [$A^{91}$, $A^{96}$]$MBP_{87-99}$ peptides either emulsified in CFA or conjugated to reduced mannan, to induce T cell responses after 1 or 2 injections respectively was measured using IFN-γ and IL-4 cytokine secretion by ELISpot analysis. Mice immunized with $MBP_{87-99}$ peptide induced high levels of IFN-γ secreting T cells when immunized with either CFA or reduced mannan ($p<0.01$) (Fig. 9a-b). No detectable IL-4 cytokine was induced after immunization with $MBP_{87-99}$ peptide in either conjugate (Fig. 10a-b). Mice immunized with linear [$A^{91}$, $A^{96}$]$MBP_{87-99}$ emulsified in CFA was able to induce high levels of IL-4 ($p<0.01$) (Fig. 10a), however, strong levels of IFN-γ were also generated ($p<0.01$) (Fig. 9a). Interestingly, when [$A^{91}$, $A^{96}$]$MBP_{87-99}$ peptide was conjugated to reduced mannan high levels of IL-4 were induced (Fig. 10b) and no IFN-γ was detected ($p<0.01$) (Fig. 9b). Overall, [$A^{91}$, $A^{96}$]$MBP_{87-99}$ emulsified in CFA generated both Th1 and Th2 responses, however, the use of reduced mannan conjugated to [$A^{91}$, $A^{96}$]$MBP_{87-99}$ is able to divert immune responses from Th1 to Th2. No peptide was used as a negative control and ConA was used as an internal positive control which consistently induced >1,000 SFU/0.5 million cells for both IFN-γ and IL-4 (not shown).

T cells from SJL/J mice immunized with [$A^{91}$, $A^{96}$]$MBP_{87-99}$ emulsified in CFA or conjugated to reduced mannan do not cross-react with the native $MBP_{87-99}$ peptide

T cells after immunization with the linear mutant [$A^{91}$, $A^{96}$]$MBP_{87-99}$ peptide emulsified in CFA or conjugated to reduced mannan were examined if they cross-reacted with the native $MBP_{87-99}$ peptide using ELISpot assay. T cells from mice immunized with linear [$A^{91}$, $A^{96}$]$MBP_{87-99}$ peptide emulsified in CFA (Fig. 11a) or conjugated to reduced mannan (Fig. 11b) did not cross-react with the native $MBP_{87-99}$ peptide (Fig. 11a-b). No peptide was used as a negative control and ConA was used as an internal positive control which consistently induced >1,000 SFU/0.5 million cells for both IFN-$\gamma$ and IL-4 (not shown). In addition, T cells from [$A^{91}$, $A^{96}$]$MBP_{87-99}$ did not cross-react with [$R^{91}$, $A^{96}$]$MBP_{87-99}$ peptide, which acted as an additional negative (background) control (Fig. 11).

Antibody responses to $MBP_{87-99}$ or [$A^{91}$, $A^{96}$]$MBP_{87-99}$ peptides emulsified in CFA or conjugated to reduced mannan

The production of total IgG antibody responses in mice immunized with $MBP_{87-99}$ or [$A^{91}$, $A^{96}$]$MBP_{87-99}$ peptide analogs emulsified in CFA (Fig. 12a), or conjugated to reduced mannan (Fig. 12b), were measured using ELISA. No IgG antibody levels were generated in mice immunized with [$A^{91}$, $A^{96}$]$MBP_{87-99}$ peptide emulsified in CFA, whilst very low levels of IgG were generated to the native $MBP_{87-99}$ (Fig. 12a). However, high antibody levels were induced to both peptides $MBP_{87-99}$ and [$A^{91}$, $A^{96}$]$MBP_{87-99}$ when conjugated to reduced mannan (titer >1/1,6400) (Fig. 12b).

Since higher levels of antibodies were generated to linear [$A^{91}$, $A^{96}$]$MBP_{87-99}$ peptide when conjugated to reduced mannan, it was determined whether sera from these mice cross-reacted with the native $MBP_{87-99}$ peptide. Sera from mice immunized with $MBP_{87-99}$ reacted with $MBP_{87-99}$ peptide (positive control), while antibodies from mice immunized with linear [$A^{91}$, $A^{96}$]$MBP_{87-99}$ conjugated to reduced mannan did not cross-react with the native $MBP_{87-99}$ peptide at 1/500 and 1/1,000 sera dilutions (Fig. 12d). Moreover, mice immunized with [$A^{91}$, $A^{96}$]$MBP_{87-99}$ emulsified in CFA also did not cross-react with the native $MBP_{87-99}$ peptide (Fig. 12c). Overall, while IgG antibodies were noted for [$A^{91}$, $A^{96}$]$MBP_{87-99}$ conjugated to reduced mannan, they did not cross-react with the native $MBP_{87-99}$ peptide.

## Discussion

Activation of $CD4^+$ T cells is initiated by the interaction between the TCR and a peptide-antigen that is presented by MHC class II molecules, and, the engagement of co-stimulatory molecules of antigen presenting cells[33]. This process is followed by T cell proliferation, stimulation of reactive T cells specific to the antigen and secretion of relevant cytokines. Many studies have shown that peptides with mutations at critical TCR contact residues, results in altered T cell function [34-36]. In particular, altered peptide ligands (or mutant peptides) have been found to shift the balance of immune responses from Th1 to Th2 [34, 37, 38]. Th1 responses (IFN-$\gamma$) involve proinflammatory cytokines that mediate autoimmune diseases, and, Th2 responses (IL-4, IL-10) reduce IFN-$\gamma$ secretion and other inflammatory cytokines, preventing autoimmunity [15, 39, 40]

The development of safe and effective vaccines and immunotherapeutic approaches against MS is actively being studied. To date, a number of clinical trials have been undertaken in MS patients with altered peptide ligands [41, 42]. Even though the peptides were demonstrated to induce appropriate responses in pre-clinical studies, these clinical trials were discontinued due to adverse reactions [41, 42]. In some patients, unanticipated cross-reactions were stimulated by the peptide analogs against the native peptide/protein [41, 42]. Thus, further pre-clinical testing is required and new modified peptides need to be designed in order to develop an effective vaccine for MS. In the cancer setting, it was demonstrated that the mutation of the HLA-A2 derived peptide (from carcinoembryonic antigen) at position 6 generated a superagonist. It induced $CD8^+$ T cells that cross reacted with high concentrations of the native peptide, however, did not recognize carcinoembryonic antigen expressing cancer cells [43]. Thus, there is a need for extensive analysis of tumor cross recognition prior to any clinical usage of APL as vaccines. Therefore, we designed and synthesized two linear mutant peptides, based on the short immunodominant epitope $MBP_{87-99}$, with mutations at the positions $K^{91}$ and $P^{96}$ ($K^{91}$ was modified to $R^{91}$ or $A^{91}$ and $P^{96}$ to $A^{96}$). The mutant analogs were injected in SJL/J mice in order to examine their ability to shift immune responses from Th1 to Th2, and whether T cells and antibodies cross reacted with the native peptide. An adjuvant (CFA) or a suitable carrier (reduced mannan) were used.

Immunization of SJL/J mice with native $MBP_{87-99}$ peptide emulsified in CFA generated high levels of IFN-$\gamma$, whilst the double mutant analog [$R^{91}$, $A^{96}$]$MBP_{87-99}$ decreased IFN-$\gamma$ secretion by 30%. These findings are similar to those previously published with the longer double mutant peptide [$R^{91}$, $A^{96}$]$MBP_{83-99}$ [11]. It is of interest that the levels of IFN-$\gamma$ were further decreased (70%) when [$R^{91}$, $A^{96}$]$MBP_{87-99}$ peptide was conjugated to the carrier reduced mannan. Furthermore, immunization of mice with the native $MBP_{87-99}$ peptide either emulsified in CFA or conjugated to reduced mannan did not induce IL-4 cytokine secreting T cells. Thus, substitution of $K^{91}$ and $P^{96}$ with $R^{91}$ and $A^{96}$, respectively, could decrease IFN-$\gamma$ levels, generate high levels of IL-4 when [$R^{91}$, $A^{96}$]$MBP_{87-99}$ peptide was conjugated to reduced mannan, but there was still secretion of IFN-$\gamma$. By contrast, immunization with the double mutant analog [$A^{91}$, $A^{96}$]$MBP_{87-99}$

conjugated to reduced mannan induced high levels of IL-4 and no IFN-γ was detected. Substitution of residues 91 and 96 to Ala ([$A^{91}$, $A^{96}$]$MBP_{87-99}$) has the ability to generate IL-4 cytokine, however, with the appropriate carrier, it is able to divert immune responses from IFN-γ to IL-4. Overall, the use of adjuvant (CFA) generates both Th1 and Th2 responses and does not seem to be beneficial, however, the use of an appropriate carrier such as reduced mannan conjugated to [$A^{91}$, $A^{96}$]$MBP_{87-99}$ was able to divert immune responses from Th1 to Th2. Most importantly, T cells secreting IFN-γ and IgG antibodies generated to the double mutant [$A^{91}$, $A^{96}$]$MBP_{87-99}$ peptide did not cross-react with the native $MBP_{87-99}$ peptide. It is clear that the double mutant [$A^{91}$, $A^{96}$]$MBP_{87-99}$ peptide analog is a promising candidate for further studies for the immunotherapy of MS.

Likewise, we recently demonstrated using single amino acid mutations of the longer peptide $MBP_{83-99}$, that a single amino acid change of $K^{91}$ to $Y^{91}$ ([$Y^{91}$]$MBP_{83-99}$) when conjugated to reduced mannan also diverted immune responses from Th1 to Th2[12], similar to [$A^{91}$, $A^{96}$]$MBP_{87-99}$ peptide conjugated to reduced mannan. It is clear that the peptide sequence and length and the type of adjuvant/carrier used are important for eliciting the desired immune response to a peptide-based vaccine.

Modeling of the ([$A^{91}$, $A^{96}$]$MBP_{87-96}$) and ([$R^{91}$, $A^{96}$]$MBP_{87-96}$) analogs in complex with H2 I-$A^s$ revealed that the side-chains of the mutated residues are exposed to make contacts with TCR and the mutations do not cause major disruptions to the complex structures and to the intermolecular interactions between the peptides and the MHC cleft. It was noted that the backbone of [$R^{91}$, $A^{96}$]$MBP_{87-96}$, [$A^{91}$, $A^{96}$]$MBP_{87-96}$ and native $NBP_{87-96}$ peptides overlapped very closely and only minor conformational changes were observed in the amino acids at positions 91 and 96 (Fig. 7d). Not unexpectedly, certain specific contacts observed in the complex with the native peptide were lost in the complexes with the mutants (Figs 14 and 15). These were compensated by novel interactions with MHC residues. Most notably, the ([$A^{91}$, $A^{96}$]$MBP_{87-96}$) analog displayed the greatest reduction of the intermolecular hydrogen bond interactions of the native complex, compared to all other mutants we have studied previously [11, 12]. This is not surprising given the extent of side-chain modification when an alanine residue is used instead of arginine and proline.

Peptides causing antagonism have been shown to have fewer hydrogen bond contacts between the peptide side chains and the CDR3 loops of the TCR [44]. Loss of hydrogen bond contact can cause agonist or super-agonist (hyper-stimulatory APL) peptides to become antagonists. For example, a single amino acid mutation of vesicular stomatitis virus peptide VSV8 (RGYVYQGL to RGYVYEGL) leads to antagonism of T cell hybridomas specific to native VSV8. The crystal structure of this APL with H-$2K^b$ demonstrated that a minor peptide modification induced profound biological effect [45]. The TCR, which recognizes VSV8 (RGYVYQGL) peptide and its APL (RGYVYEGL), was mutated by a single amino acid at the CDR3β loop, and this was able to modulate the TCR-antagonistic properties of an APL [46]. These examples validate the results of our modeling studies with respect to MBP mutant peptides. Namely, that even subtle conformational changes are sufficient to have profound biological effects.

## Experimental Procedures

### (1) Molecular modeling

Molecular modeling was carried out using HyperChem modeling package (HyperCube Inc., version 7.52) as previously described [11]. OPLS force field was used for molecular mechanics geometry optimization. The MHC-peptide complexes were generated based on the crystal structure of I-$A^u$ complex with $MBP_{1-11}$ peptide (PDB code 1K2D). This template was chosen upon the consideration of nine relevant crystal structures, based on the combination of sequence identity to the target, crystal structure, resolution, and the degree of disruption to the peptide interaction residues, upon mutation [12]. Alignment of the MBP peptide within the MHC cleft was carried out based on the analysis of all possible MBP positions and the following preferred binding register was deduced [12]

| | | | | | | | | | |
|---|---|---|---|---|---|---|---|---|---|
| 1K2D | (G | G) | $A^1$ | S | Q | Y | R | P | S | $Q^8$ |
| MBP | $V^{87}$ | H | F | F | K | N | I | V | T | $P^{96}$ |

To produce mutated MBP peptides, the lysine residue at position 91 and the proline residue at position 96 were mutated to $A^{91}$ and $A^{96}$ ([$A^{91}$, $A^{96}$]$MBP_{87-96}$) and $R^{91}$ and $A^{96}$ ([$R^{91}$, $A^{96}$]$MBP_{87-96}$), respectively. The complexes were then optimized and the intermolecular interactions in the complexes were studied using the program LigPlot [47].

### (2) Solid-phase peptide synthesis of linear analogs

Peptides $MBP_{87-99}$ (VHFFKNIVTPRTP), [$R^{91}$, $A^{96}$]$MBP_{87-99}$ (VHFFRNIVTARTP) and [$A^{91}$, $A^{96}$]$MBP_{87-99}$ (VHFFANIVTARTP) were prepared on 2-chlorotrityl chloride resin (CLTR-Cl) using Fmoc/tBu methodology [22, 48-51]. Preparative HPLC for $MBP_{87-99}$, [$R^{91}$, $A^{96}$]$MBP_{87-99}$ and [$A^{91}$, $A^{96}$]$MBP_{87-99}$ peptide analogs were performed using a Lichrosorb

RP-18 reversed phase semipreparative column with 7 μm packing material.

The peptides were > 95 % pure as analyzed by analytical RP-HPLC and ESI-MS (Fig 13).

(3) Conjugation of reduced mannan to $MBP_{87-99}$ native and mutant peptides via a KLH linker

$MBP_{87-99}$, $[R^{91}, A^{96}]MBP_{87-99}$ or $[A^{91}, A^{96}]MBP_{87-99}$ peptides were conjugated to keyhole limpet hemocyanin (KLH) via glutaraldehyde which acts as a linker between mannan and peptide.[27] 14 mg mannan (from Saccharomyces cerevisiae, SIGMA, St Louis, USA) was dissolved in 1 ml sodium phosphate buffer (pH 6.0), followed by the addition of 100 μl of 0.1 M sodium periodate (dissolved in pH 6.0 phosphate buffer) and incubated at 4° C for 1 hour (h) in the dark. Ethanediol (10 μl) was added to the mixture and incubated for 30 minutes (min) at 4° C. The resultant mixture (oxidized mannan) was passed through a PD-10 column (Sephadex G-25 M column, Amersham Biosciences, Sweden) pre-equilibrated in phosphate buffer (pH 9.0) and 2 ml solution comprising oxidized mannan collected. One mg each of $MBP_{87-99}$-KLH, $[A^{91}, A^{96}]MBP_{87-99}$-KLH or $[R^{91}, A^{96}]MBP_{87-99}$-KLH peptides were added to 2 ml of an oxidized mannan solution and incubated overnight (O/N) at room temperature (RT) in the dark. Conjugation occurs via Schiff base formation between free amino groups of KLH and oxidized mannan. Reduced mannan-KLH-$MBP_{87-99}$, reduced mannan-KLH-$[R^{91}, A^{96}]MBP_{87-99}$ or reduced mannan-KLH-$[A^{91}, A^{96}]MBP_{87-99}$ complexes were prepared by adding 1.0 mg sodium borohydride for 6-8 h at RT in the dark and were used without further purification as previously described [12, 23, 30, 31]. MBP peptide analogs were previously characterized by capillary electropheresis for conjugation to mannan [52] and by SDS PAGE gel, staining with Coomassie, Silver or Schiff's reagent (data not shown). Peptides were 100 % conjugated to reduced mannan.

(4) Mice

Female 6-8 week old SJL/J mice used in this study, were purchased from Walter and Eliza Hall Institute (Victoria, Australia) and housed at the Biological Research Laboratory at Burnet Institute, Austin campus, Heidelberg, Australia.

(5) CFA-peptide immunization

$MBP_{87-99}$, $[R^{91}, A^{96}]MBP_{87-99}$ or $[A^{91}, A^{96}]MBP_{87-99}$ peptides were dissolved in PBS and emulsified in an equal volume of complete Freund's adjuvant (CFA) which contained 1.0 mg/ml of heat killed *Mycobacterium tuberculosis* H37RA (Sigma, Victoria, Australia). SJL/J mice were given one subcutaneous injection containing 50 μg peptide into the base of tail.

(6) Reduced mannan-KLH-peptide immunization

SJL/J mice were immunized twice on days 0 and 14 intradermally (base of tail) with 50 μg $MBP_{87-99}$-KLH-reduced mannan, $[R^{91}, A^{96}]MBP_{87-99}$-KLH-reduced mannan or $[A^{91}, A^{96}]MBP_{87-99}$-KLH-reduced mannan conjugates.

(7) Enzyme-linked immunospot assay (ELISpot)

ELISpot detects specific T cell responses to antigens by measuring the secretion of specific cytokines from individual cells. Spleen cells from immunized SJL/J mice were isolated 28 days after CFA immunization or 14 days after the last immunization with reduced mannan-peptides and assessed by ELISpot for IFN-γ and IL-4 secretion by T cells. IFN-γ ELISpot assay was performed on MultiScreen-IP Filter Plate (MAIP S4510) with hydrophobic PVDF filters (Millipore, UK), while IL-4 ELISpot assays were performed on MultiScreen-HA Filter Plate (MAHA S4510) with mixed cellulose esters filters (Millipore, UK). MAIP S4510 plates were pre-weted with 50 μl of 70 % ethanol, washed 5 times with 200 μl of sterile phosphate-buffered saline (PBS) and coated with 70 μl of 5 μg/ml anti-IFN-γ capture antibody, AN18 (Mabtech, Australia) in PBS and incubated O/N at 4° C. 70 μl of 5 μg/ml anti-IL-4 capture antibody (Mabtech, Australia) was added directly to MAHA S4510 plates and incubated O/N at 4° C without 70 % ethanol treatment. Following 5 washes with PBS, plates were blocked by adding 200 μl of culture media (supplemented with 2.5 % fetal calf serum) and incubated for 2 h at 37° C. The blocking media was discarded and 10 μg/ml $MBP_{87-99}$, $[R^{91}, A^{96}]MBP_{87-99}$ or $[A^{91}, A^{96}]MBP_{87-99}$ recall peptides were added into each defined well. ConA (1.0 μg/ml) was used as an internal positive control and no peptide (cells alone) as a negative control. Triplicate wells were set up for each condition. Spleen cells (0.5 million cells) in 100 μl culture media were seeded into each well and incubated at 37° C for 18 h for IFN-γ or 24 for IL-4, respectively. Plates were washed 5 times with PBS/0.05 % Tween 20 followed by 5 times with PBS and incubated for 2 h at RT with anti-mouse IFN-γ or IL-4 monoclonal antibody-biotin. Plates were washed and streptavidin-ALP was added at 1.0 μg/ml and incubated for a further 2 h at RT. Spots of activity were detected using a colorimetric AP kit (Biorad, Hercules, CA USA) and counted using an AID ELISpot plate reader (Autoimmun Diagnostika GmbH, Germany). Data are presented

as mean spot forming units (SFU) per 0.5 million cells +/- standard error of the mean (SEM).

(8) Enzyme-linked immunosorbent assay (ELISA)

Blood was collected and sera isolated from mice prior to and 4 weeks after immunization with CFA or 14 days after the last immunization with reduced mannan conjugates. $MBP_{87-99}$ or $[A^{91}, A^{96}]MBP_{87-99}$ peptides conjugated to BSA or KLH were coated onto polyvinyl chloride (PVC) microtiter plates at 10 $\mu$g/ml in 0.2 M $NaHCO_3$ buffer, pH 9.6, O/N at 4° C. Non-specific binding was blocked with 2 % BSA for 1.0 h at RT. After washing (0.05 % Tween 20/PBS), serial dilutions of sera were added and incubated for a further 2 h at RT. The plates were washed and bound antibody was detected using HRP-conjugated sheep anti-mouse antibody (1/1000 dilution in PBS) (Amersham, UK) and developed using 2,2'-azino-di(3-ethylbenzthiazoline)6-sulfonic acid (ABTS) (Sigma, UK). Absorbance at 405nm was recorded using a Fluostar Optima microplate reader (BMG labtech, Offenburg, Germany).

(9) Statistical analysis

Mean values were compared using the Student's two-tailed *t*-test. P value threshold of p<0.01 indicates a statistically significant difference.

**References for Example 2**

1. Katsara, M.; Matsoukas, J.; Deraos, G.; Apostolopoulos, V. Towards immunotherapeutic drugs and vaccines against multiple sclerosis. Acta Biochim Biophys Sin (Shanghai) 2008, 40, 636-42.
2. Martin, R.; Howell, M. D.; Jaraquemada, D.; Flerlage, M.; Richert, J.; Brostoff, S.; Long, E. O.; McFarlin, D. E.; McFarland, H. F. A myelin basic protein peptide is recognized by cytotoxic T cells in the context of four HLA-DR types associated with multiple sclerosis. J Exp Med 1991, 173, 19-24.
3. Oksenberg, J. R.; Baranzini, S. E.; Barcellos, L. F.; Hauser, S. L. Multiple sclerosis: genomic rewards. J Neuroimmunol 2001, 113, 171-84.
4. Ota, K.; Matsui, M.; Milford, E. L.; Mackin, G. A.; Weiner, H. L.; Hafler, D. A. T-cell recognition of an immunodominant myelin basic protein epitope in multiple sclerosis. Nature 1990, 346, 183-7.
5. Zhang, J.; Markovic-Plese, S.; Lacet, B.; Raus, J.; Weiner, H. L.; Hafler, D. A. Increased frequency of interleukin 2-responsive T cells specific for myelin basic protein and proteolipid protein in peripheral blood and cerebrospinal fluid of patients with multiple sclerosis. J Exp Med 1994, 179, 973-84.
6. Hafler, D. A.; Slavik, J. M.; Anderson, D. E.; O'Connor, K. C.; De Jager, P.; Baecher-Allan, C. Multiple sclerosis. Immunol Rev 2005, 204, 208-31.
7. Steinman, L. Multiple sclerosis: a coordinated immunological attack against myelin in the central nervous system. Cell 1996, 85, 299-302.
8. Sakai, K.; Sinha, A. A.; Mitchell, D. J.; Zamvil, S. S.; Rothbard, J. B.; McDevitt, H. O.; Steinman, L. Involvement of distinct murine T-cell receptors in the autoimmune encephalitogenic response to nested epitopes of myelin basic protein. Proc Natl Acad Sci U S A 1988, 85, 8608-12.
9. Sakai, K.; Zamvil, S. S.; Mitchell, D. J.; Lim, M.; Rothbard, J. B.; Steinman, L. Characterization of a major encephalitogenic T cell epitope in SJL/J mice with synthetic oligopeptides of myelin basic protein. J Neuroimmunol 1988, 19, 21-32.
10. Karin, N.; Mitchell, D. J.; Brocke, S.; Ling, N.; Steinman, L. Reversal of experimental autoimmune encephalomyelitis by a soluble peptide variant of a myelin basic protein epitope: T cell receptor antagonism and reduction of interferon gamma and tumor necrosis factor alpha production. J Exp Med 1994, 180, 2227-37.
11. Katsara, M.; Yuriev, E.; Ramsland, P. A.; Deraos, G.; Tselios, T.; Matsoukas, J.; Apostolopoulos, V. A double mutation of MBP(83-99) peptide induces IL-4 responses and antagonizes IFN-gamma responses. J Neuroimmunol 2008.
12. Katsara, M.; Yuriev, E.; Ramsland, P. A.; Deraos, G.; Tselios, T.; Matsoukas, J.; Apostolopoulos, V. Mannosylation of mutated MBP(83-99) peptides diverts immune responses from Th1 to Th2. Mol Immunol 2008, 45, 3661-70.
13. Brocke, S.; Gijbels, K.; Allegretta, M.; Ferber, I.; Piercy, C.; Blankenstein, T.; Martin, R.; Utz, U.; Karin, N.; Mitchell, D.; Veromaa, T.; Waisman, A.; Gaur, A.; Conlon, P.; Ling, N.; Fairchild, P. J.; Wraith, D. C.; O'Garra, A.; Fathman, C. G.; Steinman, L. Treatment of experimental encephalomyelitis with a peptide analogue of myelin basic protein. Nature 1996, 379, 343-6.
14. Cannella, B.; Raine, C. S. The adhesion molecule and cytokine profile of multiple sclerosis lesions. Ann Neurol 1995, 37, 424-35.

15. Racke, M. K.; Bonomo, A.; Scott, D. E.; Cannella, B.; Levine, A.; Raine, C. S.; Shevach, E. M.; Rocken, M. Cytokine-induced immune deviation as a therapy for inflammatory autoimmune disease. J Exp Med 1994, 180, 1961-6.

16. Gaur, A.; Boehme, S. A.; Chalmers, D.; Crowe, P. D.; Pahuja, A.; Ling, N.; Brocke, S.; Steinman, L.; Conlon, P. J. Amelioration of relapsing experimental autoimmune encephalomyelitis with altered myelin basic protein peptides involves different cellular mechanisms. J Neuroimmunol 1997, 74, 149-58.

17. Gauthier, L.; Smith, K. J.; Pyrdol, J.; Kalandadze, A.; Strominger, J. L.; Wiley, D. C.; Wucherpfennig, K. W. Expression and crystallization of the complex of HLA-DR2 (DRA, DRB1*1501) and an immunodominant peptide of human myelin basic protein. Proc Natl Acad Sci U S A 1998, 95, 11828-33.

18. Li, Y.; Huang, Y.; Lue, J.; Quandt, J. A.; Martin, R.; Mariuzza, R. A. Structure of a human autoimmune TCR bound to a myelin basic protein self-peptide and a multiple sclerosis-associated MHC class II molecule. Embo J 2005, 24, 2968-79.

19. Smith, K. J.; Pyrdol, J.; Gauthier, L.; Wiley, D. C.; Wucherpfennig, K. W. Crystal structure of HLA-DR2 (DRA*0101, DRB1*1501) complexed with a peptide from human myelin basic protein. J Exp Med 1998, 188, 1511-20.

20. Wucherpfennig, K. W.; Hafler, D. A.; Strominger, J. L. Structure of human T-cell receptors specific for an immunodominant myelin basic protein peptide: positioning of T-cell receptors on HLA-DR2/peptide complexes. Proc Natl Acad Sci U S A 1995, 92, 8896-900.

21. Kalbus, M.; Fleckenstein, B. T.; Offenhausser, M.; Bluggel, M.; Melms, A.; Meyer, H. E.; Rammensee, H. G.; Martin, R.; Jung, G.; Sommer, N. Ligand motif of the autoimmune disease-associated mouse MHC class II molecule H2-A(s). Eur J Immunol 2001, 31, 551-62.

22. Matsoukas, J.; Apostolopoulos, V.; Kalbacher, H.; Papini, A. M.; Tselios, T.; Chatzantoni, K.; Biagioli, T.; Lolli, F.; Deraos, S.; Papathanassopoulos, P.; Troganis, A.; Mantzourani, E.; Mavromoustakos, T.; Mouzaki, A. Design and synthesis of a novel potent myelin basic protein epitope 87-99 cyclic analogue: enhanced stability and biological properties of mimics render them a potentially new class of immunomodulators. J Med Chem 2005, 48, 1470-80.

23. Katsara, M.; Deraos, G.; Tselios, T.; Matsoukas, J.; Apostolopoulos, V. Design of novel cyclic altered peptide ligands of myelin basic protein MBP83-99 that modulate immune responses in SJL/J mice. J Med Chem 2008, 51, 3971-8.

24. Katsara, M.; Tselios, T.; Deraos, S.; Deraos, G.; Matsoukas, M. T.; Lazoura, E.; Matsoukas, J.; Apostolopoulos, V. Round and round we go: cyclic peptides in disease. Curr Med Chem 2006, 13, 2221-32.

25. Acres, B.; Apostolopoulos, V.; Balloul, J. M.; Wreschner, D.; Xing, P. X.; Ali-Hadji, D.; Bizouarne, N.; Kieny, M. P.; McKenzie, I. F. MUC1-specific immune responses in human MUC1 transgenic mice immunized with various human MUC1 vaccines. Cancer Immunol Immunother 2000, 48, 588-94.

26. Apostolopoulos, V.; Barnes, N.; Pietersz, G. A.; McKenzie, I. F. Ex vivo targeting of the macrophage mannose receptor generates anti-tumor CTL responses. Vaccine 2000, 18, 3174-84.

27. Apostolopoulos, V.; Karanikas, V.; Haurum, J. S.; McKenzie, I. F. Induction of HLA-A2-restricted CTLs to the mucin 1 human breast cancer antigen. J Immunol 1997, 159, 5211-8.

28. Apostolopoulos, V.; McKenzie, I. F. Role of the mannose receptor in the immune response. Curr Mol Med 2001, 1, 469-74.

29. Apostolopoulos, V.; Pietersz, G. A.; Gordon, S.; Martinez-Pomares, L.; McKenzie, I. F. Aldehyde-mannan antigen complexes target the MHC class I antigen-presentation pathway. Eur J Immunol 2000, 30, 1714-23.

30. Apostolopoulos, V.; Pietersz, G. A.; Loveland, B. E.; Sandrin, M. S.; McKenzie, I. F. Oxidative/reductive conjugation of mannan to antigen selects for T1 or T2 immune responses. Proc Natl Acad Sci USA 1995, 92, 10128-32.

31. Apostolopoulos, V.; Pietersz, G. A.; McKenzie, I. F. Cell-mediated immune responses to MUC1 fusion protein coupled to mannan. Vaccine 1996, 14, 930-8.

32. Sheng, K. C.; Pouniotis, D. S.; Wright, M. D.; Tang, C. K.; Lazoura, E.; Pietersz, G. A.; Apostolopoulos, V. Mannan derivatives induce phenotypic and functional maturation of mouse dendritic cells. Immunology 2006, 118, 372-83.

33. Harding, C. V.; Leyva-Cobian, F.; Unanue, E. R. Mechanisms of antigen processing. Immunol Rev 1988, 106, 77-92.

34. Evavold, B. D.; Allen, P. M. Separation of IL-4 production from Th cell proliferation by an altered T cell receptor ligand. Science 1991, 252, 1308-10.

35. Evavold, B. D.; Sloan-Lancaster, J.; Hsu, B. L.; Allen, P. M. Separation of T helper 1 clone cytolysis from proliferation and lymphokine production using analog peptides. J Immunol 1993, 150, 3131-40.

36. Sloan-Lancaster, J.; Evavold, B. D.; Allen, P. M. Induction of T-cell anergy by altered T-cell-receptor ligand on live antigen-presenting cells. Nature 1993, 363, 156-9.

37. Nicholson, L. B.; Greer, J. M.; Sobel, R. A.; Lees, M. B.; Kuchroo, V. K. An altered peptide ligand mediates immune deviation and prevents autoimmune encephalomyelitis. Immunity 1995, 3, 397-405.

38. Windhagen, A.; Scholz, C.; Hollsberg, P.; Fukaura, H.; Sette, A.; Hafler, D. A. Modulation of cytokine patterns

of human autoreactive T cell clones by a single amino acid substitution of their peptide ligand. Immunity 1995, 2, 373-80.

39. Street, N. E.; Mosmann, T. R. Functional diversity of T lymphocytes due to secretion of different cytokine patterns. Faseb J 1991, 5, 171-7.

40. Swain, S. L. IL4 dictates T-cell differentiation. Res Immunol 1993, 144, 616-20.

41. Bielekova, B.; Goodwin, B.; Richert, N.; Cortese, L; Kondo, T.; Afshar, G.; Gran, B.; Eaton, J.; Antel, J.; Frank, J. A.; McFarland, H. F.; Martin, R. Encephalitogenic potential of the myelin basic protein peptide (amino acids 83-99) in multiple sclerosis: results of a phase II clinical trial with an altered peptide ligand. Nat Med 2000, 6, 1167-75.

42. Kappos, L.; Comi, G.; Panitch, H.; Oger, J.; Antel, J.; Conlon, P.; Steinman, L. Induction of a non-encephalitogenic type 2 T helper-cell autoimmune response in multiple sclerosis after administration of an altered peptide ligand in a placebo-controlled, randomized phase II trial. The Altered Peptide Ligand in Relapsing MS Study Group. Nat Med 2000, 6, 1176-82.

43. Iero, M.; Squarcina, P.; Romero, P.; Guillaume, P.; Scarselli, E.; Cerino, R.; Carrabba, M.; Toutirais, O.; Parmiani, G.; Rivoltini, L. Low TCR avidity and lack of tumor cell recognition in CD8(+) T cells primed with the CEA-analogue CAP1-6D peptide. Cancer Immunol Immunother 2007, 56, 1979-91.

44. Degano, M.; Garcia, K. C.; Apostolopoulos, V.; Rudolph, M. G.; Teyton, L.; Wilson, I. A. A functional hot spot for antigen recognition in a superagonist TCR/MHC complex. Immunity 2000, 12, 251-61.

45. Thomson, C. T.; Kalergis, A. M.; Sacchettini, J. C.; Nathenson, S. G. A structural difference limited to one residue of the antigenic peptide can profoundly alter the biological outcome of the TCR-peptide/MHC class I interaction. J Immunol 2001, 166, 3994-7.

46. Kalergis, A. M.; Nathenson, S. G. Altered peptide ligand-mediated TCR antagonism can be modulated by a change in a single amino acid residue within the CDR3 beta of an MHC class I-restricted TCR. J Immunol 2000, 165, 280-5.

47. Wallace, A. C.; Laskowski, R. A.; Thornton, J. M. LIGPLOT: a program to generate schematic diagrams of protein-ligand interactions. Protein Eng 1995, 8, 127-34.

48. Tselios, T.; Apostolopoulos, V.; Daliani, I.; Deraos, S.; Grdadolnik, S.; Mavromoustakos, T.; Melachrinou, M.; Thymianou, S.; Probert, L.; Mouzaki, A.; Matsoukas, J. Antagonistic effects of human cyclic MBP(87-99) altered peptide ligands in experimental allergic encephalomyelitis and human T-cell proliferation. J Med Chem 2002, 45, 275-83.

49. Tselios, T.; Daliani, I.; Deraos, S.; Thymianou, S.; Matsoukas, E.; Troganis, A.; Gerothanassis, L.; Mouzaki, A.; Mavromoustakos, T.; Probert, L.; Matsoukas, J. Treatment of experimental allergic encephalomyelitis (EAE) by a rationally designed cyclic analogue of myelin basic protein (MBP) epitope 72-85. Bioorg Med Chem Lett 2000, 10, 2713-7.

50. Tselios, T.; Daliani, I.; Probert, L.; Deraos, S.; Matsoukas, E.; Roy, S.; Pires, J.; Moore, G.; Matsoukas, J. Treatment of experimental allergic encephalomyelitis (EAE) induced by guinea pig myelin basic protein epitope 72-85 with a human MBP(87-99) analogue and effects of cyclic peptides. Bioorg Med Chem 2000, 8, 1903-9.

51. Tselios, T.; Probert, L.; Daliani, I.; Matsoukas, E.; Troganis, A.; Gerothanassis, I. P.; Mavromoustakos, T.; Moore, G. J.; Matsoukas, J. M. Design and synthesis of a potent cyclic analogue of the myelin basic protein epitope MBP72-85: importance of the Ala81 carboxyl group and of a cyclic conformation for induction of experimental allergic encephalomyelitis. J Med Chem 1999, 42, 1170-7.

52. Tselios, T. V.; Lamari, F. N.; Karathanasopoulou, I.; Katsara, M.; Apostolopoulos, V.; Pietersz, G. A.; Matsoukas, J. M.; Karamanos, N. K. Synthesis and study of the electrophoretic behavior of mannan conjugates with cyclic peptide analogue of myelin basic protein using lysine-glycine linker. Anal Biochem 2005, 347, 121-8.

## EXAMPLE 3

### Results for cyclic [$A^{91}$, $A^{96}$]$MBP_{87-99}$

Cyclo(87-99)[$A^{91}$, $A^{96}$]$MBP_{87-99}$ emulsified in complete Freund's adjuvant or conjugated to reduced mannan reduces IFN-γ production and induces moderate levels of IL-4

The ability of the linear $MBP_{87-99}$ or cyclo(87-99)[$A^{91}$, $A^{96}$]$MBP_{87-99}$ peptides either emulsified in complete Freund's adjuvant (CFA) or conjugated to reduced mannan, to induce T cell responses after 1 or 2 injections respectively was measured using IFN-γ and IL-4 cytokine secretion by ELISpot analysis. In general, mice immunized with $MBP_{87-99}$ peptide induced high levels of IFN-γ secreting T cells when immunized with either CFA or reduced mannan (Figure 20A, 20B). No detectable IL-4 cytokine was induced after immunization with $MBP_{87-99}$ peptide in either conjugate (Figure 20C, 20D). However, mice immunized with cyclo(87-99)[$A^{91}$, $A^{96}$]$MBP_{87-99}$ peptide analog emulsified in complete Freund's adjuvant or conjugated to reduced mannan was able to reduce IFN-γ production ($p<0.01$) (Figure 20A, 20B), and

induce moderate levels of IL-4 ($p<0.01$) (Figure 20C, 20D). In particular, when cyclo(87-99)[$A^{91}$, $A^{96}$]$MBP_{87-99}$ peptide was conjugated to reduced mannan significant decreased levels of IFN-γ of ~50 % were noted compared to the linear $MBP_{87-99}$ peptide ($p<0.01$) (Figure 20B). Futhermore, ~50 % decreased IFN-γ production was noted when cyclo(87-99)[$A^{91}$, $A^{96}$]$MBP_{87-99}$ peptide conjugated to reduced mannan was compared to cyclo(87-99)[$A^{91}$, $A^{96}$]$MBP_{87-99}$ peptide emulsified in CFA and ~70 % decreased when compared to linear $MBP_{87-99}$ peptide emulsified in CFA (Figure 20A, 16B) ($p<0.01$). Moreover, moderate levels of IL-4 secreting T cells were induced to cyclo(87-99)[$A^{91}$, $A^{96}$]$MBP_{87-99}$ peptide after immunization with both conjugates ($p<0.01$) (Figure 20D). No peptide was used as a negative control and ConA was used as an internal positive control which consistently induced >1,000 SFU/0.5 million cells for both IFN-γ and IL-4 (not shown). Overall, the use of reduced mannan conjugated to cyclo(87-99)[$A^{91}$, $A^{96}$]$MBP_{87-99}$ was able to significantly reduce IFN-γ production and induce significant levels of IL-4 in SJL/J mice. Recently, the linear counterpart of this double mutant peptide, linear [$A^{91}$, $A^{96}$]$MBP_{87-99}$ was shown to inhibit IFN-γ production when conjugated to reduced mannan (Katsara et al, submitted). The cyclic analog is not as efficient in reducing IFN-γ production as compared to its linear counterpart in SJL/J mcie, however, significant levels of IFN-γ reduction is observed with the cyclic analog. Moreover, cyclic peptides being more stable than their linear counterparts are of considerable interest in the design of novel peptide based vaccines [1, 2]. Previously, we had demonstrated that a cyclic peptide from $MBP_{83-99}$ with a single Ala mutation at position 91, (cyclo(83-99)[$A^{91}$]$MBP_{83-99}$) conjugated to reduced mannan was able to antagonize IFN-γ responses *in vitro* and enhanced IL-4 responses in SJL/J mice [3]. In addition, the single Tyr mutation at position 91, ([$Y^{91}$]$MBP_{83-99}$) conjugated to reduced mannan led to the diversion of IFN-γ responses to IL-4 responses [4]. When the mutant peptides in all studies were emulsified in CFA, the antagonistic effects were not as pronounced. Thus, demonstrating the importance of reduced mannan in regulating the appropriate immune response. Reduced mannan has been shown to induce Th2 responses and induce Th2 cytokine secretion by murine dendritic cells and macrophages [3-10].

T cells and antibodies from SJL/J mice immunized with cyclo(87-99)[$A^{91}$, $A^{96}$]$MBP_{87-99}$ emulsified in CFA or conjugated to reduced mannan do not cross-react with the native $MBP_{87-99}$ peptide

While cyclo(87-99)[$A^{91}$, $A^{96}$]$MBP_{87-99}$ peptide emulsified in CFA or conjugated to reduced mannan generated IFN-γ responses, we examined whether the IFN-γ producing T cells cross-reacted with the native $MBP_{87-99}$ peptide using ELISpot assay. SJL/J mice were immunized with cyclo(87-99)[$A^{91}$, $A^{96}$]$MBP_{87-99}$ peptide and assessed IFN-γ production against $MBP_{87-99}$ peptide. T cells from mice immunized with cyclo(87-99)[$A^{91}$, $A^{96}$]$MBP_{87-99}$ emulsified CFA (Figure 21 A) or conjugated to reduced mannan (Figure 21 B) induced IFN-γ responses, but did not cross-react with the native $MBP_{87-99}$ peptide (Figure 21A, 21B).

The production of total IgG antibody responses in mice immunized with $MBP_{87-99}$ or cyclo(87-99)[$A^{91}$, $A^{96}$]$MBP_{87-99}$ peptide analogs emulsified in CFA (Figure 22A), or conjugated to reduced mannan (Figure 22B), were measured using ELISA. Low IgG antibody levels were generated in mice immunized with $MBP_{87-99}$ peptide emulsified in CFA (Figure 22A), whilst no IgG antibodies were generated to cyclo(87-99)[$A^{91}$, $A^{96}$]$MBP_{87-99}$ peptide analog (Figure 22A). However, higher levels were induced to both $MBP_{87-99}$ and cyclo(87-99)[$A^{91}$, $A^{96}$]$MBP_{87-99}$ peptides when conjugated to reduced mannan (Figure 22B). Since antibodies were generated to cyclo(87-99)[$A^{91}$, $A^{96}$]$MBP_{87-99}$ peptide, we determined whether sera from these mice cross reacted with the native $MBP_{87-99}$ peptide. Mice immunized with $MBP_{87-99}$ emulsified in CFA or conjugated to reduced mannan reacted with $MBP_{87-99}$ peptide (Figure 22C, 22D) (positive control), while antibodies from mice immunized with cyclo(87-99)[$A^{91}$, $A^{96}$]$MBP_{87-99}$ conjugated to reduced mannan or emulsified in CFA did not react with the native $MBP_{87-99}$ peptide at 1/500 and 1/1,000 sera dilution (Figure 22C, 22D). Overall, while IgG total production was noted for analogs conjugated to reduced mannan, these antibodies did not cross react to the native $MBP_{87-99}$ peptide.

These findings are of importance in the clinical setting, as cross reactive T cells and antibodies to the native peptide were induced in clinical trials with altered peptide ligands of MBP peptide which were the reason for premature termination of the trials [11, 12]. It is clear that cyclization alters the conformation of the peptide, thus, being presented in an altered form by the MHC, and hence, the T cells that are induced recognize a different conformation compared to the native form. We had previously demonstrated that cyclic peptides bind to human MHC class II, HLA-DR4 [13], and, a cartoon model of how cyclic peptides may be presented by murine MHC was shown [3].

**Experimental Procedures**

(1) Solid-phase peptide synthesis of cyclic analogs

Peptides $MBP_{87-99}$ (VHFFKNIVTPRTP) and cyclic double mutant peptide with Ala mutations at positions 91 and 96, cyclo(87-99)[$A^{91}$, $A^{96}$]$MBP_{87-99}$ (cyclo, head-to-tail, VHFFANIVTARTP) were prepared on 2-chlorotrityl chloride resin (CLTR-Cl) using Fmoc/tBu methodology. Head-to-tail cyclization of $MBP_{87-99}$[$A^{91}$, $A^{96}$] peptide was achieved using O-benzotriazol-1-yl-N,N,N',N'-tetramethyluronium tetrafluoroborate (TBTU) and 1-hydroxy-7-azabenzotriazole, 2,4,6

collidine in DMF solution, allowing fast reactions and high yield cyclization products. Results indicate that HOAt, a 4-nitrogen containing variant, is a very effective coupling additive, more efficient than HOBt for solution or solid phase synthesis [14-17]. Preparative HPLC for peptide analogs were performed using a Lichrosorb RP-18 reversed phase semi-preparative column with 7 μm packing material. The peptides were >95 % pure as analyzed by mass spectrometry (ESI-MS).

(2) Reduced mannan-KLH-peptide conjugation and immunization

$MBP_{87-99}$ or cyclo(83-99)[$A^{91}$,$A^{96}$]$MBP_{87-99}$ peptides were conjugated to keyhole limpet hemocyanin (KLH) via glutaraldehyde; KLH acts as a linker between mannan and peptide [18]. Conjugation procedures were previously published [3-9, 18]. Briefly, mannan (from *Saccharomyces cerevisiae*, SIGMA, St Louis, USA) was dissolved pH 6.0 sodium phosphate buffer, followed by the addition of sodium periodate and incubated for 1 hour at 4°C. Ethanediol was added to the mixture and incubated for 30 mins at 4°C. The mixture (oxidized mannan) was passed through a PD-10 column (Sephadex G-25 M column, Amersham Biosciences, Sweden) pre-equilibrated in pH 9.0 phosphate buffer. Oxidized mannan was eluted, to which peptides were added and allowed to react overnight at room temperature in the dark. Conjugation occurs via Schiff base formation between free amino groups of KLH and oxidized mannan. Reduced mannan-KLH-$MBP_{87-99}$ or reduced mannan-KLH-cyclo(83-99)[$A^{91}$, $A^{96}$]$MBP_{87-99}$ complexes were prepared by adding sodium borohydride. MBP peptide analogs were previously characterized by capillary electrophoresis for conjugation to mannan [19] and by SDS PAGE gel, staining with Coomassie, Silver or Schiff's reagent (data not shown). Female 6-8 week old SJL/J mice were immunized twice with 50 μg $MBP_{87-99}$ or cyclo(83-99)[$A^{91}$, $A^{96}$]$MBP_{87-99}$-KLH-reduced mannan conjugates on days 0, 14.

(3) CFA-peptide immunization

$MBP_{87-99}$ or cyclo(87-99)[$A^{91}$, $A^{96}$]$MBP_{87-99}$ peptides were dissolved in PBS and emulsified in an equal volume of CFA (Sigma, VIC Australia). SJL/J mice were given one subcutaneous injection containing 50 μg of $MBP_{87-99}$ or cyclo(87-99)[$A^{91}$, $A^{96}$]$MBP_{87-99}$ peptide.

(4) Immunological assays - ELISpot and ELISA and Statistical analysis

ELISpot assays for IFN-γ and IL-4 secretion by T cells and ELISA assays for binding of sera to native MBP protein or peptide were carried out as previously described [3, 4, 20, 21].

Mean values were compared using the Student's two-tailed *t*-test. P value threshold of $p<0.01$ indicates a statistically significant difference.

**References for Example 3**

1. Katsara, M.; Matsoukas, J.; Deraos, G.; Apostolopoulos, V. Towards immunotherapeutic drugs and vaccines against multiple sclerosis. Acta Biochim Biophys Sin (Shanghai) 2008, 40, 636-42.
2. Katsara, M.; Tselios, T.; Deraos, S.; Deraos, G.; Matsoukas, M. T.; Lazoura, E.; Matsoukas, J.; Apostolopoulos, V. Round and round we go: cyclic peptides in disease. Curr Med Chem 2006, 13, 2221-32.
3. Katsara, M.; Deraos, G.; Tselios, T.; Matsoukas, J.; Apostolopoulos, V. Design of novel cyclic altered peptide ligands of myelin basic protein MBP83-99 that modulate immune responses in SJL/J mice. J Med Chem 2008, 51, 3971-8.
4. Katsara, M.; Yuriev, E.; Ramsland, P. A.; Deraos, G.; Tselios, T.; Matsoukas, J.; Apostolopoulos, V. Mannosylation of mutated MBP(83-99) peptides diverts immune responses from Th1 to Th2. Mol Immunol 2008, 45, 3661-70.
5. Apostolopoulos, V.; Barnes, N.; Pietersz, G. A.; McKenzie, I. F. Ex vivo targeting of the macrophage mannose receptor generates anti-tumor CTL responses. Vaccine 2000, 18,3174-84.
6. Apostolopoulos, V.; McKenzie, I. F. Role of the mannose receptor in the immune response. Curr Mol Med 2001, 1, 469-74.
7. Apostolopoulos, V.; Pietersz, G. A.; Gordon, S.; Martinez-Pomares, L.; McKenzie, I. F. Aldehyde-mannan antigen complexes target the MHC class I antigen-presentation pathway. Eur J Immunol 2000, 30, 1714-23.
8. Apostolopoulos, V.; Pietersz, G. A.; Loveland, B. E.; Sandrin, M. S.; McKenzie, I. F. Oxidative/reductive conjugation of mannan to antigen selects for T1 or T2 immune responses. Proc Natl Acad Sci USA 1995, 92, 10128-32.
9. Apostolopoulos, V.; Pietersz, G. A.; McKenzie, I. F. Cell-mediated immune responses to MUCI fusion protein coupled to mannan. Vaccine 1996, 14, 930-8.

10. Sheng, K. C.; Pouniotis, D. S.; Wright, M. D.; Tang, C. K.; Lazoura, E.; Pietersz, G. A.; Apostolopoulos, V. Mannan derivatives induce phenotypic and functional maturation of mouse dendritic cells. Immunology 2006, 118, 372-83.
11. Bielekova, B.; Goodwin, B.; Richert, N.; Cortese, I.; Kondo, T.; Afshar, G.; Gran, B.; Eaton, J.; Antel, J.; Frank, J. A.; McFarland, H. F.; Martin, R. Encephalitogenic potential of the myelin basic protein peptide (amino acids 83-99) in multiple sclerosis: results of a phase II clinical trial with an altered peptide ligand. Nat Med 2000, 6, 1167-75.
12. Kappos, L.; Comi, G.; Panitch, H.; Oger, J.; Antel, J.; Conlon, P.; Steinman, L. Induction of a non-encephalitogenic type 2 T helper-cell autoimmune response in multiple sclerosis after administration of an altered peptide ligand in a placebo-controlled, randomized phase II trial. The Altered Peptide Ligand in Relapsing MS Study Group. Nat Med 2000, 6, 1176-82.
13. Matsoukas, J.; Apostolopoulos, V.; Kalbacher, H.; Papini, A. M.; Tselios, T.; Chatzantoni, K.; Biagioli, T.; Lolli, F.; Deraos, S.; Papathanassopoulos, P.; Troganis, A.; Mantzourani, E.; Mavromoustakos, T.; Mouzaki, A. Design and synthesis of a novel potent myelin basic protein epitope 87-99 cyclic analogue: enhanced stability and biological properties of mimics render them a potentially new class of immunomodulators. J Med Chem 2005, 48, 1470-80.
14. Tselios, T.; Apostolopoulos, V.; Daliani, I.; Deraos, S.; Grdadolnik, S.; Mavromoustakos, T.; Melachrinou, M.; Thymianou, S.; Probert, L.; Mouzaki, A.; Matsoukas, J. Antagonistic effects of human cyclic MBP(87-99) altered peptide ligands in experimental allergic encephalomyelitis and human T-cell proliferation. J Med Chem 2002, 45, 275-83.
15. Tselios, T.; Daliani, I.; Deraos, S.; Thymianou, S.; Matsoukas, E.; Troganis, A.; Gerothanassis, I.; Mouzaki, A.; Mavromoustakos, T.; Probert, L.; Matsoukas, J. Treatment of experimental allergic encephalomyelitis (EAE) by a rationally designed cyclic analogue of myelin basic protein (MBP) epitope 72-85. Bioorg Med Chem Lett 2000, 10, 2713-7.
16. Tselios, T.; Daliani, I.; Probert, L.; Deraos, S.; Matsoukas, E.; Roy, S.; Pires, J.; Moore, G.; Matsoukas, J. Treatment of experimental allergic encephalomyelitis (EAE) induced by guinea pig myelin basic protein epitope 72-85 with a human MBP(87-99) analogue and effects of cyclic peptides. Bioorg Med Chem 2000, 8, 1903-9.
17. Tselios, T.; Probert, L.; Daliani, I.; Matsoukas, E.; Troganis, A.; Gerothanassis, I. P.; Mavromoustakos, T.; Moore, G. J.; Matsoukas, J. M. Design and synthesis of a potent cyclic analogue of the myelin basic protein epitope MBP72-85: importance of the Ala81 carboxyl group and of a cyclic conformation for induction of experimental allergic encephalomyelitis. J Med Chem 1999, 42, 1170-7.
18. Apostolopoulos, V.; Karanikas, V.; Haurum, J. S.; McKenzie, I. F. Induction of HLA-A2-restricted CTLs to the mucin 1 human breast cancer antigen. J Immunol 1997, 159, 5211-8.
19. Tselios, T. V.; Lamari, F. N.; Karathanasopoulou, I.; Katsara, M.; Apostolopoulos, V.; Pietersz, G. A.; Matsoukas, J. M.; Karamanos, N. K. Synthesis and study of the electrophoretic behavior of mannan conjugates with cyclic peptide analogue of myelin basic protein using lysine-glycine linker. Anal Biochem 2005, 347, 121-8.
20. Apostolopoulos, V.; Yuriev, E.; Ramsland, P. A.; Halton, J.; Osinski, C.; Li, W.; Plebanski, M.; Paulsen, H.; McKenzie, I. F. A glycopeptide in complex with MHC class I uses the GalNAc residue as an anchor. Proc Natl Acad Sci U S A 2003, 100, 15029-34.
21. Katsara, M.; Yuriev, E.; Ramsland, P. A.; Deraos, G.; Tselios, T.; Matsoukas, J.; Apostolopoulos, V. A double mutation of MBP(83-99) peptide induces IL-4 responses and antagonizes IFN-gamma responses. J Neuroimmunol 2008.

**References for Background, Description and Example 1**

Abromson-Leeman, S. et al. T-cell properties determine disease site, clinical presentation, and cellular pathology of experimental autoimmune encephalomyelitis. Am J Pathol 165, 1519-33 (2004).

Acres, B., V. Apostolopoulos, J. M. Balloul, D. Wreschner, P. X. Xing, D. Ali-Hadji, N. Bizouarne, M. P. Kieny, and I. F. McKenzie. 2000. MUC1-specific immune responses in human MUC1 transgenic mice immunized with various human MUC1 vaccines. Cancer Immunol Immunother 48:588-594.

Apostolopoulos, V., G. A. Pietersz, and I. F. McKenzie. 1996. Cell-mediated immune responses to MUC1 fusion protein coupled to mannan. Vaccine 14:930-938.

Apostolopoulos, V., G. A. Pietersz, B. E. Loveland, M. S. Sandrin, and I. F. McKenzie. 1995. Oxidative/reductive conjugation of mannan to antigen selects for T1 or T2 immune responses. Proc Natl Acad Sci U S A 92:10128-10132.

Apostolopoulos, V., V. Karanikas, J. S. Haurum, and I. F. McKenzie. 1997. Induction of HLA-A2-restricted CTLs to the mucin 1 human breast cancer antigen. J Immunol 159:5211-5218.

Apostolopoulos, V.; Lofthouse, S.; Popovski, V.; Chelvanayagam G.; Sandrin, M.; McKenzie I. Nature Biot. 1998, 16, 278.

Bankovich, A. J., A. T. Girvin, A. K. Moesta, and K. C. Garcia. 2004. Peptide register shifting within the MHC groove: theory becomes reality. Molecular immunology 40:1033-1039.

Barlos, K., and D. Gatos. 1999. 9-Fluorenylmethyloxycarbonyl/ tbutyl-based convergent protein synthesis. Biopolymers 51:266-278.

Barlos, K., D. Gatos, and S. Koutsogianni. 1998. Fmoc/Trt-amino acids: comparison to Fmoc/tBu-amino acids in peptide synthesis. J Pept Res 51:194-200.

Bauer, J. et al. T-cell apoptosis in inflammatory brain lesions: destruction of T cells does not depend on antigen recognition. Am J Pathol 153, 715-24 (1998).

Berger, T. et al. Experimental autoimmune encephalomyelitis: the antigen specificity of T lymphocytes determines the topography of lesions in the central and peripheral nervous system. Lab Invest 76, 355-64 (1997).

Bieganowska, K. D., L. J. Ausubel, Y. Modabber, E. Slovik, W. Messersmith, and D. A. Hafler. 1997. Direct ex vivo analysis of activated, Fas-sensitive autoreactive T cells in human autoimmune disease. J Exp Med 185:1585-1594.

Brocke, S.; Gijbels, K.; Allegretta, M.; Ferber, I.; Piercy, C.; Blankenstein, T.; Martin, R.; Utz, U.; Karin, N.; Mitchell, D.; Veroma, T.; Waisman, A.; Gaur, A.; Conlon, P.; Ling, N.; Fairchild, P.; Wraith, D.; O'Carra, A.; Farhman, G.; Steinman, L. Nature 1996, 379, 343.

Bruggle, F.; Timmermans, M.; Van Unen, A.; Ten Hove, J.; Van De Werken, G.; Poolman, T.; Hoogerhout, P. J. Pept. Sci. 1999, 5,381.

Chaloin, L.; Mery, J.; Van Mau, N.; Divita, G.; Heitz, F. J. Pept. Sci. 1999, 5, 381.

Corper, A. L., T. Stratmann, V. Apostolopoulos, C. A. Scott, K. C. Garcia, A. S. Kang, I. A. Wilson, and L. Teyton. 2000. A structural framework for deciphering the link between I-Ag7 and autoimmune diabetes. Science 288:505-511.

Davis, W. C., R. L. Konzek, K. Haas, D. M. Estes, M. J. Hamilton, D. R. Call, V. Apostolopoulos, and I. F. McKenzie. 2002. Use of the mannan receptor to selectively target vaccine antigens for processing and antigen presentation through the MHC class I and class II pathways. Ann N Y Acad Sci 969:119-125.

Encinas, J. A. et al. Identification of genetic loci associated with paralysis, inflammation and weight loss in mouse experimental autoimmune encephalomyelitis. Int Immunol 13, 257-64 (2001).

Fleming, K. K. et al. Statistical analysis of data from studies on experimental autoimmune encephalomyelitis. J Neuroimmunol 170, 71-84 (2005).

Fontoura, P., Garren, H. & Steinman, L. Antigen-specific therapies in Multiple Sclerosis: Going beyond proteins and peptides. Int Rev Immunol 24, 415-446 (2005).

Fremont, D. H., D. Monnaie, C. A. Nelson, W. A. Hendrickson, and E. R. Unanue. 1998. Crystal structure of I-Ak in complex with a dominant epitope of lysozyme. Immunity 8:305-317.

Gauthier, L., K. J. Smith, J. Pyrdol, A. Kalandadze, J. L. Strominger, D. C. Wiley, and K. W. Wucherpfennig. 1998. Expression and crystallization of the complex of HLA-DR2 (DRA, DRB1*1501) and an immunodominant peptide of human myelin basic protein. Proc Natl Acad Sci USA 95:11828-11833.

Grigoriadis, N., Ben-Hur, T., Karussis, D. & Milonas, I. Axonal damage in multiple sclerosis: a complex issue in a complex disease. Clin Neurol Neurosurg 106, 211-7 (2004).

Hafler, D.; Weiner, H. Immunol. Rev. 1995, 144, 75.

He, X. L., C. Radu, J. Sidney, A. Sette, E. S. Ward, and K. C. Garcia. 2002. Structural snapshot of aberrant antigen presentation linked to autoimmunity: the immunodominant epitope of MBP complexed with I-Au. Immunity 17:83-94.

Iwai, H.; Pluckthun, A. FEBS Lett. 1999, 459, 166.

Kalbus, M., B. T. Fleckenstein, M. Offenhausser, M. Bluggel, A. Melms, H. E. Meyer, H. G. Rammensee, R. Martin, G. Jung, and N. Sommer. 2001. Ligand motif of the autoimmune disease-associated mouse MHC class II molecule H2-A(s). European journal of immunology 31:551-562.

Karanikas, V., L. A. Hwang, J. Pearson, C. S. Ong, V. Apostolopoulos, H. Vaughan, P. X. Xing, G. Jamieson, G. Pietersz, B. Tait, R. Broadbent, G. Thynne, and I. F. McKenzie. 1997. Antibody and T cell responses of patients with adenocarcinoma immunized with mannan-MUC1 fusion protein. J Clin Invest 100:2783-2792.

Krakowski, M. L. & Owens, T. Naive T lymphocytes traffic to inflamed central nervous system, but require antigen recognition for activation. Eur J Immunol 30, 1002-9 (2000).

Krissinel, E., and K. Henrick. 2007. Inference of Macromolecular Assemblies from Crystalline State. J Mol Biol.

Krogsgaard, M., K. W. Wucherpfennig, B. Cannella, B. E. Hansen, A. Svejgaard, J. Pyrdol, H. Ditzel, C. Raine, J. Engberg, and L. Fugger. 2000. Visualization of myelin basic protein (MBP) T cell epitopes in multiple sclerosis lesions using a monoclonal antibody specific for the human histocompatibility leukocyte antigen (HLA)-DR2-MBP 85-99 complex. J Exp Med 191:1395-1412.

Kuchroo, V. K., J. M. Greer, D. Kaul, G. Ishioka, A. Franco, A. Sette, R. A. Sobel, and M. B. Lees. 1994. A single TCR antagonist peptide inhibits experimental allergic encephalomyelitis mediated by a diverse T cell repertoire. J Immunol 153:3326-3336.

Lambert, J. D., R. O. Meyers, B. N. Timmermann, and R. T. Dorr. 2001. Pharmacokinetic analysis by high-performance liquid chromatography of intravenous nordihydroguaiaretic acid in the mouse. J Chromatogr B Biomed Sci Appl 754:85-90.

Lassmann H. Experimental models of multiple sclerosis.Rev Neurol, 2007;163(6-7):651-5.

Latek, R. R., A. Suri, S. J. Petzold, C. A. Nelson, O. Kanagawa, E. R. Unanue, and D. H. Fremont. 2000. Structural basis of peptide binding and presentation by the type I diabetes-associated MHC class II molecule of NOD mice. Immunity 12:699-710.

Lees, C. J., V. Apostolopoulos, B. Acres, C. S. Ong, V. Popovski, and I. F. McKenzie. 2000. The effect of T1 and T2 cytokines on the cytotoxic T cell response to mannan-MUC 1. Cancer Immunol Immunother 48:644-652.

Lees, C. J., V. Apostolopoulos, B. Acres, I. Ramshaw, A. Ramsay, C. S. Ong, and I. F. McKenzie. 2000. Immunotherapy with mannan-MUC1 and IL-12 in MUC1 transgenic mice. Vaccine 19:158-162.

Liu, X., S. Dai, F. Crawford, R. Fruge, P. Marrack, and J. Kappler. 2002. Alternate interactions define the binding of peptides to the MHC molecule IA(b). Proc Natl Acad Sci U S A 99:8820-8825.

Lofthouse, S. A., V. Apostolopoulos, G. A. Pietersz, W. Li, and I. F. McKenzie. 1997. Induction of T1 (cytotoxic lymphocyte) and/or T2 (antibody) responses to a mucin-1 tumour antigen. Vaccine 15:1586-1593.

Lourbopoulos, S. Grigoriadis, C. Symeonidou, G. Deretzi, N. Taskos, E. Shohami and N. Grigoriadis. Modified Bielschowsky silver impregnation combined with Hematoxylin or Cresyl Violet counterstaining as a potential tool for the simultaneous study of inflammation and axonal injury in the central nervous system. Aristotle University Med J, 2007;34:31-39.

Martin, R., M. D. Howell, D. Jaraquemada, M. Flerlage, J. Richert, S. Brostoff, E. O. Long, D. E. McFarlin, and H. F. McFarland. 1991. A myelin basic protein peptide is recognized by cytotoxic T cells in the context of four HLA-DR

types associated with multiple sclerosis. J Exp Med 173:19-24.

Martin, R.; McFarland, H.; McFarlin, D. Ann. Rev. Immunol. 1992, 10,153

Matsoukas, J., V. Apostolopoulos, H. Kalbacher, A. M. Papini, T. Tselios, K. Chatzantoni, T. Biagioli, F. Lolli, S. Deraos, P. Papathanassopoulos, A. Troganis, E. Mantzourani, T. Mavromoustakos, and A. Mouzaki. 2005. Design and synthesis of a novel potent myelin basic protein epitope 87-99 cyclic analogue: enhanced stability and biological properties of mimics render them a potentially new class of immunomodulators. J Med Chem 48:1470-1480.

Mezo, G.; Majer, Z.; valero, L.; Andreu, D.; Hudecz, F. J. Pept. Sci. 1999, 5, 272.

Moreau, T., A. Coles, M. Wing, J. Isaacs, G. Hale, H. Waldmann, and A. Compston. 1996. Transient increase in symptoms associated with cytokine release in patients with multiple sclerosis. Brain 119 (Pt 1):225-237.

Oligino, L.; Lung, T.; Sastry, L.; Bigelow, J.; Cao, T.; Curran, M.; Burke, R.; Wang, S.; Krag, D.; Roller, P.; King, R. J. Biol. Chem. 1997, 272, 29046.

Ota, K., M. Matsui, E. L. Milford, G. A. Mackin, H. L. Weiner, and D. A. Hafler. 1990. T-cell recognition of an immunodominant myelin basic protein epitope in multiple sclerosis. Nature 346:183-187.

Papadopoulos, D., Pham-Dinh, D. & Reynolds, R. Axon loss is responsible for chronic neurological deficit following inflammatory demyelination in the rat. Exp Neurol 197, 373-85 (2006).

Pender MP, Rist MJ Apoptosis of inflammatory cells in immune control of the nervous system: role of glia. Glia, 2001;36(2):137-44.

Reizis, B., M. Eisenstein, F. Mor, and I. R. Cohen. 1998. The peptide-binding strategy of the MHC class II I-A molecules. Immunology today 19:212-216.

Rudensky, A., P. Preston-Hurlburt, B. K. al-Ramadi, J. Rothbard, and C. A. Janeway, Jr. 1992. Truncation variants of peptides isolated from MHC class II molecules suggest sequence motifs. Nature 359:429-431.

Scott, C. A., P. A. Peterson, L. Teyton, and I. A. Wilson. 1998. Crystal structures of two I-Ad-peptide complexes reveal that high affinity can be achieved without large anchor residues. Immunity 8:319-329.

Scott, P.; Abel-Santos, E.; Wall, M.; Wahnon, C.; Benkovic, J. Proc. Natl. Acad. Sci. USA 1999, 16,16.

Smith, K. J., J. Pyrdol, L. Gauthier, D. C. Wiley, and K. W. Wucherpfennig. 1998. Crystal structure of HLA-DR2 (DRA*0101, DRB1*1501) complexed with a peptide from human myelin basic protein. J Exp Med 188:1511-1520.

Sommer, N., R. Martin, H. F. McFarland, L. Quigley, B. Cannella, C. S. Raine, D. E. Scott, P. A. Loschmann, and M. K. Racke. 1997. Therapeutic potential of phosphodiesterase type 4 inhibition in chronic autoimmune demyelinating disease. J Neuroimmunol 79:54-61.

Steinman, L. 1996. Multiple sclerosis: a coordinated immunological attack against myelin in the central nervous system. Cell 85:299-302.

Suvannavejh GC, Dal Canto MC, Matis LA, Miller SD. Fas-mediated apoptosis in clinical remissions of relapsing experimental autoimmune encephalomyelitis. J Clin Invest 2000; 105(2):223-31.

Tam, P.; Clavijo, P.; Lu, Y.; Nussenzweig, S.; Nussenzweig, S.; Zavala, F. J. Exp. Med 1990,171,299.

Tamilarasu, N.; Huq, I.; Rana, T. Bioor. Med. Chem. Let. 2000, 10, 971.

Tselios, T. V., F. N. Lamari, I. Karathanasopoulou, M. Katsara, V. Apostolopoulos, G. A. Pietersz, J. M. Matsoukas, and N. K. Karamanos. 2005. Synthesis and study of the electrophoretic behavior of mannan conjugates with cyclic peptide analogue of myelin basic protein using lysine-glycine linker. Anal Biochem 347:121-128.

Tselios, T., I. Daliani, L. Probert, S. Deraos, E. Matsoukas, S. Roy, J. Pires, G. Moore, and J. Matsoukas. 2000. Treatment of experimental allergic encephalomyelitis (EAE) induced by guinea pig myelin basic protein epitope 72-85 with a human MBP(87-99) analogue and effects of cyclic peptides. Bioorg Med Chem 8:1903-1909.

Tselios, T., I. Daliani, S. Deraos, S. Thymianou, E. Matsoukas, A. Troganis, I. Gerothanassis, A. Mouzaki, T. Mavromoustakos, L. Probert, and J. Matsoukas. 2000. Treatment of experimental allergic encephalomyelitis (EAE) by a rationally designed cyclic analogue of myelin basic protein (MBP) epitope 72-85. Bioorg Med Chem Lett 10:2713-2717.

Tselios, T., L. Probert, I. Daliani, E. Matsoukas, A. Troganis, I. P. Gerothanassis, T. Mavromoustakos, G. J. Moore, and J. M. Matsoukas. 1999. Design and synthesis of a potent cyclic analogue of the myelin basic protein epitope MBP72-85: importance of the Ala81 carboxyl group and of a cyclic conformation for induction of experimental allergic encephalomyelitis. J Med Chem 42:1170-1177.

Tselios, T., V. Apostolopoulos, I. Daliani, S. Deraos, S. Grdadolnik, T. Mavromoustakos, M. Melachrinou, S. Thymianou, L. Probert, A. Mouzaki, and J. Matsoukas. 2002. Antagonistic effects of human cyclic MBP(87-99) altered peptide ligands in experimental allergic encephalomyelitis and human T-cell proliferation. J Med Chem 45:275-283.

Valli, A., A. Sette, L. Kappos, C. Oseroff, J. Sidney, G. Miescher, M. Hochberger, E. D. Albert, and L. Adorini. 1993. Binding of myelin basic protein peptides to human histocompatibility leukocyte antigen class II molecules and their recognition by T cells from multiple sclerosis patients. J Clin Invest 91:616-628.

Vaughan, H. A., D. W. Ho, V. A. Karanikas, C. S. Ong, L. A. Hwang, J. M. Pearson, I. F. McKenzie, and G. A. Pietersz. 1999. Induction of humoral and cellular responses in cynomolgus monkeys immunized with mannan-human MUC1 conjugates. Vaccine 17:2740-2752.

Vergeli, M.; Hemmer, B.; Utz, U.; Vogt, A.; Kalbus, M.; Tranquill, L.; Conlon, P.; Ling, N.; Steinman, L.; McFarland, H.; Martin R. Eur. J. Immunol. 1996, 26, 2624.

Wallace, A. C., R. A. Laskowski, and J. M. Thornton. 1995. LIGPLOT: a program to generate schematic diagrams of protein-ligand interactions. Protein engineering 8:127-134.

Wang, J. H., R. Meijers, Y. Xiong, J. H. Liu, T. Sakihama, R. Zhang, A. Joachimiak, and E. L. Reinherz. 2001. Crystal structure of the human CD4 N-terminal two-domain fragment complexed to a class II MHC molecule. Proc Natl Acad Sci U S A 98:10799-10804.

Willenborg, D. O., and M. A. Staykova. 2003. Cytokines in the pathogenesis and therapy of autoimmune encephalomyelitis and multiple sclerosis. Adv Exp Med Biol 520:96-119.

Wucherpfennig, K. W., D. A. Hafler, and J. L. Strominger. 1995. Structure of human T-cell receptors specific for an immunodominant myelin basic protein peptide: positioning of T-cell receptors on HLA-DR2/peptide complexes. Proc Natl Acad Sci U S A 92:8896-8900.

Zamvil, S. S., and L. Steinman. 1990. The T lymphocyte in experimental allergic encephalomyelitis. Annu Rev Immunol 8:579-621.

Zamvil, S., P. Nelson, J. Trotter, D. Mitchell, R. Knobler, R. Fritz, and L. Steinman. 1985. T-cell clones specific for myelin basic protein induce chronic relapsing paralysis and demyelination. Nature 317:355-358.

Zhang, J., S. Markovic-Plese, B. Lacet, J. Raus, H. L. Weiner, and D. A. Hafler. 1994. Increased frequency of interleukin 2-responsive T cells specific for myelin basic protein and proteolipid protein in peripheral blood and cerebrospinal fluid of patients with multiple sclerosis. J Exp Med 179:973-984.

Zhu, Y., A. Y. Rudensky, A. L. Corper, L. Teyton, and I. A. Wilson. 2003. Crystal structure of MHC class II I-Ab in complex with a human CLIP peptide: prediction of an I-Ab peptide-binding motif. J Mol Biol 326:1157-1174.

## Claims

1. A peptide comprising the amino acid sequence of formula (I),

ENPVVHFF$K^{91}$NIVT$P^{96}$RTP (I)

wherein $K^{91}$ is substituted by an amino acid selected from A, R, E, F and Y and $P^{96}$ is substituted by the amino acid A, wherein said peptide is in linear or cyclic form.

2. A peptide according to any preceding claim which is selected from the following:

| | |
|---|---|
| [$R^{91}$,$A^{96}$]$MBP_{83-99}$ | ENPVVHFFRNIVTARTP |
| [$A^{91}$,$A^{96}$]$MBP_{83-99}$ | ENPVVHFFANIVTARTP |

3. A peptide comprising the amino acid sequence of formula (Ib),

ENPVVHFF$K^{91}$NIVT$P^{96}$RTP (Ib)

wherein at least one of $K^{91}$ and $P^{96}$ is substituted by an amino acid selected from R, E, F and Y.

4. A peptide according to claim 3 which is selected from the following:

| | |
|---|---|
| [$E^{91}$]$MBP_{83-99}$ | ENPVVHFFENIVTPRTP |
| [$F^{91}$]$MBP_{83-99}$ | ENPVVHFFFNIVTPRTP |
| [$Y^{91}$]$MBP_{83-99}$ | ENPVVHFFYNIVTPRTP |

5. A peptide according to any preceding claim wherein the peptide is in cyclic form.

6. A peptide consisting of the amino acid sequence of formula (IIa),

VHFF$K^{91}$NIVT$P^{96}$RTP (IIa)

wherein $K^{91}$ is substituted by the amino acid A and $P^{96}$ is substituted by the amino acid A, and wherein said peptide is cyclised head to tail.

7. A conjugate comprising a peptide according to any one of claims 1 to 6 and mannan, preferably where the mannan is reduced mannan.

8. A conjugate according to claim 7 wherein the peptide is linked to the mannan via a linker group, more preferably where the linker group is a Keyhole Limpit Hemocyanin (KLH).

9. A peptide according to any one of claims 1 to 6, or a conjugate according to any one of claims 7 and 8, for use in medicine.

10. Use of a peptide according to any one of claims 1 to 6, or a conjugate according to any one of claims 7 and 8, in the preparation of a medicament for treating an immune disorder.

11. Use according to claim 10 wherein the immune disorder is an autoimmune disease, preferably multiple sclerosis (MS), or experimental autoimmune encephalomyelitis (EAE).

12. A pharmaceutical composition comprising a peptide according to any one of claims 1 to 6, or a conjugate according to any one of claims 7 and 8, admixed with a pharmaceutically acceptable diluent, excipient or carrier.

13. Use of a peptide according to any one of claims 1 to 6, or a conjugate according to any one of claims 7 and 8, in

an assay for elucidating agents capable of regulating experimental autoimmune encephalomyelitis (EAE) or regulating multiple sclerosis.

**14.** A process for preparing a conjugate according to claim 8, said process comprising the steps of:

(i) reacting a peptide according to any one of claims 1 to 6 with a Keyhole Limpit Hemocyanin (KLH);
(ii) reacting the product formed in step (i) with oxidized mannan; and
(iii) reducing the product formed in step (ii) to form a reduced mannan conjugate.

**15.** A peptide according to any one of claims 1 to 6, or a conjugate according to any one of claims 7 and 8 for use in the treatment of an autoimmune disease.

**16.** A peptide according to any one of claims 1 to 6, or a conjugate according to any one of claims 7 and 8 for use in diverting the immune response in a subject from a Th1 response to a Th2 response or for inducing a Th2-specific immune response in a subject.

**17.** Use according to any one of claims 10 or 11 wherein the peptide or conjugate is to be administered in an amount sufficient to inversely modulate the Th1 and Th2 responses of lymphocytes in the subject so as to increase the Th2 response and decrease the Th1 response respectively above and below the levels prevailing without said treatment.

**18.** Use according to any one of claims 10 or 11 wherein the peptide according to any one of claims 1 to 6, or a conjugate according to any one of claims 7 and 8 is to be administered in an amount sufficient to enhance/induce the Th2-type response in a subject, as compared to the Th2-type response prior to peptide/conjugate administration and reduce the Th1-type response as compared to the Th1-type response prior to peptide/conjugate administration.

**19.** A vaccine composition comprising a peptide according to any one of claims 1 to 6 or a conjugate according to any one of claims 7 and 8.

**20.** Use of a peptide according to any one of claims 1 to 6 or a conjugate according to any one of claims 7 and 8 in the preparation of a vaccine composition.

**Patentansprüche**

**1.** Peptid, umfassend die Aminosäuresequenz der Formel (I),

$$ENPVVHFFK^{91}NIVTP^{96}RTP \qquad (I)$$

wobei $K^{91}$ durch eine aus A, R, E, F und Y ausgewählte Aminosäure und $P^{96}$ durch die Aminosäure A substituiert ist, wobei das Peptid in linearer oder cyclischer Form vorliegt.

**2.** Peptid nach einem vorhergehenden Anspruch, das aus Folgendem ausgewählt ist:

$[R^{91},A^{96}]MBP_{83-99}$ ENPVVHFFRNIVTARTP
$[A^{91},A^{96}]MBP_{83-99}$ ENPVVHFFANIVTARTP.

**3.** Peptid, umfassend die Aminosäuresequenz der Formel (Ib),

$$ENPVVHFFK^{91}NIVTP^{96}RTP \qquad (Ib)$$

wobei $K^{91}$ oder/und $P^{96}$ durch eine aus R, E, F und Y ausgewählte Aminosäure substituiert ist/sind.

**4.** Peptid nach Anspruch 3, das aus Folgendem ausgewählt ist:

$[E^{91}]MBP_{83-99}$ ENPVVHFFENIVTPRTP

(fortgesetzt)

| | |
|---|---|
| $[F^{91}]MBP_{83-99}$ | ENPVVHFFFNIVTPRTP |
| $[Y^{91}]MBP_{83-99}$ | ENPVVHFFYNIVTPRTP. |

**5.** Peptid nach einem vorhergehenden Anspruch, wobei das Peptid in cyclischer Form vorliegt.

**6.** Peptid, bestehend aus der Aminosäuresequenz der Formel (IIa),

$$VHFFK^{91}NIVTP^{96}RTP \quad \text{(IIa)}$$

wobei $K^{91}$ durch die Aminosäure A und $P^{96}$ durch die Aminosäure A substituiert ist und wobei das Peptid Kopfende zu Fußende cyclisiert ist.

**7.** Konjugat, umfassend ein Peptid nach einem der Ansprüche 1 bis 6 und Mannan, vorzugsweise wobei es sich bei dem Mannan um reduziertes Mannan handelt.

**8.** Konjugat nach Anspruch 7, wobei das Peptid mit dem Mannan über eine Linkergruppe verknüpft ist, stärker bevorzugt wobei es sich bei der Linkergruppe um ein KLH (Keyhole Limpit Hemocyanin) handelt.

**9.** Peptid nach einem der Ansprüche 1 bis 6 oder Konjugat nach einem der Ansprüche 7 und 8 zur Verwendung in der Medizin.

**10.** Verwendung eines Peptids nach einem der Ansprüche 1 bis 6 oder eines Konjugats nach einem der Ansprüche 7 und 8 bei der Herstellung eines Arzneimittels zur Behandlung einer Immunkrankheit.

**11.** Verwendung nach Anspruch 10, wobei es sich bei der Immunkrankheit um eine Autoimmunkrankheit, vorzugsweise multiple Sklerose (MS) oder experimentelle Autoimmunenzephalomyelitis (EAE) handelt.

**12.** Pharmazeutische Zusammensetzung, umfassend ein Peptid nach einem der Ansprüche 1 bis 6 oder ein Konjugat nach einem der Ansprüche 7 und 8 im Gemisch mit einem pharmazeutisch unbedenklichen Verdünnungs-, Hilfs- oder Trägermittel.

**13.** Verwendung eines Peptids nach einem der Ansprüche 1 bis 6 oder eines Konjugats nach einem der Ansprüche 7 und 8 bei einem Testverfahren zum Aufklären von Agentien, die experimentelle Autoimmunenzephalomyelitis (EAE) oder multiple Sklerose regulieren können.

**14.** Verfahren zur Herstellung eines Konjugats nach Anspruch 8, wobei das Verfahren die folgenden Schritte umfasst:

(i) Umsetzen eines Peptids nach einem der Ansprüche 1 bis 6 mit einem KLH (Keyhole Limpit Hemocyanin);
(ii) Umsetzen des in Schritt (i) gebildeten Produkts mit oxidiertem Mannan; und
(iii) Reduzieren des in Schritt (ii) gebildeten Produkts unter Bildung eines reduzierten Mannankonjugats.

**15.** Peptid nach einem der Ansprüche 1 bis 6 oder Konjugat nach einem der Ansprüche 7 und 8 zur Verwendung bei der Behandlung einer Autoimmunkrankheit.

**16.** Peptid nach einem der Ansprüche 1 bis 6 oder Konjugat nach einem der Ansprüche 7 und 8 zur Verwendung beim Umleiten der Immunantwort bei einem Individuum von einer Th1-Antwort zu einer Th2-Antwort oder zum Induzieren einer Th2-spezifischen Immunantwort bei einem Patienten.

**17.** Verwendung nach einem der Ansprüche 10 oder 11, wobei das Peptid oder Konjugat in einer hinreichenden Menge zu verabreichen ist, um die Th1-und Th2-Antworten von Lymphozyten im Individuum invers zu modulieren, so dass die Th2-Antwort über das ohne die Behandlung herrschende Niveau angehoben bzw. die Th1-Antwort unter das ohne die Behandlung herrschende Niveau abgesenkt wird.

**18.** Verwendung nach einem der Ansprüche 10 oder 11, wobei das Peptid nach einem der Ansprüche 1 bis 6 oder ein Konjugat nach einem der Ansprüche 7 und 8 in einer hinreichenden Menge zu verabreichen ist, um die Antwort vom Th2-Typ bei einem Individuum im Vergleich zur Antwort vom Th2-Typ vor Peptid/Konjugat-Verabreichung zu

verbessern/induzieren und die Antwort vom Th1-Typ im Vergleich zur Antwort vom Th1-Typ vor Peptid/Konjugat-Verabreichung zu reduzieren.

**19.** Impfstoffzusammensetzung, umfassend ein Peptid nach einem der Ansprüche 1 bis 6 oder ein Konjugat nach einem der Ansprüche 7 und 8.

**20.** Verwendung eines Peptids nach einem der Ansprüche 1 bis 6 oder eines Konjugats nach einem der Ansprüche 7 und 8 bei der Herstellung einer Impfstoffzusammensetzung.

## Revendications

**1.** Peptide comprenant la séquence d'acides aminés de formule (I),

$ENPVVHFFK^{91}NIVTP^{96}RTP$ (I)

dans laquelle $K^{91}$ est substitué par un acide aminé choisi parmi les A, R, E, F et Y, et $P^{96}$ est substitué par l'acide aminé A, dans lequel ledit peptide est sous forme linéaire ou cyclique.

**2.** Peptide, selon l'une quelconque des revendications précédentes, qui est choisi parmi ce qui suit :

| | |
|---|---|
| $[R^{91}, A^{96}]$ $MBP_{83-99}$ | ENPVVHFFRNIVTARTP |
| $[A^{91}, A^{96}]$ $MBP_{83-99}$ | ENPVVHFFANTVTARTP |

**3.** Peptide comprenant la séquence d'acides aminés de formule (Ib),

$ENPVVHFFK^{91}NIVTP^{96}RTP$ (Ib)

dans laquelle au moins un parmi les $K^{91}$ et $P^{96}$ est substitué par un acide aminé choisi parmi les R, E, F et Y.

**4.** Peptide, selon la revendication 3, qui est choisi parmi ce qui suit :

| | |
|---|---|
| $[E^{91}]MBP_{83-99}$ | ENPVVHFFENIVTPRTP |
| $[F^{91}]MBP_{83-99}$ | ENPVVHFFFNIVTPRTP |
| $[Y^{91}]MBP_{83-99}$ | ENPVVHFFYNIVTPRTP |

**5.** Peptide selon l'une quelconque des revendications précédentes, dans laquelle le peptide est sous forme cyclique.

**6.** Peptide constitué par la séquence d'acides aminés de formule (IIa),

$VHFFK^{91}NIVTP^{96}RTP$ (IIa)

dans laquelle $K^{91}$ est substitué par l'acide aminé A et $P^{96}$ est substitué par l'acide aminé A, et dans lequel ledit peptide est cyclisé "en tête à queue".

**7.** Conjugué comprenant un peptide, selon l'une quelconque des revendications 1 à 6, et du mannane, de préférence où le mannane est un mannane réduit.

**8.** Conjugué selon la revendication 7, dans laquelle le peptide est lié au mannane via un groupe lieur, mieux préféré dans laquelle le groupe lieur est une hémocyanine de patelle (KLH).

**9.** Peptide selon l'une quelconque des revendications 1 à 6, ou conjugué, selon l'une quelconque des revendications 7 et 8, destiné à être utilisé en médecine.

**10.** Utilisation d'un peptide, selon l'une quelconque des revendications 1 à 6, ou d'un conjugué, selon l'une quelconque des revendications 7 et 8, dans la préparation d'un médicament destiné à traiter un trouble immunologique.

**11.** Utilisation selon la revendication 10, dans laquelle le trouble immunologique est une maladie auto-immune, de préférence la sclérose en plaques (SEP) ou une encéphalomyélite auto-immune expérimentale (EAE).

**12.** Composition pharmaceutique comprenant un peptide, selon l'une quelconque des revendications 1 à 6, ou un conjugué, selon l'une quelconque des revendications 7 et 8, mélangé avec un diluant, un excipient ou un transporteur acceptable d'un point de vue pharmaceutique.

**13.** Utilisation d'un peptide, selon l'une quelconque des revendications 1 à 6, ou d'un conjugué, selon l'une quelconque des revendications 7 et 8, dans un dosage destiné à élucider des agents étant capables de réguler l'encéphalomyélite auto-immune expérimentale (EAE) ou de réguler la sclérose en plaques (SEP).

**14.** Processus de préparation d'un conjugué, selon la revendication 8, ledit processus comprenant les étapes consistant à :

(i) faire réagir un peptide, selon l'une quelconque des revendications 1 à 6, avec une hémocyanine de patelle (KLH) ;
(ii) faire réagir le produit formé dans l'étape (i) avec du mannane oxydé ; et
(iii) réduire le produit formé dans l'étape (ii) pour former un conjugué de mannane réduit.

**15.** Peptide selon l'une quelconque des revendications 1 à 6, ou conjugué, selon l'une quelconque des revendications 7 et 8, destiné à être utilisé dans le traitement d'une maladie auto--immune.

**16.** Peptide selon l'une quelconque des revendications 1 à 6, ou conjugué, selon l'une quelconque des revendications 7 et 8, destiné à être utilisé dans le détournement de la réponse immunitaire chez un sujet, à partir d'une réponse Th1 vers une réponse Th2, ou à induire une réponse immunitaire spécifique de Th2 chez un sujet.

**17.** Utilisation selon l'une quelconque des revendications 10 ou 11, dans laquelle le peptide ou un conjugué doit être administré en une quantité suffisante pour moduler inversement les réponses Th1 et Th2 de lymphocytes chez le sujet, de sorte à augmenter la réponse Th2 et à diminuer la réponse Th1 respectivement au-dessus et en-dessous des niveaux prévalant sans ledit traitement.

**18.** Utilisation selon l'une quelconque des revendications 10 ou 11, dans laquelle le peptide, selon l'une quelconque des revendications 1 à 6, ou un conjugué, selon l'une quelconque des revendications 7 et 8, doit être administré en une quantité suffisante pour renforcer/induire la réponse de type Th2 chez un sujet, en comparaison avec la réponse de type Th2 avant l'administration du peptide/conjugué, et pour réduire la réponse de type Th1, en comparaison avec la réponse de type Th1 avant l'administration du peptide/conjugué.

**19.** Composition vaccinale comprenant un peptide, selon l'une quelconque des revendications 1 à 6, ou un conjugué selon l'une quelconque des revendications 7 et 8.

**20.** Utilisation d'un peptide, selon l'une quelconque des revendications 1 à 6, ou d'un conjugué, selon l'une quelconque des revendications 7 et 8, dans la préparation d'une composition vaccinale.

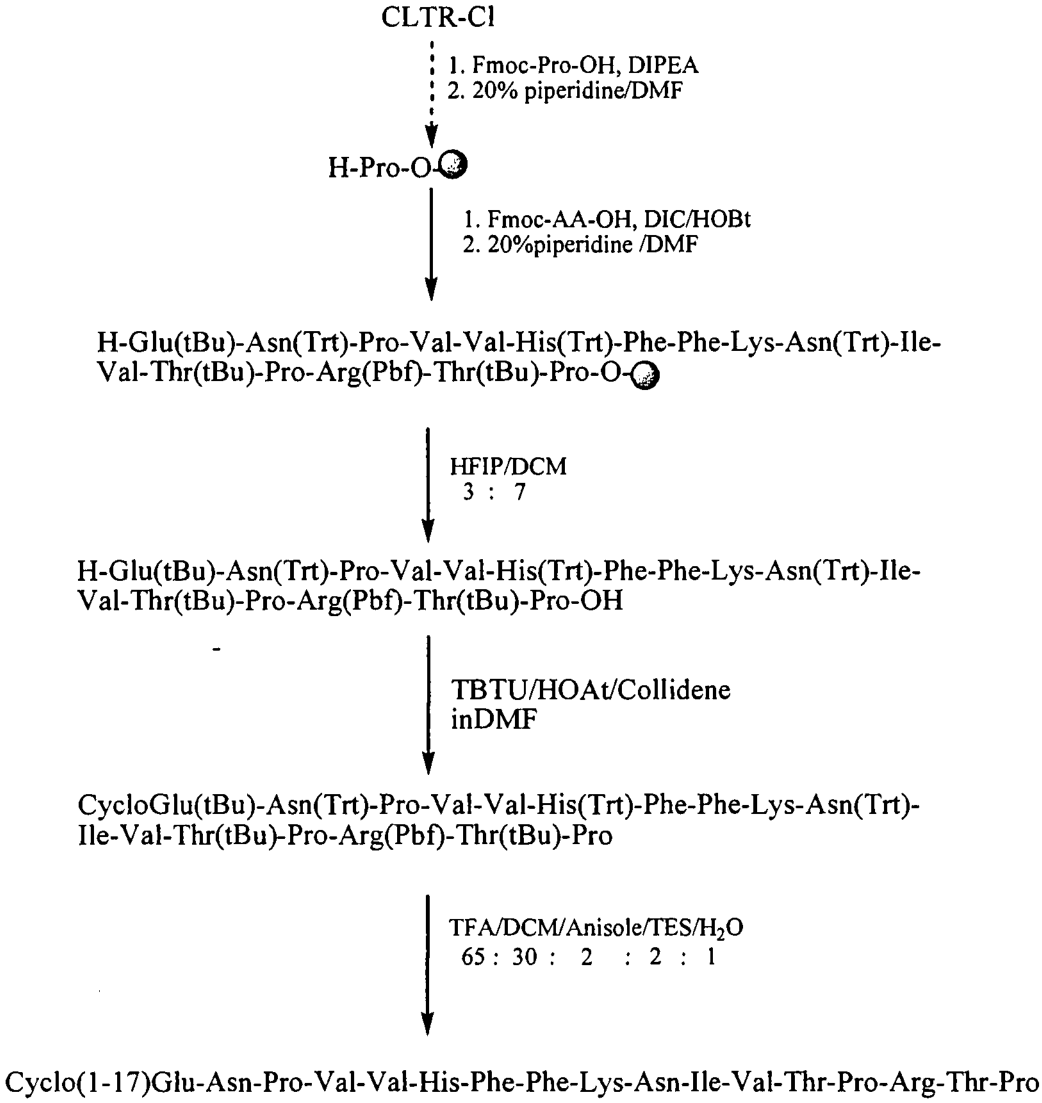
CLTR-Cl
1. Fmoc-Pro-OH, DIPEA
2. 20% piperidine/DMF
H-Pro-O-
1. Fmoc-AA-OH, DIC/HOBt
2. 20%piperidine /DMF
H-Glu(tBu)-Asn(Trt)-Pro-Val-Val-His(Trt)-Phe-Phe-Lys-Asn(Trt)-Ile-
Val-Thr(tBu)-Pro-Arg(Pbf)-Thr(tBu)-Pro-O-
HFIP/DCM
3 : 7
H-Glu(tBu)-Asn(Trt)-Pro-Val-Val-His(Trt)-Phe-Phe-Lys-Asn(Trt)-Ile-
Val-Thr(tBu)-Pro-Arg(Pbf)-Thr(tBu)-Pro-OH
TBTU/HOAt/Collidene
inDMF
CycloGlu(tBu)-Asn(Trt)-Pro-Val-Val-His(Trt)-Phe-Phe-Lys-Asn(Trt)-
Ile-Val-Thr(tBu)-Pro-Arg(Pbf)-Thr(tBu)-Pro
TFA/DCM/Anisole/TES/H2O
65 : 30 : 2 : 2 : 1
Cyclo(1-17)Glu-Asn-Pro-Val-Val-His-Phe-Phe-Lys-Asn-Ile-Val-Thr-Pro-Arg-Thr-Pro

**FIGURE 1**

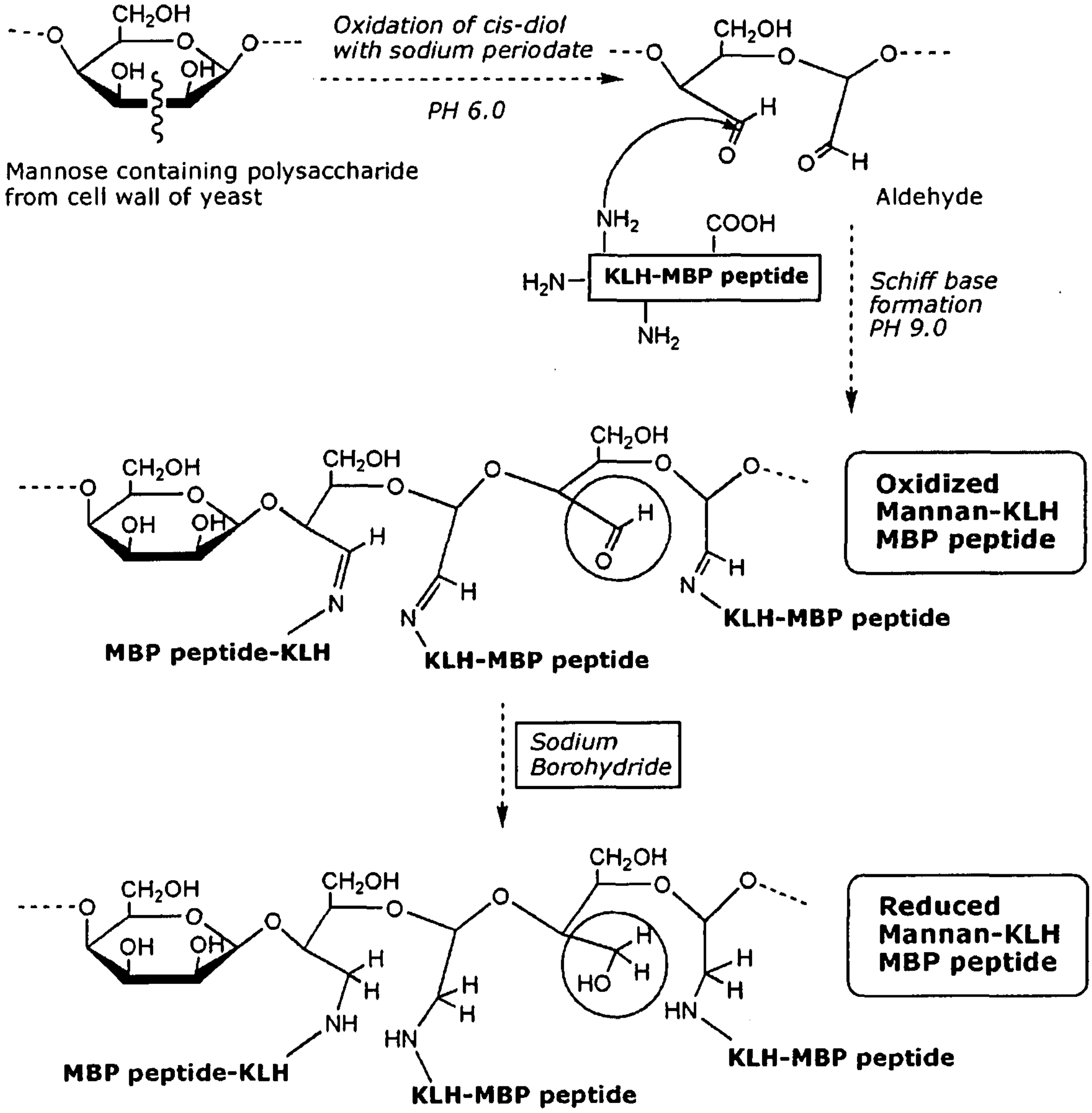
Oxidation of cis-diol
with sodium periodate
PH 6.0
Mannose containing polysaccharide
from cell wall of yeast
Aldehyde
Schiff base
formation
PH 9.0
Oxidized
Mannan-KLH
MBP peptide
Sodium
Borohydride
Reduced
Mannan-KLH
MBP peptide

**FIGURE 2**

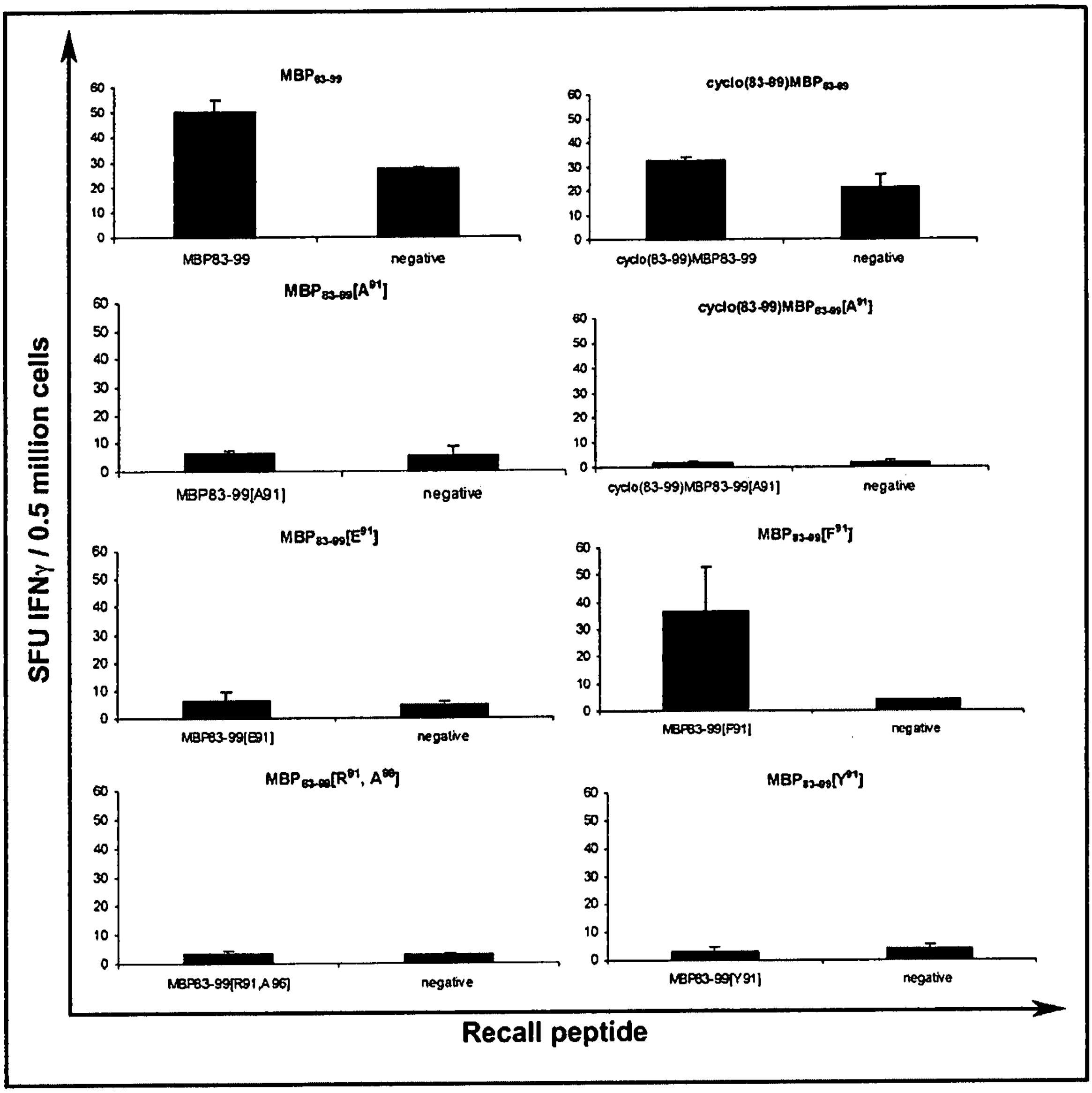
SFU IFNγ / 0.5 million cells
MBP83-99
MBP83-99
negative
cyclo(83-99)MBP83-99
cyclo(83-99)MBP83-99
negative
MBP83-99[A91]
MBP83-99[A91]
negative
cyclo(83-99)MBP83-99[A91]
cyclo(83-99)MBP83-99[A91]
negative
MBP83-99[E91]
MBP83-99[E91]
negative
MBP83-99[F91]
MBP83-99[F91]
negative
MBP83-99[R91, A96]
MBP83-99[R91,A96]
negative
MBP83-99[Y91]
MBP83-99[Y91]
negative
Recall peptide

FIGURE 3

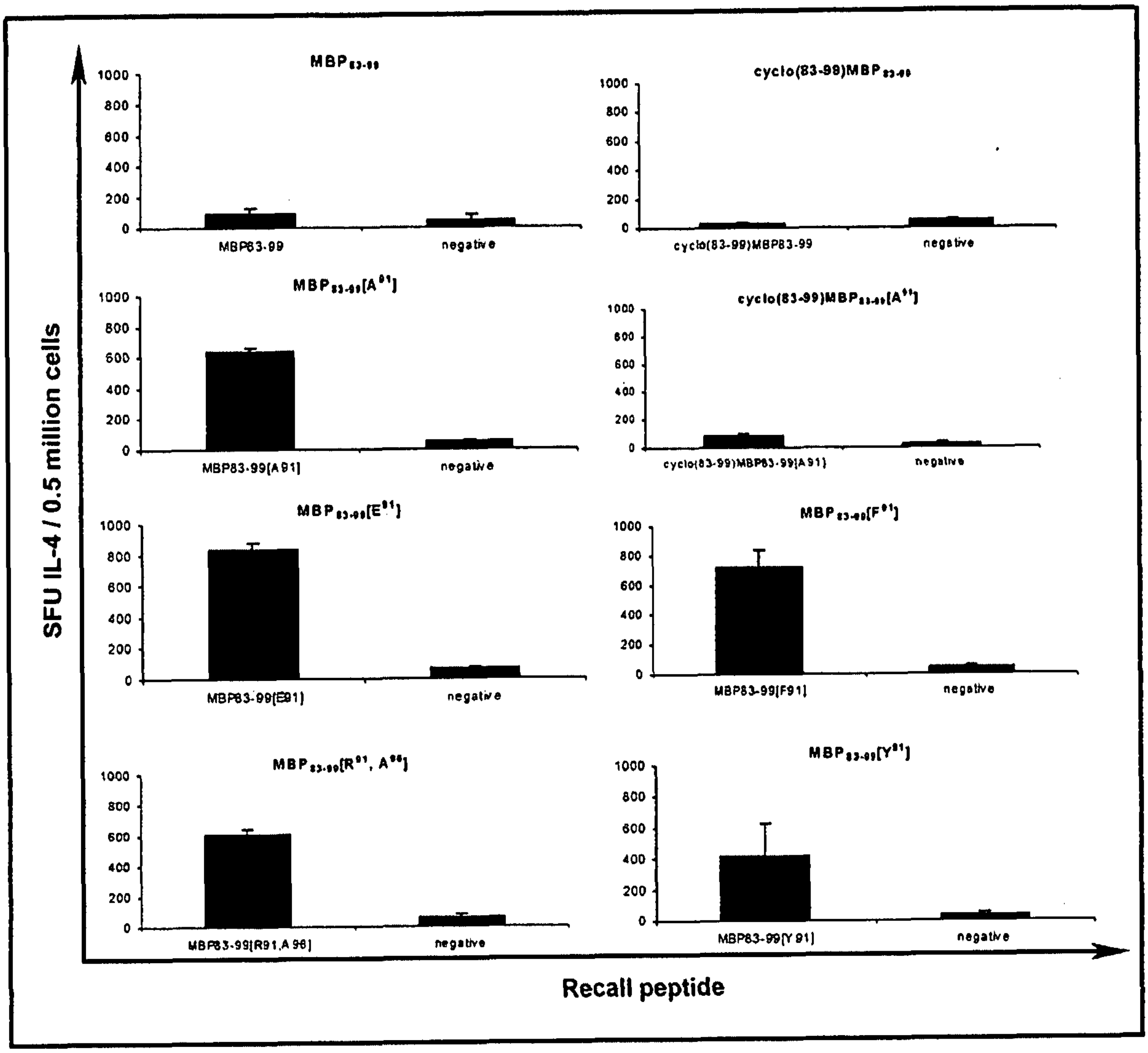
SFU IL-4 / 0.5 million cells
Recall peptide
MBP83-99
cyclo(83-99)MBP83-99
MBP83-99[A91]
cyclo(83-99)MBP83-99[A91]
MBP83-99[E91]
MBP83-99[F91]
MBP83-99[R91, A96]
MBP83-99[Y91]
negative

FIGURE 4

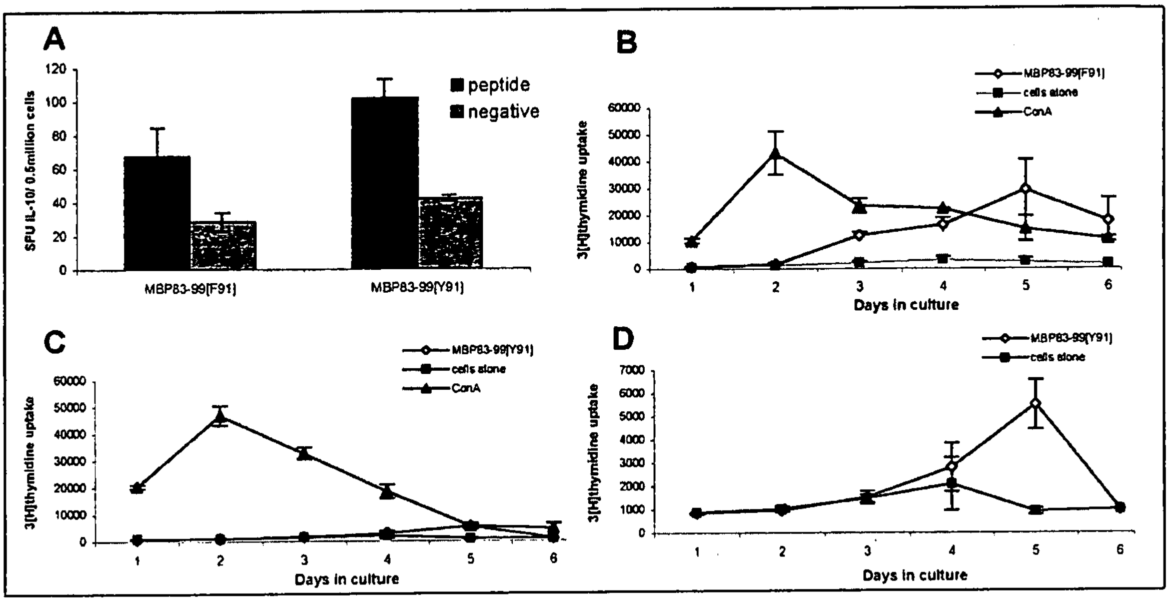


FIGURE 5

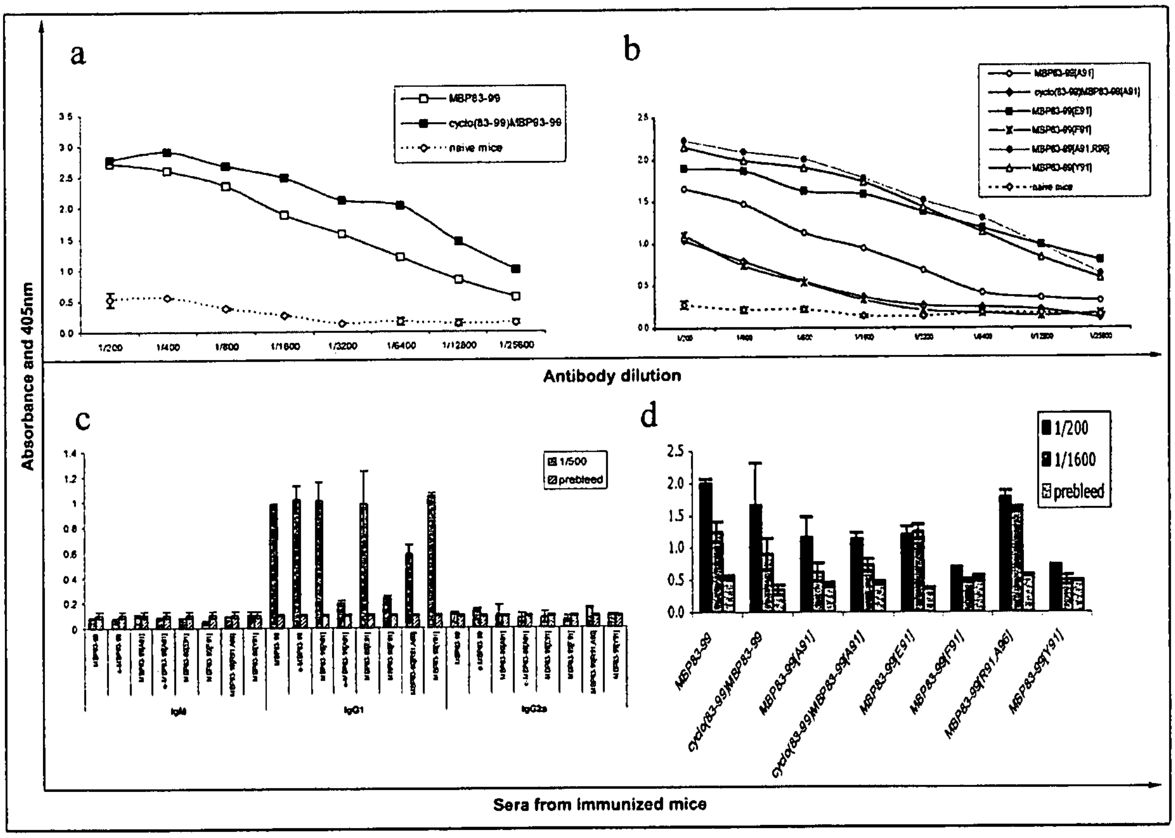


FIGURE 6

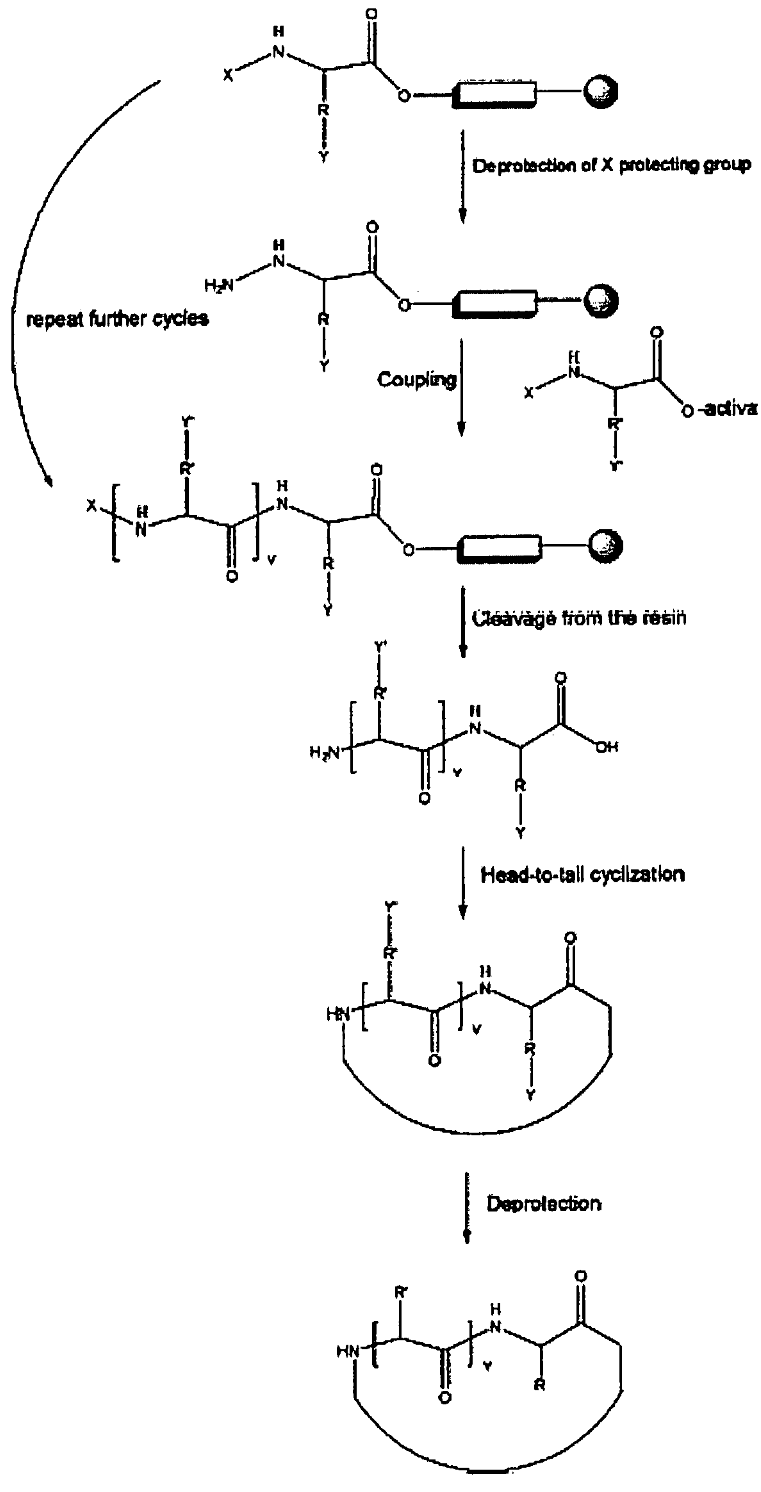
Deprotection of X protecting group
repeat further cycles
Coupling
Cleavage from the resin
Head-to-tail cyclization
Deprotection

FIGURE 7

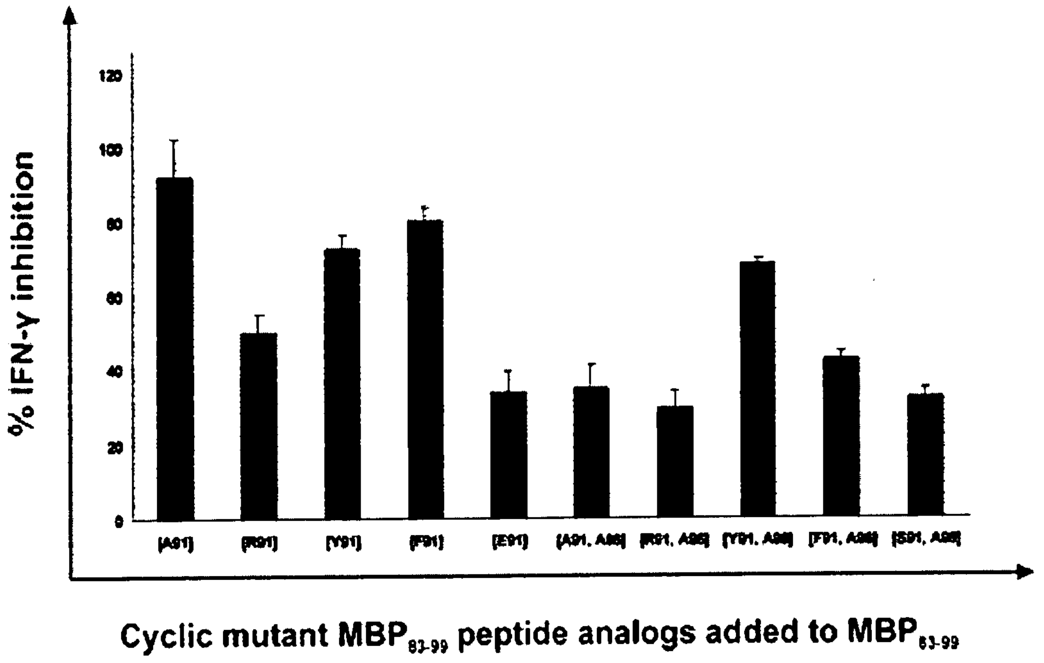
% IFN-γ inhibition
120
100
80
60
40
20
0
[A91]
[R91]
[Y91]
[F91]
[E91]
Cyclic mutant MBP83-99 peptide analogs added to MBP83-99

Figure 8

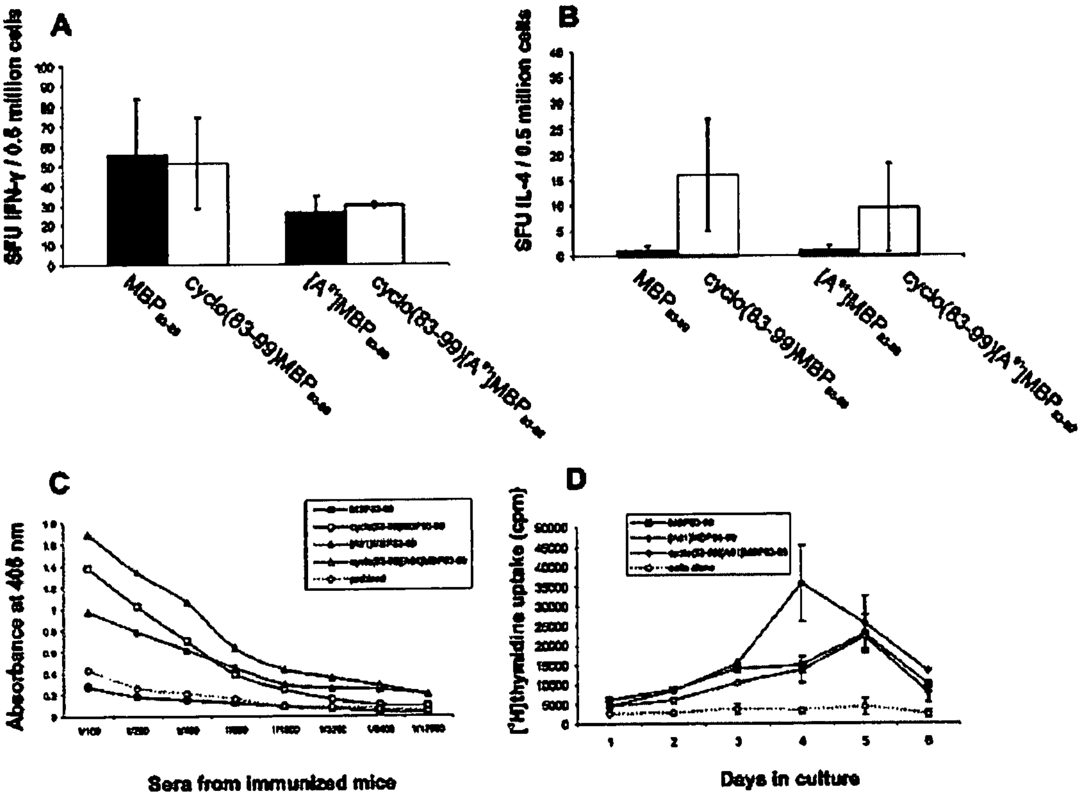
A
SFU IFN-γ / 0.5 million cells
MBP 83-99
cyclo(83-99)MBP 83-99
[A91]MBP 83-99
cyclo(83-99)[A91]MBP 83-99
B
SFU IL-4 / 0.5 million cells
MBP 83-99
cyclo(83-99)MBP 83-99
[A91]MBP 83-99
cyclo(83-99)[A91]MBP 83-99
C
Absorbance at 405 nm
Sera from immunized mice
D
[3H]thymidine uptake (cpm)
Days in culture

Figure 9

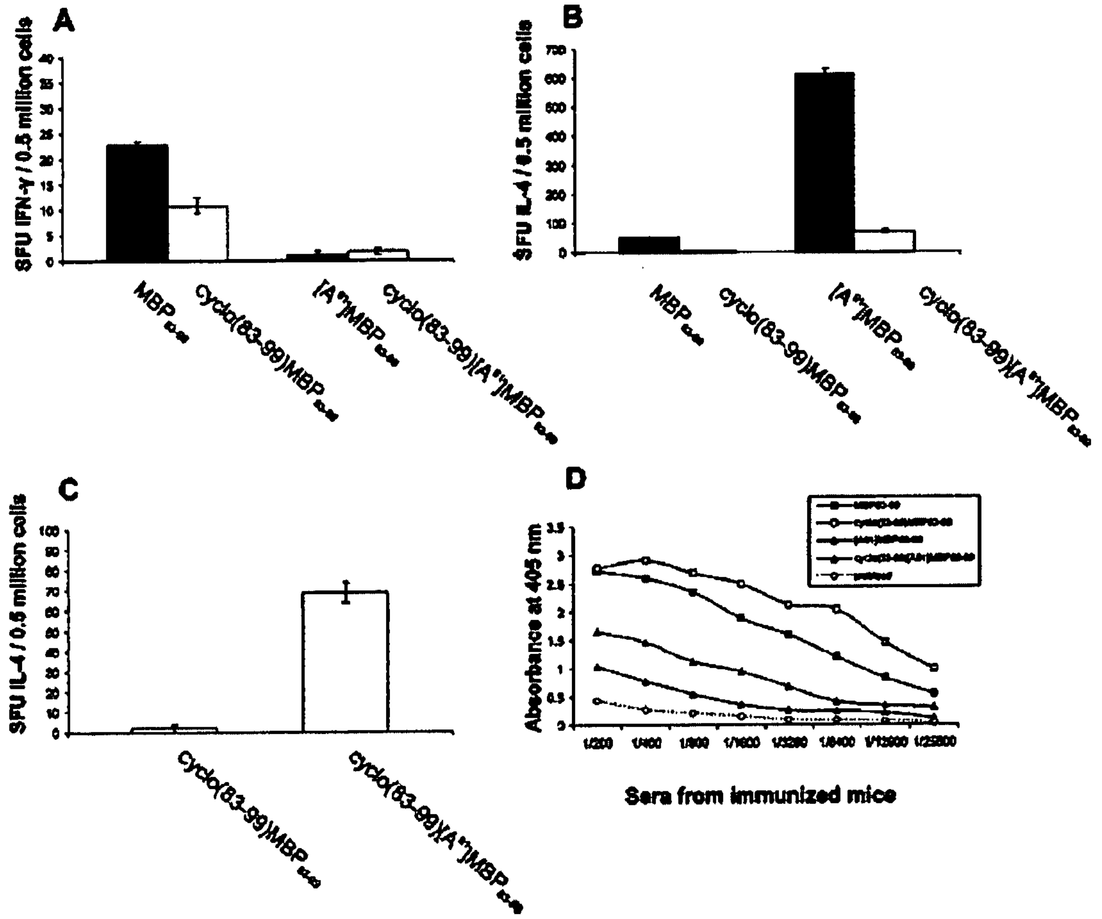
A
SFU IFN-γ / 0.5 million cells
B
SFU IL-4 / 0.5 million cells
C
SFU IL-4 / 0.5 million cells
D
Absorbance at 405 nm
1/200
1/400
1/800
1/1600
1/3200
1/6400
1/12800
1/25600
Sera from immunized mice

FIGURE 10

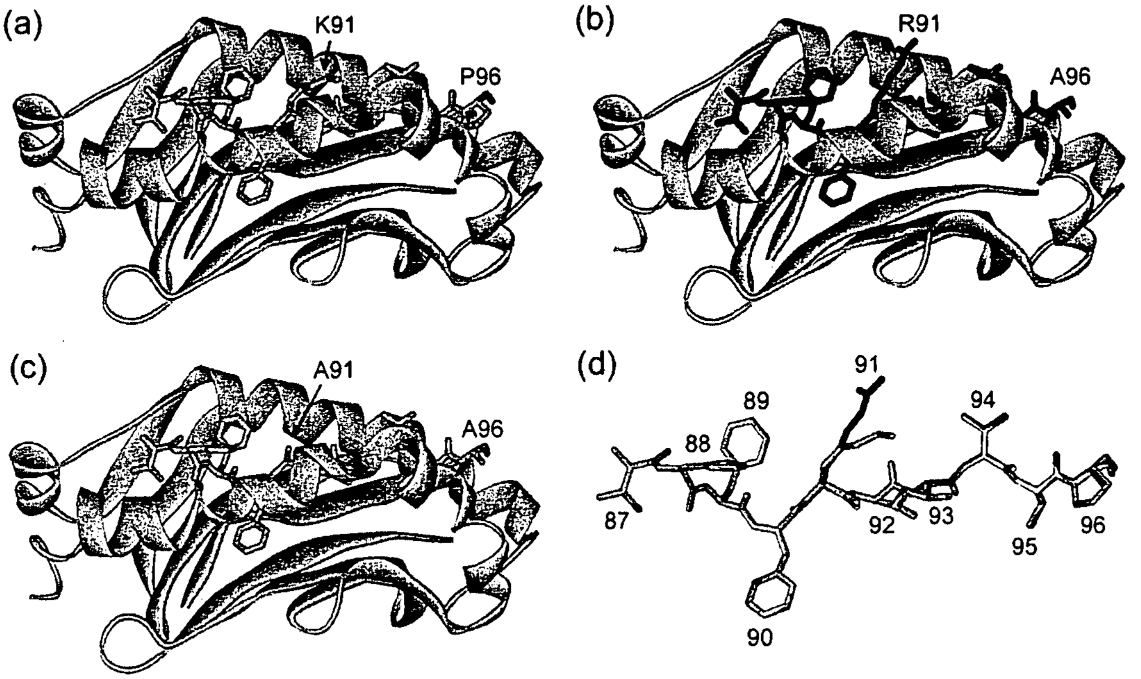


FIGURE 11

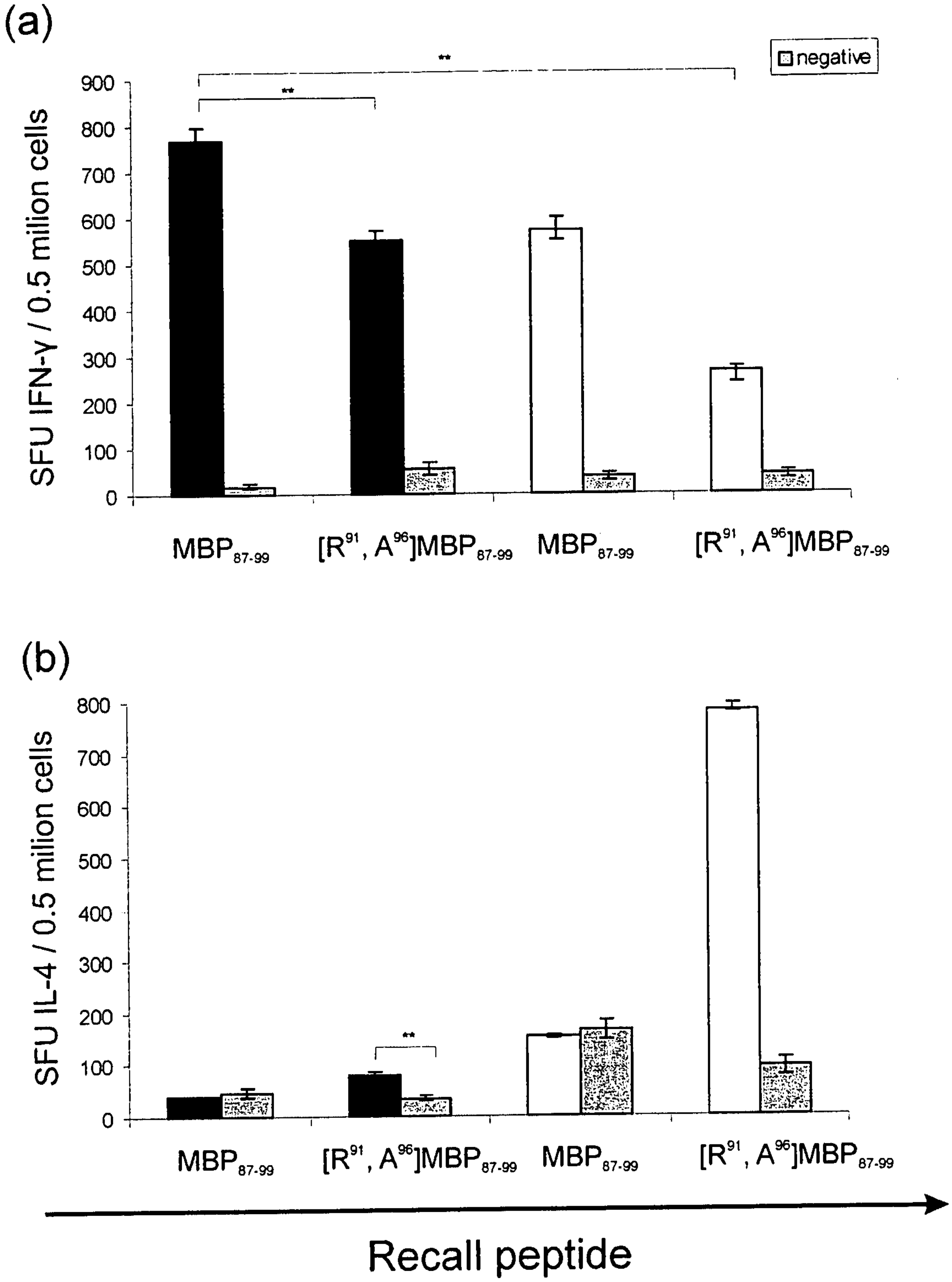
(a)
negative
**
**
SFU IFN-γ / 0.5 milion cells
900
800
700
600
500
400
300
200
100
0
MBP87-99
[R91, A96]MBP87-99
MBP87-99
[R91, A96]MBP87-99
(b)
SFU IL-4 / 0.5 milion cells
800
700
600
500
400
300
200
100
0
**
MBP87-99
[R91, A96]MBP87-99
MBP87-99
[R91, A96]MBP87-99
Recall peptide

FIGURE 12

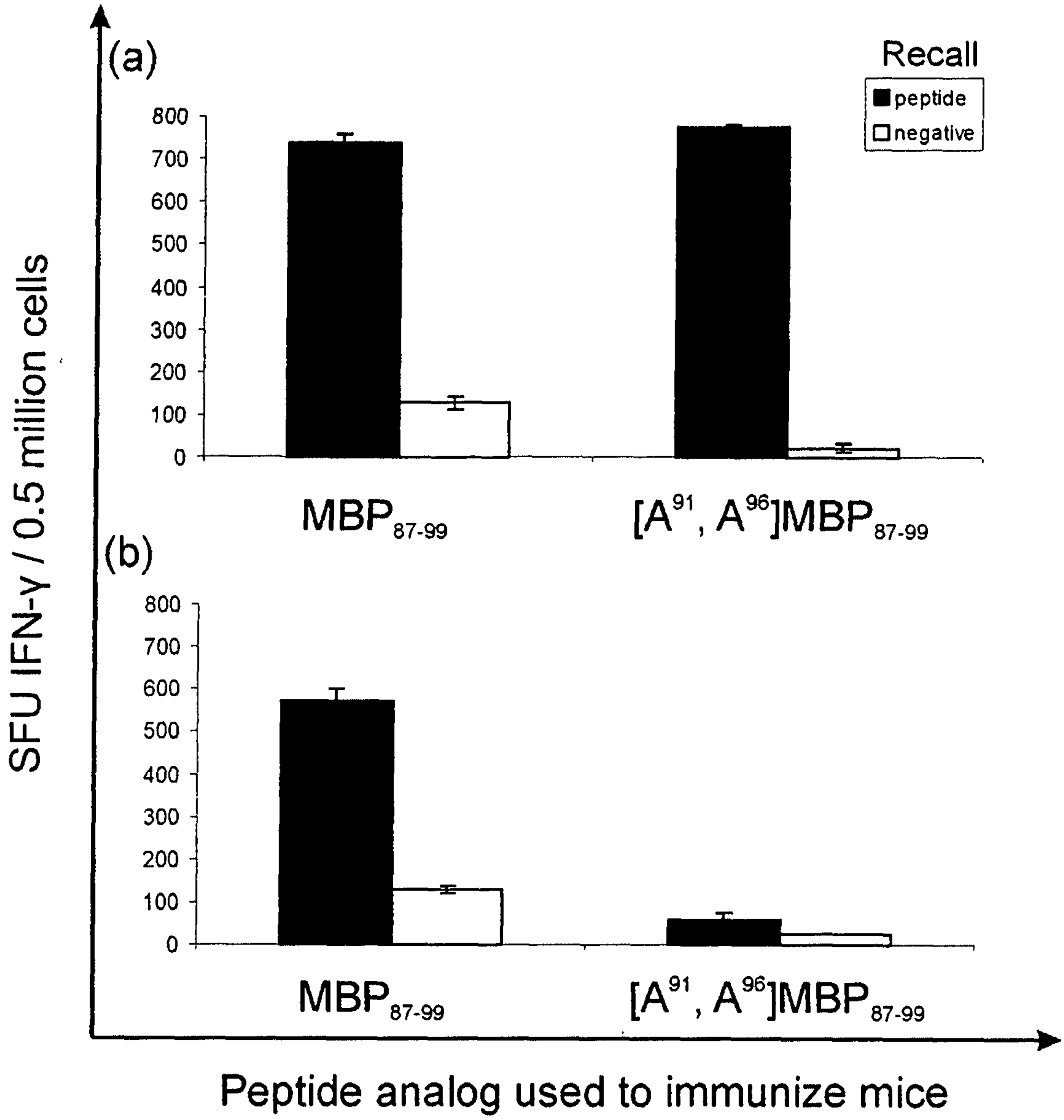
(a)
Recall
peptide
negative
800
700
600
500
400
300
200
100
0
MBP87-99
[A91, A96]MBP87-99
(b)
800
700
600
500
400
300
200
100
0
MBP87-99
[A91, A96]MBP87-99
SFU IFN-γ / 0.5 million cells
Peptide analog used to immunize mice

**FIGURE 13**

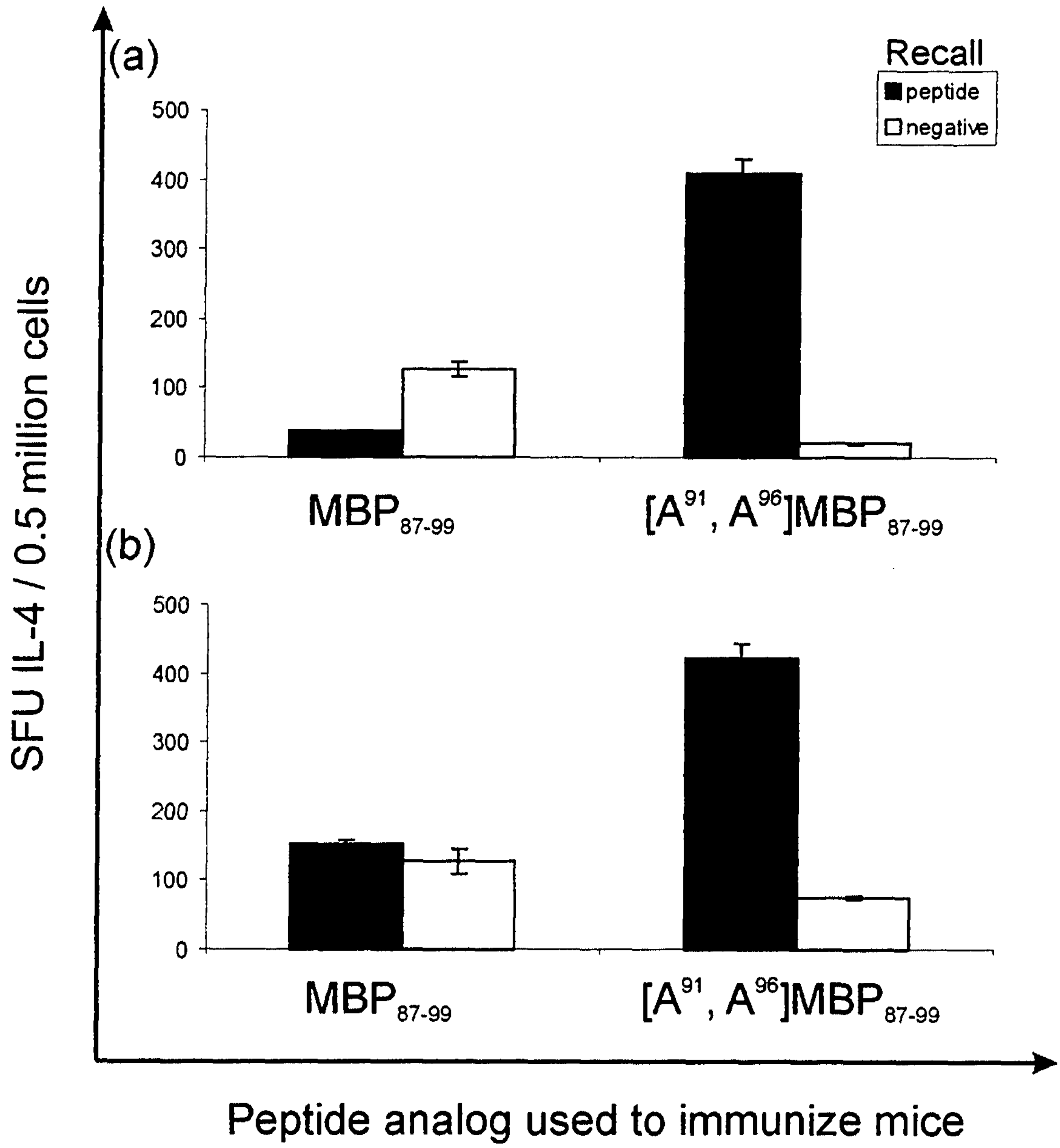
(a)
Recall
peptide
negative
500
400
300
200
100
0
MBP87-99
[A91, A96]MBP87-99
(b)
500
400
300
200
100
0
MBP87-99
[A91, A96]MBP87-99
SFU IL-4 / 0.5 million cells
Peptide analog used to immunize mice

FIGURE 14

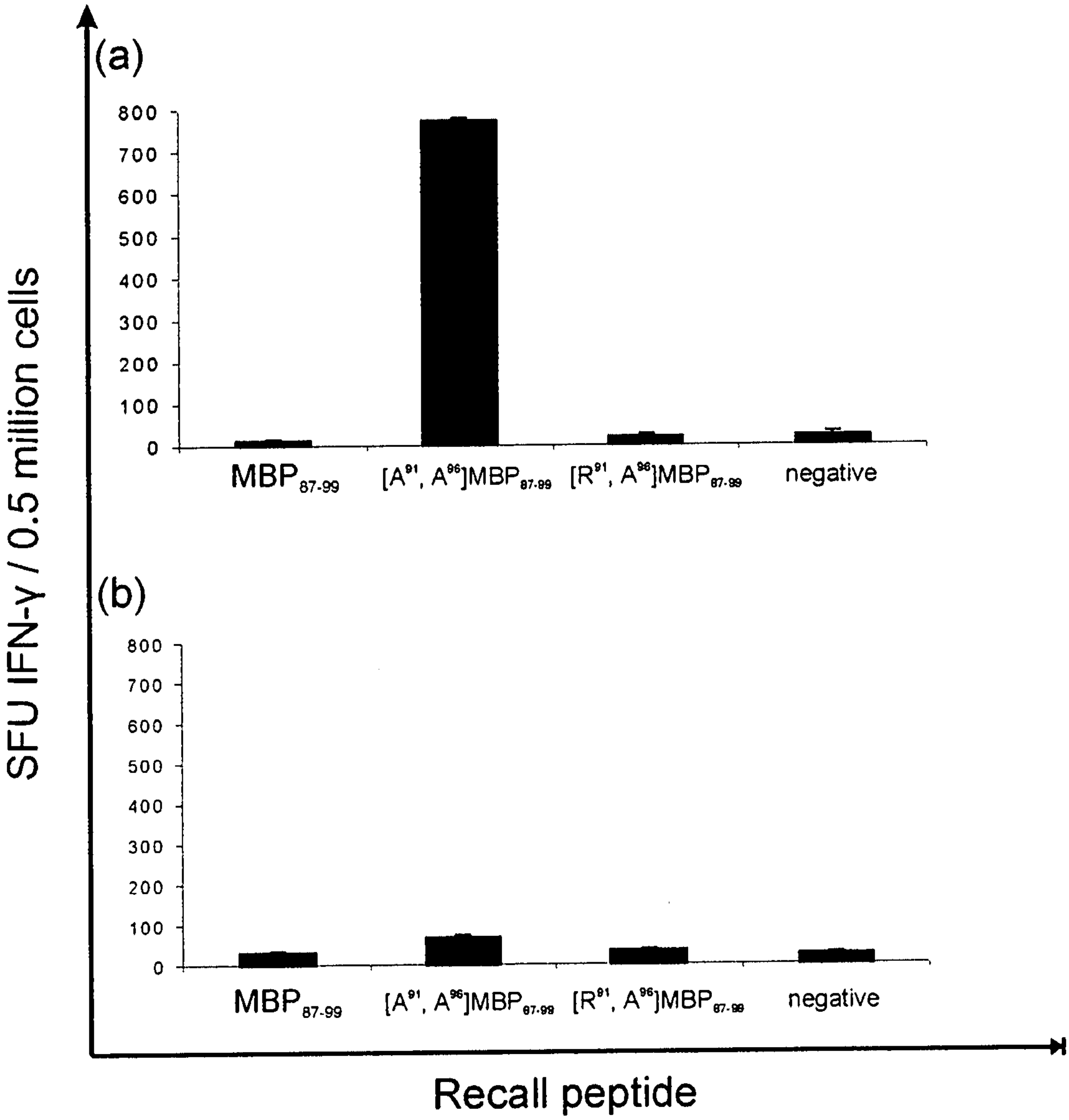

(a)
800
700
600
500
400
300
200
100
0
MBP87-99
[A91, A96]MBP87-99
[R91, A96]MBP87-99
negative
(b)
800
700
600
500
400
300
200
100
0
MBP87-99
[A91, A96]MBP87-99
[R91, A96]MBP87-99
negative
SFU IFN-γ / 0.5 million cells
Recall peptide


FIGURE 15

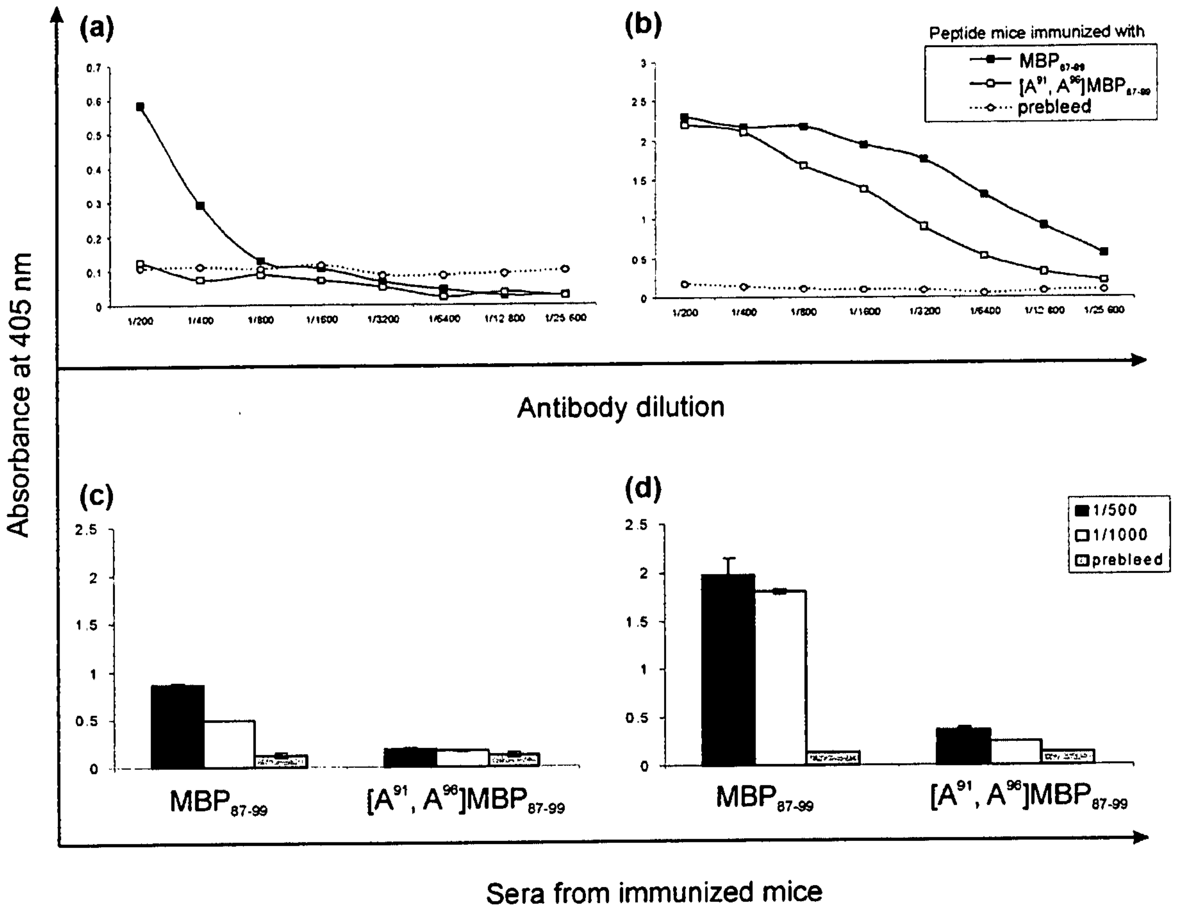
(a)
(b)
Peptide mice immunized with
MBP87-99
[A91, A96]MBP87-99
prebleed
Absorbance at 405 nm
1/200
1/400
1/800
1/1600
1/3200
1/6400
1/12 800
1/25 600
Antibody dilution
(c)
(d)
1/500
1/1000
prebleed
MBP87-99
[A91, A96]MBP87-99
Sera from immunized mice

FIGURE 16

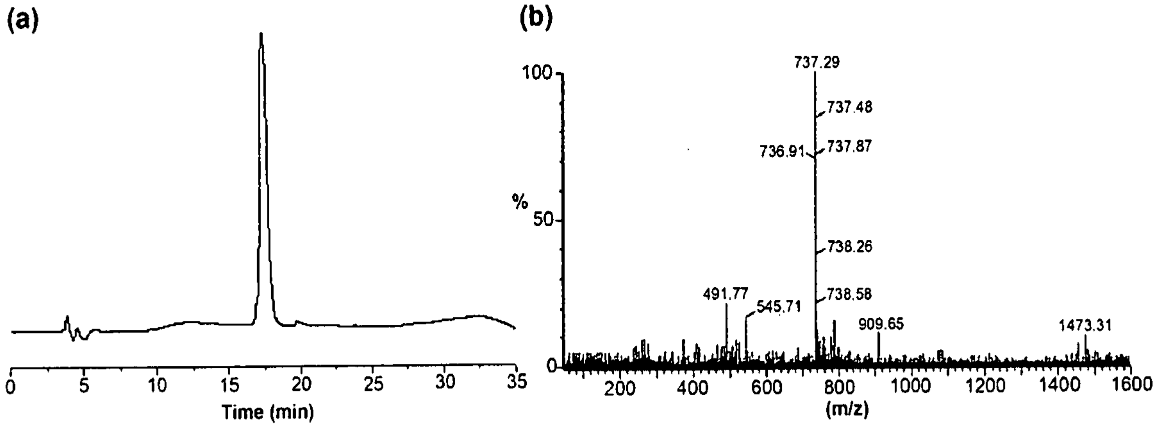
(a)
0
5
10
15
20
25
30
35
Time (min)
(b)
100
%
50
0
737.29
737.48
736.91
737.87
738.26
491.77
545.71
738.58
909.65
1473.31
200
400
600
800
1000
1200
1400
1600
(m/z)

FIGURE 17

| Peptide | | MHC | | $MBP_{87-96}$ | $[A^{91},A^{96}]MBP_{87-96}$ | $[R^{91},A^{96}]MBP_{87-96}$ |
|---|---|---|---|---|---|---|
| residue | atom | residue | atom | | | |
| $V^{87}$ | N | $S^{53}(A)^{\dagger}$ | O | + | + | + |
| $H^{88}$ | N | $N^{82}(B)^{*}$ | OD1 | + | – | + |
| $H^{88}$ | NE2 | $E^{74}(B)$ | OE2 | + | + | + |
| $H^{88}$ | O | $N^{82}(B)$ | ND2 | + | + | + |
| $H^{88}$ | NE2 | $T^{77}(B)$ | OG1 | – | + | – |
| $F^{90}$ | N | $Y^{9}(A)$ | O | + | – | + |
| $K^{91}/A^{91}/R^{91}$ | N | $E^{74}(B)$ | OE1 | + | – | + |
| $K^{91}$ | NZ | $Q^{70}(B)$ | OE1 | + | n/a# | +§ |
| $N^{92}$ | N | $N^{62}(A)$ | OD1 | + | + | + |
| $N^{92}$ | ND2 | $N^{62}(A)$ | O | + | + | + |
| $I^{93}$ | N | $Y^{30}(B)$ | OH | + | + | + |
| $V^{94}$ | N | $Y^{67}(B)$ | OH | + | – | + |
| $V^{94}$ | O | $Y^{67}(B)$ | OH | + | – | + |
| $T^{95}$ | O | $Y^{68}(A)$ | OH | + | – | + |
| $P^{96}/A^{96}$ | N | $D^{57}(B)$ | OD1 | – | + | – |

†α-chain, *β-chain, #not applicable, §the NH1 atom of $R^{91}$ in the peptide makes this contact.

**FIGURE 18**

| peptide residue | MHC residue | $MBP_{87-96}$ | $[A^{91},A^{96}]MBP_{87-96}$ | $[R^{91},A^{96}]MBP_{87-96}$ |
|---|---|---|---|---|
| $V^{87}$ | $Y^{9}(A)^{\dagger}$ | + | – | + |
| $V^{87}$ | $F^{54}(A)$ | + | + | + |
| $V^{87}$ | $H^{81}(B)^{*}$ | + | – | – |
| $V^{87}$ | $N^{82}(B)$ | + | – | + |
| $H^{88}$ | $E^{74}(B)$ | + | + | + |
| $H^{88}$ | $T^{77}(B)$ | + | + | + |
| $H^{88}$ | $V^{78}(B)$ | + | + | + |
| $F^{89}$ | $Y^{9}(A)$ | + | – | + |
| $F^{89}$ | $F^{54}(A)$ | + | + | + |
| $F^{89}$ | $G^{58}(A)$ | + | + | + |
| $F^{89}$ | $V^{78}(B)$ | – | + | – |
| $F^{90}$ | $F^{11}(B)$ | + | + | + |
| $F^{90}$ | $G^{13}(B)$ | + | + | + |
| $F^{90}$ | $E^{14}(B)$ | + | + | + |
| $F^{90}$ | $C^{15}(B)$ | + | + | + |
| $F^{90}$ | $V^{78}(B)$ | + | + | – |
| $F^{90}$ | $C^{79}(B)$ | – | + | – |
| $K^{91}$ | $Q^{70}(B)$ | + | n/a$^{\S}$ | n/a |
| $R^{91}/A^{91}$ | $F^{11}(B)$ | n/a | + | + |
| $R^{91}$ | $Y^{67}(B)$ | n/a | n/a | + |
| $N^{92}$ | $T^{65}(A)$ | + | + | + |
| $N^{92}$ | $F^{11}(B)$ | + | + | + |
| $I^{93}$ | $T^{65}(A)$ | + | + | + |
| $I^{93}$ | $Y^{61}(B)$ | + | + | + |
| $I^{93}$ | $Y^{67}(B)$ | + | + | + |
| $V^{94}$ | $T^{65}(A)$ | + | – | + |
| $V^{94}$ | $Y^{61}(B)$ | – | + | – |
| $V^{94}$ | $Y^{68}(A)$ | + | + | + |
| $T^{95}$ | $D^{57}(B)$ | + | + | + |
| $T^{95}$ | $T^{69}(A)$ | – | + | + |
| $T^{95}$ | $Y^{61}(B)$ | – | + | – |
| $P^{96}$ | $Y^{60}(B)$ | + | n/a | n/a |

$^{\dagger}$α-chain, $^{*}$β-chain, $^{\S}$not applicable.

**FIGURE 19**

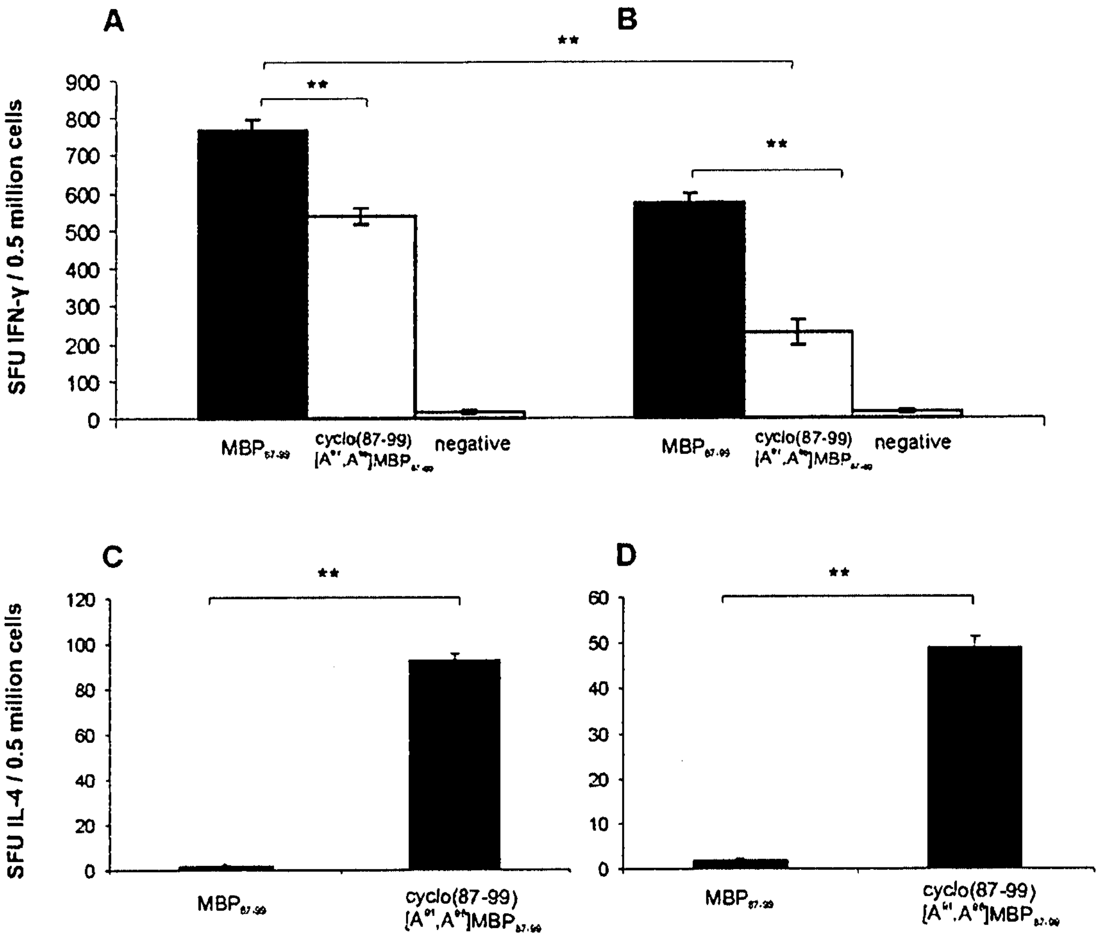
A
B
**
900
800
700
600
500
400
300
200
100
0
SFU IFN-γ / 0.5 million cells
MBP87-99
cyclo(87-99) [A91,A96]MBP87-99
negative
C
D
120
100
80
60
40
20
SFU IL-4 / 0.5 million cells
50
30
10
cyclo(87-99) [A91,A96]MBP87-99

FIGURE 20

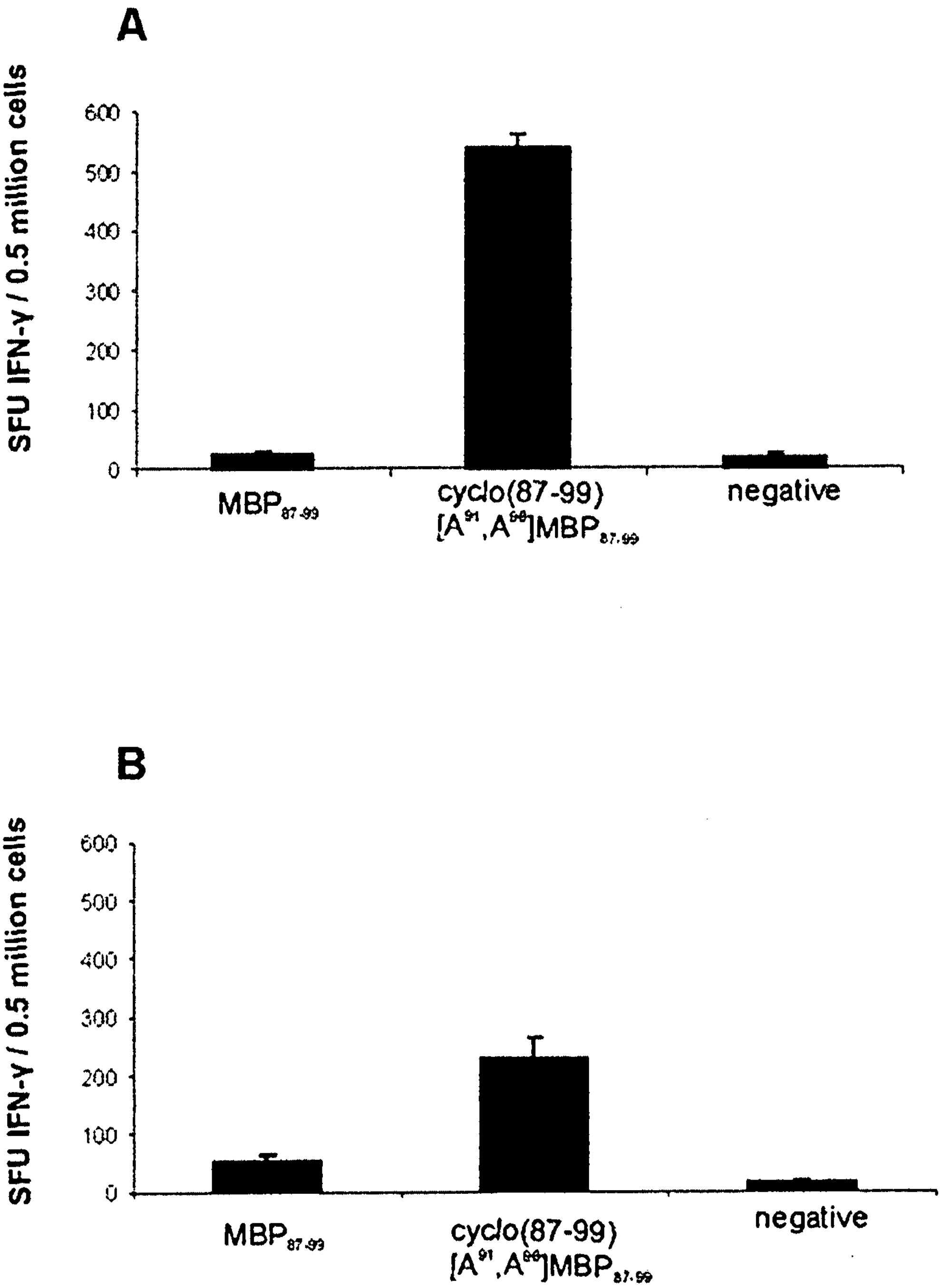
A
SFU IFN-γ / 0.5 million cells
600
500
400
300
200
100
0
MBP87-99
cyclo(87-99)
[A91,A96]MBP87-99
negative
B
SFU IFN-γ / 0.5 million cells
600
500
400
300
200
100
0
MBP87-99
cyclo(87-99)
[A91,A96]MBP87-99
negative

FIGURE 21

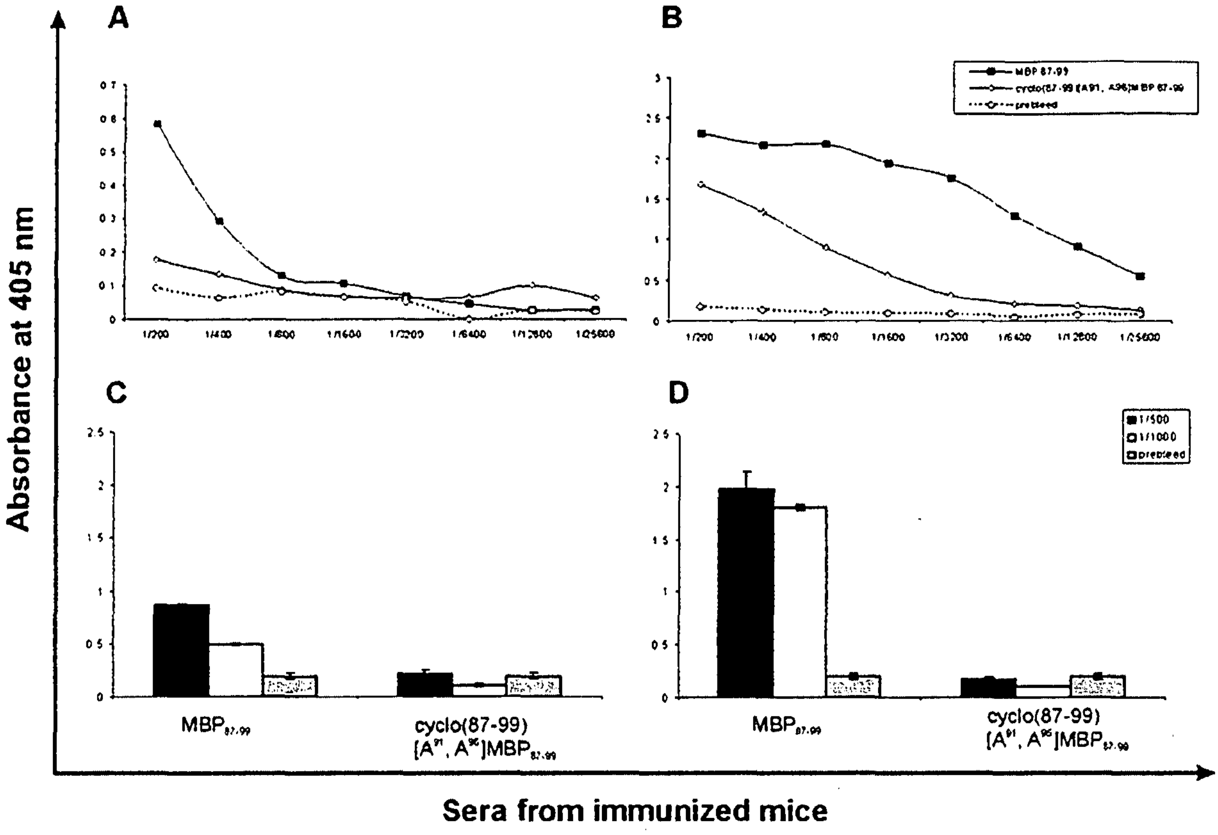
A
B
C
D
Absorbance at 405 nm
$MBP_{87-99}$
cyclo(87-99) $[A^{91}, A^{96}]MBP_{87-99}$
$MBP_{87-99}$
cyclo(87-99) $[A^{91}, A^{96}]MBP_{87-99}$
Sera from immunized mice

FIGURE 22

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- GB 9901855 W


### Non-patent literature cited in the description


- **VALLI et al.** *J Clin. Invest.,* 1993
- **ZAMVIL et al.** *Annu Rev Immunol.,* 1990
- **SOMMER et al.** *J Neuroimmunol,* 1997
- **KALBUS et al.** *Eur J Immunol.,* 2001
- **SIMON RJ et al.** *PNAS,* 1992, vol. 89 (20), 9367-9371
- **HORWELL DC.** *Trends Biotechnol.,* 1995, vol. 13 (4), 132-134
- **ROMAGNANI.** *Immunol Today,* 1991, vol. 12, 256-257
- **MOSMANN.** *Annu Rev Immunol,* 1989, vol. 7, 145-173
- **MOSMANN.** *Annu Rev Immunol,* 1989, vol. 7, 145-173
- Handbook of Pharmaceutical Excipients. 1994
- Remington's Pharmaceutical Sciences. Mack Publishing Co, 1985
- **BERGE et al.** *J Pharm Sci,* 1977, vol. 66, 1-19
- **KATSARA, M. ; MATSOUKAS, J. ; DERAOS, G. ; APOSTOLOPOULOS, V.** Towards immunotherapeutic drugs and vaccines against multiple sclerosis. *Acta Biochim Biophys Sin (Shanghai),* 2008, vol. 40, 636-42
- **MARTIN, R. ; HOWELL, M. D. ; JARAQUEMADA, D. ; FLERLAGE, M. ; RICHERT, J. ; BROSTOFF, S. ; LONG, E. O. ; MCFARLIN, D. E. ; MCFARLAND, H. F.** A myelin basic protein peptide is recognized by cytotoxic T cells in the context of four HLA-DR types associated with multiple sclerosis. *J Exp Med,* 1991, vol. 173, 19-24
- **OKSENBERG, J. R. ; BARANZINI, S. E. ; BARCELLOS, L. F. ; HAUSER, S. L.** Multiple sclerosis: genomic rewards. *J Neuroimmunol,* 2001, vol. 113, 171-84
- **OTA, K. ; MATSUI, M. ; MILFORD, E. L. ; MACKIN, G. A. ; WEINER, H. L. ; HAFLER, D. A.** T-cell recognition of an immunodominant myelin basic protein epitope in multiple sclerosis. *Nature,* 1990, vol. 346, 183-7
- **ZHANG, J. ; MARKOVIC-PLESE, S. ; LACET, B. ; RAUS, J. ; WEINER, H. L. ; HAFLER, D. A.** Increased frequency of interleukin 2-responsive T cells specific for myelin basic protein and proteolipid protein in peripheral blood and cerebrospinal fluid of patients with multiple sclerosis. *J Exp Med,* 1994, vol. 179, 973-84
- **HAFLER, D. A. ; SLAVIK, J. M. ; ANDERSON, D. E. ; O'CONNOR, K. C. ; DE JAGER, P. ; BAECHER-ALLAN, C.** Multiple sclerosis. *Immunol Rev,* 2005, vol. 204, 208-31
- **STEINMAN, L.** Multiple sclerosis: a coordinated immunological attack against myelin in the central nervous system. *Cell,* 1996, vol. 85, 299-302
- **SAKAI, K. ; SINHA, A. A. ; MITCHELL, D. J. ; ZAMVIL, S. S. ; ROTHBARD, J. B. ; MCDEVITT, H. O. ; STEINMAN, L.** Involvement of distinct murine T-cell receptors in the autoimmune encephalitogenic response to nested epitopes of myelin basic protein. *Proc Natl Acad Sci U S A,* 1988, vol. 85, 8608-12
- **SAKAI, K. ; ZAMVIL, S. S. ; MITCHELL, D. J. ; LIM, M. ; ROTHBARD, J. B. ; STEINMAN, L.** Characterization of a major encephalitogenic T cell epitope in SJL/J mice with synthetic oligopeptides of myelin basic protein. *J Neuroimmunol,* 1988, vol. 19, 21-32
- **KARIN, N. ; MITCHELL, D. J. ; BROCKE, S. ; LING, N. ; STEINMAN, L.** Reversal of experimental autoimmune encephalomyelitis by a soluble peptide variant of a myelin basic protein epitope: T cell receptor antagonism and reduction of interferon gamma and tumor necrosis factor alpha production. *J Exp Med,* 1994, vol. 180, 2227-37
- **KATSARA, M. ; YURIEV, E. ; RAMSLAND, P. A. ; DERAOS, G. ; TSELIOS, T. ; MATSOUKAS, J. ; APOSTOLOPOULOS, V.** A double mutation of MBP(83-99) peptide induces IL-4 responses and antagonizes IFN-gamma responses. *J Neuroimmunol,* 2008

- **KATSARA, M. ; YURIEV, E. ; RAMSLAND, P. A. ; DERAOS, G. ; TSELIOS, T. ; MATSOUKAS, J. ; APOSTOLOPOULOS, V.** Mannosylation of mutated MBP(83-99) peptides diverts immune responses from Th1 to Th2. *Mol Immunol,* 2008, vol. 45, 3661-70
- **BROCKE, S. ; GIJBELS, K. ; ALLEGRETTA, M. ; FERBER, I. ; PIERCY, C. ; BLANKENSTEIN, T. ; MARTIN, R. ; UTZ, U. ; KARIN, N. ; MITCHELL, D.** Treatment of experimental encephalomyelitis with a peptide analogue of myelin basic protein. *Nature,* 1996, vol. 379, 343-6
- **CANNELLA, B. ; RAINE, C. S.** The adhesion molecule and cytokine profile of multiple sclerosis lesions. *Ann Neurol,* 1995, vol. 37, 424-35
- **RACKE, M. K. ; BONOMO, A. ; SCOTT, D. E. ; CANNELLA, B. ; LEVINE, A. ; RAINE, C. S. ; SHEVACH, E. M. ; ROCKEN, M.** Cytokine-induced immune deviation as a therapy for inflammatory autoimmune disease. *J Exp Med,* 1994, vol. 180, 1961-6
- **GAUR, A. ; BOEHME, S. A. ; CHALMERS, D. ; CROWE, P. D. ; PAHUJA, A. ; LING, N. ; BROCKE, S. ; STEINMAN, L. ; CONLON, P. J.** Amelioration of relapsing experimental autoimmune encephalomyelitis with altered myelin basic protein peptides involves different cellular mechanisms. *J Neuroimmunol,* 1997, vol. 74, 149-58
- **GAUTHIER, L. ; SMITH, K. J. ; PYRDOL, J. ; KALANDADZE, A. ; STROMINGER, J. L. ; WILEY, D. C. ; WUCHERPFENNIG, K. W.** Expression and crystallization of the complex of HLA-DR2 (DRA, DRB1*1501) and an immunodominant peptide of human myelin basic protein. *Proc Natl Acad Sci U S A,* 1998, vol. 95, 11828-33
- **LI, Y. ; HUANG, Y. ; LUE, J. ; QUANDT, J. A. ; MARTIN, R. ; MARIUZZA, R. A.** Structure of a human autoimmune TCR bound to a myelin basic protein self-peptide and a multiple sclerosis-associated MHC class II molecule. *Embo J,* 2005, vol. 24, 2968-79
- **SMITH, K. J. ; PYRDOL, J. ; GAUTHIER, L. ; WILEY, D. C. ; WUCHERPFENNIG, K. W.** Crystal structure of HLA-DR2 (DRA*0101, DRB1*1501) complexed with a peptide from human myelin basic protein. *J Exp Med,* 1998, vol. 188, 1511-20
- **WUCHERPFENNIG, K. W. ; HAFLER, D. A. ; STROMINGER, J. L.** Structure of human T-cell receptors specific for an immunodominant myelin basic protein peptide: positioning of T-cell receptors on HLA-DR2/peptide complexes. *Proc Natl Acad Sci U S A,* 1995, vol. 92, 8896-900
- **KALBUS, M. ; FLECKENSTEIN, B. T. ; OFFENHAUSSER, M. ; BLUGGEL, M. ; MELMS, A. ; MEYER, H. E. ; RAMMENSEE, H. G. ; MARTIN, R. ; JUNG, G. ; SOMMER, N.** Ligand motif of the autoimmune disease-associated mouse MHC class II molecule H2-A(s). *Eur J Immunol,* 2001, vol. 31, 551-62
- **MATSOUKAS, J. ; APOSTOLOPOULOS, V. ; KALBACHER, H. ; PAPINI, A. M. ; TSELIOS, T. ; CHATZANTONI, K. ; BIAGIOLI, T. ; LOLLI, F. ; DERAOS, S. ; PAPATHANASSOPOULOS, P.** Design and synthesis of a novel potent myelin basic protein epitope 87-99 cyclic analogue: enhanced stability and biological properties of mimics render them a potentially new class of immunomodulators. *J Med Chem,* 2005, vol. 48, 1470-80
- **KATSARA, M. ; DERAOS, G. ; TSELIOS, T. ; MATSOUKAS, J. ; APOSTOLOPOULOS, V.** Design of novel cyclic altered peptide ligands of myelin basic protein MBP83-99 that modulate immune responses in SJL/J mice. *J Med Chem,* 2008, vol. 51, 3971-8
- **KATSARA, M. ; TSELIOS, T. ; DERAOS, S. ; DERAOS, G. ; MATSOUKAS, M. T. ; LAZOURA, E. ; MATSOUKAS, J. ; APOSTOLOPOULOS, V.** Round and round we go: cyclic peptides in disease. *Curr Med Chem,* 2006, vol. 13, 2221-32
- **ACRES, B. ; APOSTOLOPOULOS, V. ; BALLOUL, J. M. ; WRESCHNER, D. ; XING, P. X. ; ALI-HADJI, D. ; BIZOUARNE, N. ; KIENY, M. P. ; MCKENZIE, I. F.** MUC1-specific immune responses in human MUC1 transgenic mice immunized with various human MUC1 vaccines. *Cancer Immunol Immunother,* 2000, vol. 48, 588-94
- **APOSTOLOPOULOS, V. ; BARNES, N. ; PIETERSZ, G. A. ; MCKENZIE, I. F.** Ex vivo targeting of the macrophage mannose receptor generates anti-tumor CTL responses. *Vaccine,* 2000, vol. 18, 3174-84
- **APOSTOLOPOULOS, V. ; KARANIKAS, V. ; HAURUM, J. S. ; MCKENZIE, I. F.** Induction of HLA-A2-restricted CTLs to the mucin 1 human breast cancer antigen. *J Immunol,* 1997, vol. 159, 5211-8
- **APOSTOLOPOULOS, V. ; MCKENZIE, I. F.** Role of the mannose receptor in the immune response. *Curr Mol Med,* 2001, vol. 1, 469-74
- **APOSTOLOPOULOS, V. ; PIETERSZ, G. A. ; GORDON, S. ; MARTINEZ-POMARES, L. ; MCKENZIE, I. F.** Aldehyde-mannan antigen complexes target the MHC class I antigen-presentation pathway. *Eur J Immunol,* 2000, vol. 30, 1714-23
- **APOSTOLOPOULOS, V. ; PIETERSZ, G. A. ; LOVELAND, B. E. ; SANDRIN, M. S. ; MCKENZIE, I. F.** Oxidative/reductive conjugation of mannan to antigen selects for T1 or T2 immune responses. *Proc Natl Acad Sci USA,* 1995, vol. 92, 10128-32
- **APOSTOLOPOULOS, V. ; PIETERSZ, G. A. ; MCKENZIE, I. F.** Cell-mediated immune responses to MUC1 fusion protein coupled to mannan. *Vaccine,* 1996, vol. 14, 930-8

- **SHENG, K. C. ; POUNIOTIS, D. S. ; WRIGHT, M. D. ; TANG, C. K. ; LAZOURA, E. ; PIETERSZ, G. A. ; APOSTOLOPOULOS, V.** Mannan derivatives induce phenotypic and functional maturation of mouse dendritic cells. *Immunology,* 2006, vol. 118, 372-83
- **HARDING, C. V. ; LEYVA-COBIAN, F. ; UNANUE, E. R.** Mechanisms of antigen processing. *Immunol Rev,* 1988, vol. 106, 77-92
- **EVAVOLD, B. D. ; ALLEN, P. M.** Separation of IL-4 production from Th cell proliferation by an altered T cell receptor ligand. *Science,* 1991, vol. 252, 1308-10
- **EVAVOLD, B. D. ; SLOAN-LANCASTER, J. ; HSU, B. L. ; ALLEN, P. M.** Separation of T helper 1 clone cytolysis from proliferation and lymphokine production using analog peptides. *J Immunol,* 1993, vol. 150, 3131-40
- **SLOAN-LANCASTER, J. ; EVAVOLD, B. D. ; ALLEN, P. M.** Induction of T-cell anergy by altered T-cell-receptor ligand on live antigen-presenting cells. *Nature,* 1993, vol. 363, 156-9
- **NICHOLSON, L. B. ; GREER, J. M. ; SOBEL, R. A. ; LEES, M. B. ; KUCHROO, V. K.** An altered peptide ligand mediates immune deviation and prevents autoimmune encephalomyelitis. *Immunity,* 1995, vol. 3, 397-405
- **WINDHAGEN, A. ; SCHOLZ, C. ; HOLLSBERG, P. ; FUKAURA, H. ; SETTE, A. ; HAFLER, D. A.** Modulation of cytokine patterns of human autoreactive T cell clones by a single amino acid substitution of their peptide ligand. *Immunity,* 1995, vol. 2, 373-80
- **STREET, N. E. ; MOSMANN, T. R.** Functional diversity of T lymphocytes due to secretion of different cytokine patterns. *Faseb J,* 1991, vol. 5, 171-7
- **SWAIN, S. L.** IL4 dictates T-cell differentiation. *Res Immunol,* 1993, vol. 144, 616-20
- **BIELEKOVA, B. ; GOODWIN, B. ; RICHERT, N. ; CORTESE, L ; KONDO, T. ; AFSHAR, G. ; GRAN, B. ; EATON, J. ; ANTEL, J. ; FRANK, J. A.** Encephalitogenic potential of the myelin basic protein peptide (amino acids 83-99) in multiple sclerosis: results of a phase II clinical trial with an altered peptide ligand. *Nat Med,* 2000, vol. 6, 1167-75
- **KAPPOS, L. ; COMI, G. ; PANITCH, H. ; OGER, J. ; ANTEL, J. ; CONLON, P. ; STEINMAN, L.** Induction of a non-encephalitogenic type 2 T helper-cell autoimmune response in multiple sclerosis after administration of an altered peptide ligand in a placebo-controlled, randomized phase II trial. The Altered Peptide Ligand in Relapsing MS Study Group. *Nat Med,* 2000, vol. 6, 1176-82
- **IERO, M. ; SQUARCINA, P. ; ROMERO, P. ; GUILLAUME, P. ; SCARSELLI, E. ; CERINO, R. ; CARRABBA, M. ; TOUTIRAIS, O. ; PARMIANI, G. ; RIVOLTINI, L.** Low TCR avidity and lack of tumor cell recognition in CD8(+) T cells primed with the CEA-analogue CAP1-6D peptide. *Cancer Immunol Immunother,* 2007, vol. 56, 1979-91
- **DEGANO, M. ; GARCIA, K. C. ; APOSTOLOPOULOS, V. ; RUDOLPH, M. G. ; TEYTON, L. ; WILSON, I. A.** A functional hot spot for antigen recognition in a superagonist TCR/MHC complex. *Immunity,* 2000, vol. 12, 251-61
- **THOMSON, C. T. ; KALERGIS, A. M. ; SACCHETTINI, J. C. ; NATHENSON, S. G.** A structural difference limited to one residue of the antigenic peptide can profoundly alter the biological outcome of the TCR-peptide/MHC class I interaction. *J Immunol,* 2001, vol. 166, 3994-7
- **KALERGIS, A. M. ; NATHENSON, S. G.** Altered peptide ligand-mediated TCR antagonism can be modulated by a change in a single amino acid residue within the CDR3 beta of an MHC class I-restricted TCR. *J Immunol,* 2000, vol. 165, 280-5
- **WALLACE, A. C. ; LASKOWSKI, R. A. ; THORNTON, J. M.** LIGPLOT: a program to generate schematic diagrams of protein-ligand interactions. *Protein Eng,* 1995, vol. 8, 127-34
- **TSELIOS, T. ; APOSTOLOPOULOS, V. ; DALIANI, I. ; DERAOS, S. ; GRDADOLNIK, S. ; MAVROMOUSTAKOS, T. ; MELACHRINOU, M. ; THYMIANOU, S. ; PROBERT, L. ; MOUZAKI, A.** Antagonistic effects of human cyclic MBP(87-99) altered peptide ligands in experimental allergic encephalomyelitis and human T-cell proliferation. *J Med Chem,* 2002, vol. 45, 275-83
- **TSELIOS, T. ; DALIANI, I. ; DERAOS, S. ; THYMIANOU, S. ; MATSOUKAS, E. ; TROGANIS, A. ; GEROTHANASSIS, L. ; MOUZAKI, A. ; MAVROMOUSTAKOS, T. ; PROBERT, L.** Treatment of experimental allergic encephalomyelitis (EAE) by a rationally designed cyclic analogue of myelin basic protein (MBP) epitope 72-85. *Bioorg Med Chem Lett,* 2000, vol. 10, 2713-7
- **TSELIOS, T. ; DALIANI, I. ; PROBERT, L. ; DERAOS, S. ; MATSOUKAS, E. ; ROY, S. ; PIRES, J. ; MOORE, G. ; MATSOUKAS, J.** Treatment of experimental allergic encephalomyelitis (EAE) induced by guinea pig myelin basic protein epitope 72-85 with a human MBP(87-99) analogue and effects of cyclic peptides. *Bioorg Med Chem,* 2000, vol. 8, 1903-9

- **TSELIOS, T. ; PROBERT, L. ; DALIANI, I. ; MATSOUKAS, E. ; TROGANIS, A. ; GEROTHANASSIS, I. P. ; MAVROMOUSTAKOS, T. ; MOORE, G. J. ; MATSOUKAS, J. M.** Design and synthesis of a potent cyclic analogue of the myelin basic protein epitope MBP72-85: importance of the Ala81 carboxyl group and of a cyclic conformation for induction of experimental allergic encephalomyelitis. *J Med Chem,* 1999, vol. 42, 1170-7
- **TSELIOS, T. V. ; LAMARI, F. N. ; KARATHANASOPOULOU, I. ; KATSARA, M. ; APOSTOLOPOULOS, V. ; PIETERSZ, G. A. ; MATSOUKAS, J. M. ; KARAMANOS, N. K.** Synthesis and study of the electrophoretic behavior of mannan conjugates with cyclic peptide analogue of myelin basic protein using lysine-glycine linker. *Anal Biochem,* 2005, vol. 347, 121-8
- **APOSTOLOPOULOS, V. ; PIETERSZ, G. A. ; MCKENZIE, I. F.** Cell-mediated immune responses to MUCI fusion protein coupled to mannan. *Vaccine,* 1996, vol. 14, 930-8
- **BIELEKOVA, B. ; GOODWIN, B. ; RICHERT, N. ; CORTESE, I. ; KONDO, T. ; AFSHAR, G. ; GRAN, B. ; EATON, J. ; ANTEL, J. ; FRANK, J. A.** Encephalitogenic potential of the myelin basic protein peptide (amino acids 83-99) in multiple sclerosis: results of a phase II clinical trial with an altered peptide ligand. *Nat Med,* 2000, vol. 6, 1167-75
- **TSELIOS, T. ; DALIANI, I. ; DERAOS, S. ; THYMIANOU, S. ; MATSOUKAS, E. ; TROGANIS, A. ; GEROTHANASSIS, I. ; MOUZAKI, A. ; MAVROMOUSTAKOS, T. ; PROBERT, L.** Treatment of experimental allergic encephalomyelitis (EAE) by a rationally designed cyclic analogue of myelin basic protein (MBP) epitope 72-85. *Bioorg Med Chem Lett,* 2000, vol. 10, 2713-7
- **APOSTOLOPOULOS, V. ; YURIEV, E. ; RAMSLAND, P. A. ; HALTON, J. ; OSINSKI, C. ; LI, W. ; PLEBANSKI, M. ; PAULSEN, H. ; MCKENZIE, I. F.** A glycopeptide in complex with MHC class I uses the GalNAc residue as an anchor. *Proc Natl Acad Sci U S A,* 2003, vol. 100, 15029-34
- **ABROMSON-LEEMAN, S. et al.** T-cell properties determine disease site, clinical presentation, and cellular pathology of experimental autoimmune encephalomyelitis. *Am J Pathol,* 2004, vol. 165, 1519-33
- **ACRES, B. ; V. APOSTOLOPOULOS ; J. M. BALLOUL ; D. WRESCHNER ; P. X. XING ; D. ALI-HADJI ; N. BIZOUARNE ; M. P. KIENY ; I. F. MCKENZIE.** MUC1-specific immune responses in human MUC1 transgenic mice immunized with various human MUC1 vaccines. *Cancer Immunol Immunother,* 2000, vol. 48, 588-594
- **APOSTOLOPOULOS, V. ; G. A. PIETERSZ ; I. F. MCKENZIE.** Cell-mediated immune responses to MUC1 fusion protein coupled to mannan. *Vaccine,* 1996, vol. 14, 930-938
- **APOSTOLOPOULOS, V. ; G. A. PIETERSZ ; B. E. LOVELAND ; M. S. SANDRIN ; I. F. MCKENZIE.** Oxidative/reductive conjugation of mannan to antigen selects for T1 or T2 immune responses. *Proc Natl Acad Sci U S A,* 1995, vol. 92, 10128-10132
- **APOSTOLOPOULOS, V. ; V. KARANIKAS ; J. S. HAURUM ; I. F. MCKENZIE.** Induction of HLA-A2-restricted CTLs to the mucin 1 human breast cancer antigen. *J Immunol,* 1997, vol. 159, 5211-5218
- **APOSTOLOPOULOS, V. ; LOFTHOUSE, S. ; POPOVSKI, V. ; CHELVANAYAGAM G. ; SANDRIN, M. ; MCKENZIE I.** *Nature Biot.,* 1998, vol. 16, 278
- **BANKOVICH, A. J. ; A. T. GIRVIN ; A. K. MOESTA ; K. C. GARCIA.** Peptide register shifting within the MHC groove: theory becomes reality. *Molecular immunology,* 2004, vol. 40, 1033-1039
- **BARLOS, K. ; D. GATOS.** 9-Fluorenylmethyloxycarbonyl/ tbutyl-based convergent protein synthesis. *Biopolymers,* 1999, vol. 51, 266-278
- **BARLOS, K. ; D. GATOS ; S. KOUTSOGIANNI.** Fmoc/Trt-amino acids: comparison to Fmoc/tBu-amino acids in peptide synthesis. *J Pept Res,* 1998, vol. 51, 194-200
- **BAUER, J. et al.** T-cell apoptosis in inflammatory brain lesions: destruction of T cells does not depend on antigen recognition. *Am J Pathol,* 1998, vol. 153, 715-24
- **BERGER, T. et al.** Experimental autoimmune encephalomyelitis: the antigen specificity of T lymphocytes determines the topography of lesions in the central and peripheral nervous system. *Lab Invest,* 1997, vol. 76, 355-64
- **BIEGANOWSKA, K. D. ; L. J. AUSUBEL ; Y. MODABBER ; E. SLOVIK ; W. MESSERSMITH ; D. A. HAFLER.** Direct ex vivo analysis of activated, Fas-sensitive autoreactive T cells in human autoimmune disease. *J Exp Med,* 1997, vol. 185, 1585-1594
- **BROCKE, S. ; GIJBELS, K. ; ALLEGRETTA, M. ; FERBER, I. ; PIERCY, C. ; BLANKENSTEIN, T. ; MARTIN, R. ; UTZ, U. ; KARIN, N. ; MITCHELL, D.** *Nature,* 1996, vol. 379, 343
- **BRUGGLE, F. ; TIMMERMANS, M. ; VAN UNEN, A. ; TEN HOVE, J. ; VAN DE WERKEN, G. ; POOLMAN, T. ; HOOGERHOUT, P.** *J. Pept. Sci.,* 1999, vol. 5, 381
- **CHALOIN, L. ; MERY, J. ; VAN MAU, N. ; DIVITA, G. ; HEITZ, F.** *J. Pept. Sci.,* 1999, vol. 5, 381
- **CORPER, A. L. ; T. STRATMANN ; V. APOSTOLOPOULOS ; C. A. SCOTT ; K. C. GARCIA ; A. S. KANG ; I. A. WILSON ; L. TEYTON.** A structural framework for deciphering the link between I-Ag7 and autoimmune diabetes. *Science,* 2000, vol. 288, 505-511

- **DAVIS, W. C. ; R. L. KONZEK ; K. HAAS ; D. M. ESTES ; M. J. HAMILTON ; D. R. CALL ; V. APOSTOLOPOULOS ; I. F. MCKENZIE.** Use of the mannan receptor to selectively target vaccine antigens for processing and antigen presentation through the MHC class I and class II pathways. *Ann N Y Acad Sci,* 2002, vol. 969, 119-125
- **ENCINAS, J. A. et al.** Identification of genetic loci associated with paralysis, inflammation and weight loss in mouse experimental autoimmune encephalomyelitis. *Int Immunol,* 2001, vol. 13, 257-64
- **FLEMING, K. K. et al.** Statistical analysis of data from studies on experimental autoimmune encephalomyelitis. *J Neuroimmunol,* 2005, vol. 170, 71-84
- **FONTOURA, P. ; GARREN, H. ; STEINMAN, L.** Antigen-specific therapies in Multiple Sclerosis: Going beyond proteins and peptides. *Int Rev Immunol,* 2005, vol. 24, 415-446
- **FREMONT, D. H. ; D. MONNAIE ; C. A. NELSON ; W. A. HENDRICKSON ; E. R. UNANUE.** Crystal structure of I-Ak in complex with a dominant epitope of lysozyme. *Immunity,* 1998, vol. 8, 305-317
- **GAUTHIER, L. ; K. J. SMITH ; J. PYRDOL ; A. KALANDADZE ; J. L. STROMINGER ; D. C. WILEY ; K. W. WUCHERPFENNIG.** Expression and crystallization of the complex of HLA-DR2 (DRA, DRB1*1501) and an immunodominant peptide of human myelin basic protein. *Proc Natl Acad Sci USA,* 1998, vol. 95, 11828-11833
- **GRIGORIADIS, N. ; BEN-HUR, T. ; KARUSSIS, D. ; MILONAS, I.** Axonal damage in multiple sclerosis: a complex issue in a complex disease. *Clin Neurol Neurosurg,* 2004, vol. 106, 211-7
- **HAFLER, D. ; WEINER, H.** *Immunol. Rev.,* 1995, vol. 144, 75
- **HE, X. L. ; C. RADU ; J. SIDNEY ; A. SETTE ; E. S. WARD ; K. C. GARCIA.** Structural snapshot of aberrant antigen presentation linked to autoimmunity: the immunodominant epitope of MBP complexed with I-Au. *Immunity,* 2002, vol. 17, 83-94
- **IWAI, H. ; PLUCKTHUN, A.** *FEBS Lett.,* 1999, vol. 459, 166
- **KALBUS, M. ; B. T. FLECKENSTEIN ; M. OFFENHAUSSER ; M. BLUGGEL ; A. MELMS ; H. E. MEYER ; H. G. RAMMENSEE ; R. MARTIN ; G. JUNG ; N. SOMMER.** Ligand motif of the autoimmune disease-associated mouse MHC class II molecule H2-A(s). *European journal of immunology,* 2001, vol. 31, 551-562
- **KARANIKAS, V. ; L. A. HWANG ; J. PEARSON ; C. S. ONG ; V. APOSTOLOPOULOS ; H. VAUGHAN ; P. X. XING ; G. JAMIESON ; G. PIETERSZ ; B. TAIT.** Antibody and T cell responses of patients with adenocarcinoma immunized with mannan-MUC1 fusion protein. *J Clin Invest,* 1997, vol. 100, 2783-2792
- **KRAKOWSKI, M. L. ; OWENS, T.** Naive T lymphocytes traffic to inflamed central nervous system, but require antigen recognition for activation. *Eur J Immunol,* 2000, vol. 30, 1002-9
- **KRISSINEL, E. ; K. HENRICK.** Inference of Macromolecular Assemblies from Crystalline State. *J Mol Biol.,* 2007
- **KROGSGAARD, M. ; K. W. WUCHERPFENNIG ; B. CANNELLA ; B. E. HANSEN ; A. SVEJGAARD ; J. PYRDOL ; H. DITZEL ; C. RAINE ; J. ENGBERG ; L. FUGGER.** Visualization of myelin basic protein (MBP) T cell epitopes in multiple sclerosis lesions using a monoclonal antibody specific for the human histocompatibility leukocyte antigen (HLA)-DR2-MBP 85-99 complex. *J Exp Med,* 2000, vol. 191, 1395-1412
- **KUCHROO, V. K. ; J. M. GREER ; D. KAUL ; G. ISHIOKA ; A. FRANCO ; A. SETTE ; R. A. SOBEL ; M. B. LEES.** A single TCR antagonist peptide inhibits experimental allergic encephalomyelitis mediated by a diverse T cell repertoire. *J Immunol,* 1994, vol. 153, 3326-3336
- **LAMBERT, J. D. ; R. O. MEYERS ; B. N. TIMMERMANN ; R. T. DORR.** Pharmacokinetic analysis by high-performance liquid chromatography of intravenous nordihydroguaiaretic acid in the mouse. *J Chromatogr B Biomed Sci Appl,* 2001, vol. 754, 85-90
- **LASSMANN H.** Experimental models of multiple sclerosis. *Rev Neurol,* 2007, vol. 163 (6-7), 651-5
- **LATEK, R. R. ; A. SURI ; S. J. PETZOLD ; C. A. NELSON ; O. KANAGAWA ; E. R. UNANUE ; D. H. FREMONT.** Structural basis of peptide binding and presentation by the type I diabetes-associated MHC class II molecule of NOD mice. *Immunity,* 2000, vol. 12, 699-710
- **LEES, C. J. ; V. APOSTOLOPOULOS ; B. ACRES ; C. S. ONG ; V. POPOVSKI ; I. F. MCKENZIE.** The effect of T1 and T2 cytokines on the cytotoxic T cell response to mannan-MUC 1. *Cancer Immunol Immunother,* 2000, vol. 48, 644-652
- **LEES, C. J. ; V. APOSTOLOPOULOS ; B. ACRES ; I. RAMSHAW ; A. RAMSAY ; C. S. ONG ; I. F. MCKENZIE.** Immunotherapy with mannan-MUC1 and IL-12 in MUC1 transgenic mice. *Vaccine,* 2000, vol. 19, 158-162
- **LIU, X. ; S. DAI ; F. CRAWFORD ; R. FRUGE ; P. MARRACK ; J. KAPPLER.** Alternate interactions define the binding of peptides to the MHC molecule IA(b). *Proc Natl Acad Sci U S A,* 2002, vol. 99, 8820-8825
- **LOFTHOUSE, S. A. ; V. APOSTOLOPOULOS ; G. A. PIETERSZ ; W. LI ; I. F. MCKENZIE.** Induction of T1 (cytotoxic lymphocyte) and/or T2 (antibody) responses to a mucin-1 tumour antigen. *Vaccine,* 1997, vol. 15, 1586-1593

- **LOURBOPOULOS, S. GRIGORIADIS ; C. SYMEONIDOU ; G. DERETZI ; N. TASKOS ; E. SHOHAMI ; N. GRIGORIADIS.** Modified Bielschowsky silver impregnation combined with Hematoxylin or Cresyl Violet counterstaining as a potential tool for the simultaneous study of inflammation and axonal injury in the central nervous system. *Aristotle University Med J,* 2007, vol. 34, 31-39
- **MARTIN, R. ; M. D. HOWELL ; D. JARAQUEMADA ; M. FLERLAGE ; J. RICHERT ; S. BROSTOFF ; E. O. LONG ; D. E. MCFARLIN ; H. F. MCFARLAND.** A myelin basic protein peptide is recognized by cytotoxic T cells in the context of four HLA-DR types associated with multiple sclerosis. *J Exp Med,* 1991, vol. 173, 19-24
- **MARTIN, R. ; MCFARLAND, H. ; MCFARLIN, D.** *Ann. Rev. Immunol.,* 1992, vol. 10, 153
- **MATSOUKAS, J. ; V. APOSTOLOPOULOS ; H. KALBACHER ; A. M. PAPINI ; T. TSELIOS ; K. CHATZANTONI ; T. BIAGIOLI ; F. LOLLI ; S. DERAOS ; P. PAPATHANASSOPOULOS.** Design and synthesis of a novel potent myelin basic protein epitope 87-99 cyclic analogue: enhanced stability and biological properties of mimics render them a potentially new class of immunomodulators. *J Med Chem,* 2005, vol. 48, 1470-1480
- **MEZO, G. ; MAJER, Z. ; VALERO, L. ; ANDREU, D. ; HUDECZ, F.** *J. Pept. Sci.,* 1999, vol. 5, 272
- **MOREAU, T. ; A. COLES ; M. WING ; J. ISAACS ; G. HALE ; H. WALDMANN ; A. COMPSTON.** Transient increase in symptoms associated with cytokine release in patients with multiple sclerosis. *Brain,* 1996, vol. 119, 225-237
- **OLIGINO, L. ; LUNG, T. ; SASTRY, L. ; BIGELOW, J. ; CAO, T. ; CURRAN, M. ; BURKE, R. ; WANG, S. ; KRAG, D. ; ROLLER, P.** *J. Biol. Chem.,* 1997, vol. 272, 29046
- **OTA, K. ; M. MATSUI ; E. L. MILFORD ; G. A. MACKIN ; H. L. WEINER ; D. A. HAFLER.** T-cell recognition of an immunodominant myelin basic protein epitope in multiple sclerosis. *Nature,* 1990, vol. 346, 183-187
- **PAPADOPOULOS, D. ; PHAM-DINH, D. ; REYNOLDS, R.** Axon loss is responsible for chronic neurological deficit following inflammatory demyelination in the rat. *Exp Neurol,* 2006, vol. 197, 373-85
- **PENDER MP ; RIST MJ.** Apoptosis of inflammatory cells in immune control of the nervous system: role of glia. *Glia,* 2001, vol. 36 (2), 137-44
- **REIZIS, B. ; M. EISENSTEIN ; F. MOR ; I. R. COHEN.** The peptide-binding strategy of the MHC class II I-A molecules. *Immunology today,* 1998, vol. 19, 212-216
- **RUDENSKY, A. ; P. PRESTON-HURLBURT ; B. K. AL-RAMADI ; J. ROTHBARD ; C. A. JANEWAY, JR.** Truncation variants of peptides isolated from MHC class II molecules suggest sequence motifs. *Nature,* 1992, vol. 359, 429-431
- **SCOTT, C. A. ; P. A. PETERSON ; L. TEYTON ; I. A. WILSON.** Crystal structures of two I-Ad-peptide complexes reveal that high affinity can be achieved without large anchor residues. *Immunity,* 1998, vol. 8, 319-329
- **SCOTT, P. ; ABEL-SANTOS, E. ; WALL, M. ; WAHNON, C. ; BENKOVIC, J.** *Proc. Natl. Acad. Sci. USA,* 1999, vol. 16, 16
- **SMITH, K. J. ; J. PYRDOL ; L. GAUTHIER ; D. C. WILEY ; K. W. WUCHERPFENNIG.** Crystal structure of HLA-DR2 (DRA*0101, DRB1*1501) complexed with a peptide from human myelin basic protein. *J Exp Med,* 1998, vol. 188, 1511-1520
- **SOMMER, N. ; R. MARTIN ; H. F. MCFARLAND ; L. QUIGLEY ; B. CANNELLA ; C. S. RAINE ; D. E. SCOTT ; P. A. LOSCHMANN ; M. K. RACKE.** Therapeutic potential of phosphodiesterase type 4 inhibition in chronic autoimmune demyelinating disease. *J Neuroimmunol,* 1997, vol. 79, 54-61
- **SUVANNAVEJH GC ; DAL CANTO MC ; MATIS LA ; MILLER SD.** Fas-mediated apoptosis in clinical remissions of relapsing experimental autoimmune encephalomyelitis. *J Clin Invest,* 2000, vol. 105 (2), 223-31
- **TAM, P. ; CLAVIJO, P. ; LU, Y. ; NUSSENZWEIG, S. ; NUSSENZWEIG, S. ; ZAVALA, F.** *J. Exp. Med,* 1990, vol. 171, 299
- **TAMILARASU, N. ; HUQ, I. ; RANA, T.** *Bioor. Med. Chem. Let.,* 2000, vol. 10, 971
- **TSELIOS, T. V. ; F. N. LAMARI ; I. KARATHANASOPOULOU ; M. KATSARA ; V. APOSTOLOPOULOS ; A. PIETERSZ ; J. M. MATSOUKAS ; N. K. KARAMANOS.** Synthesis and study of the electrophoretic behavior of mannan conjugates with cyclic peptide analogue of myelin basic protein using lysine-glycine linker. *Anal Biochem,* 2005, vol. 347, 121-128
- **TSELIOS, T. ; I. DALIANI ; L. PROBERT ; S. DERAOS ; E. MATSOUKAS ; S. ROY ; J. PIRES ; G. MOORE ; J. MATSOUKAS.** Treatment of experimental allergic encephalomyelitis (EAE) induced by guinea pig myelin basic protein epitope 72-85 with a human MBP(87-99) analogue and effects of cyclic peptides. *Bioorg Med Chem,* 2000, vol. 8, 1903-1909
- **TSELIOS, T. ; I. DALIANI ; S. DERAOS ; S. THYMIANOU ; E. MATSOUKAS ; A. TROGANIS ; I. GEROTHANASSIS ; A. MOUZAKI ; T. MAVROMOUSTAKOS ; L. PROBERT.** Treatment of experimental allergic encephalomyelitis (EAE) by a rationally designed cyclic analogue of myelin basic protein (MBP) epitope 72-85. *Bioorg Med Chem Lett,* 2000, vol. 10, 2713-2717

- **TSELIOS, T. ; L. PROBERT ; I. DALIANI ; E. MATSOUKAS ; A. TROGANIS ; I. P. GEROTHANASSIS ; T. MAVROMOUSTAKOS ; G. J. MOORE ; J. M. MATSOUKAS.** Design and synthesis of a potent cyclic analogue of the myelin basic protein epitope MBP72-85: importance of the Ala81 carboxyl group and of a cyclic conformation for induction of experimental allergic encephalomyelitis. *J Med Chem,* 1999, vol. 42, 1170-1177
- **TSELIOS, T. ; V. APOSTOLOPOULOS ; I. DALIANI ; S. DERAOS ; S. GRDADOLNIK ; T. MAVROMOUSTAKOS ; M. MELACHRINOU ; S. THYMIANOU ; L. PROBERT ; A. MOUZAKI.** Antagonistic effects of human cyclic MBP(87-99) altered peptide ligands in experimental allergic encephalomyelitis and human T-cell proliferation. *J Med Chem,* 2002, vol. 45, 275-283
- **VALLI, A. ; A. SETTE ; L. KAPPOS ; C. OSEROFF ; J. SIDNEY ; G. MIESCHER ; M. HOCHBERGER ; E. D. ALBERT ; L. ADORINI.** Binding of myelin basic protein peptides to human histocompatibility leukocyte antigen class II molecules and their recognition by T cells from multiple sclerosis patients. *J Clin Invest,* 1993, vol. 91, 616-628
- **VAUGHAN, H. A. ; D. W. HO ; V. A. KARANIKAS ; C. S. ONG ; L. A. HWANG ; J. M. PEARSON ; I. F. MCKENZIE ; G. A. PIETERSZ.** Induction of humoral and cellular responses in cynomolgus monkeys immunized with mannan-human MUC1 conjugates. *Vaccine,* 1999, vol. 17, 2740-2752
- **VERGELI, M. ; HEMMER, B. ; UTZ, U. ; VOGT, A. ; KALBUS, M. ; TRANQUILL, L. ; CONLON, P. ; LING, N. ; STEINMAN, L. ; MCFARLAND, H.** *Eur. J. Immunol.,* 1996, vol. 26, 2624
- **WALLACE, A. C. ; R. A. LASKOWSKI ; J. M. THORNTON.** LIGPLOT: a program to generate schematic diagrams of protein-ligand interactions. *Protein engineering,* 1995, vol. 8, 127-134
- **WANG, J. H. ; R. MEIJERS ; Y. XIONG ; J. H. LIU ; T. SAKIHAMA ; R. ZHANG ; A. JOACHIMIAK ; E. L. REINHERZ.** Crystal structure of the human CD4 N-terminal two-domain fragment complexed to a class II MHC molecule. *Proc Natl Acad Sci U S A,* 2001, vol. 98, 10799-10804
- **WILLENBORG, D. O. ; M. A. STAYKOVA.** Cytokines in the pathogenesis and therapy of autoimmune encephalomyelitis and multiple sclerosis. *Adv Exp Med Biol,* 2003, vol. 520, 96-119
- **WUCHERPFENNIG, K. W. ; D. A. HAFLER ; J. L. STROMINGER.** Structure of human T-cell receptors specific for an immunodominant myelin basic protein peptide: positioning of T-cell receptors on HLA-DR2/peptide complexes. *Proc Natl Acad Sci U S A,* 1995, vol. 92, 8896-8900
- **ZAMVIL, S. S. ; L. STEINMAN.** The T lymphocyte in experimental allergic encephalomyelitis. *Annu Rev Immunol,* 1990, vol. 8, 579-621
- **ZAMVIL, S. ; P. NELSON ; J. TROTTER ; D. MITCHELL ; R. KNOBLER ; R. FRITZ ; L. STEINMAN.** T-cell clones specific for myelin basic protein induce chronic relapsing paralysis and demyelination. *Nature,* 1985, vol. 317, 355-358
- **ZHANG, J. ; S. MARKOVIC-PLESE ; B. LACET ; J. RAUS ; H. L. WEINER ; D. A. HAFLER.** Increased frequency of interleukin 2-responsive T cells specific for myelin basic protein and proteolipid protein in peripheral blood and cerebrospinal fluid of patients with multiple sclerosis. *J Exp Med,* 1994, vol. 179, 973-984
- **ZHU, Y. ; A. Y. RUDENSKY ; A. L. CORPER ; L. TEYTON ; I. A. WILSON.** Crystal structure of MHC class II I-Ab in complex with a human CLIP peptide: prediction of an I-Ab peptide-binding motif. *J Mol Biol,* 2003, vol. 326, 1157-1174